# EUROPEAN PATENT APPLICATION

(11) **EP 4 032 896 A1**
(43) Date of publication of application: **27.07.2022**
(21) Application number: 21382044.2
(22) Date of filing: 20.01.2021
(51) Int. Cl.: C07D 513/04, A61P 1/16, A61P 11/00, A61P 35/00, A61K 31/429, A61K 31/519

(54) **THIAZOLOPYRIMIDONES AS INHIBITORS OF DDR1/2 AND THERAPEUTIC USES THEREOF**

(71) Applicant: Fundación del Sector Público Estatal Centro Nacional de Investigaciones Oncológicas Carlos III (F.S.P. CNIO), 28029 Madrid (ES)
(72) Inventor: PASTOR FERNÁNDEZ, Joaquín, 28029 Madrid (ES); MARTÍNEZ GONZÁLEZ, Sonia, 28029 Madrid (ES); BLANCO-APARICIO, Carmen, 28029 Madrid (ES); GARCÍA CAMPOS, FRANCISCO JAVIER, 28029 Madrid (ES)
(74) Representative: Elzaburu S.L.P.

(57) **Abstract**

The present invention relates to a group of compounds of formula (I): 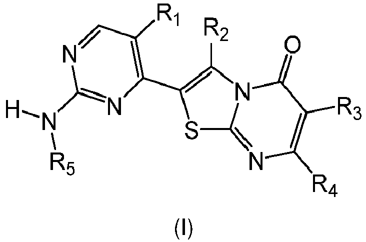 which are inhibitors of the Discoidin Domain Receptors (DDR1/2), whose overexpression is produced in several diseases such as cancer, pulmonary fibrosis, cirrhotic liver, liver fibrosis, lupus nephritis, osteoarthritis, rheumatoid arthritis and atherosclerosis, so the compounds of the invention are useful for the treatment of said diseases.

## Description

### FIELD OF THE INVENTION

The present invention relates to a group of compounds with a a thienopyrimidone core linked to a second pyrimidine moiety. These compounds are inhibitors of the Discoidin Domain Receptors (DDR1/2), whose overexpression is detected in several disease such as cancer, pulmonary fibrosis, osteoarthritis, rheumatoid arthritis or cirrhotic liver, so the compounds of the invention are useful for the treatment these diseases. Therefore, the present invention belongs to the field of pharmacology or medicinal chemistry.

### BACKGROUND OF THE INVENTION

Discoidin domain receptors, DDR1 and DDR2, are two members of collagen receptor family that belong to tyrosine kinase receptor subgroup. DDR1 has 5 isoforms derived from an alternative splicing and DDR2 has only one known form. DDR1 is preferentially expressed in epithelial cells whereas DDR2 is preferentially expressed in connective tissues of the embryonic mesoderm. Both DDRs are expressed early during embryonic development. Indeed, mice lacking DDR1 or DDR2 exhibit major defects in skeletal development, reproduction, inflammation, and cardiovascular system (Leitinger, B., Discoidin domain receptor functions in physiological and pathological conditions. Int Rev Cell Mol Biol 2014, 310, 39-87).

DDRs act as a cellular sensor of the ECM microenvironment and can crosstalk with several transmembrane receptors, such as TGF-β and adhesive receptors, and influence their signaling activity upon collagen deposition. In physiological conditions, DDRs regulate cell polarity, adhesion, migration, and proliferation. In addition to its interaction with collagen fibers, DDR1 has been shown to have other collagen-independent functions, for example, in cell/cell junctions and in collective migration and in leading edge of different cell types (Leitinger, B., Molecular analysis of collagen binding by the human discoidin domain receptors, DDR1 and DDR2. Identification of collagen binding sites in DDR2. J Biol Chem 2003, 278 (19), 16761-9).

The relevance of DDR1 in proliferation, invasion, migration and metastasis has been shown on one hand overexpressing or silencing DDR1 and on the other hand with pharmacological inhibition. For example, hepatocarcinoma, glioblastoma and gastric cancer cells overexpressing DDR1 displayed increased migration and invasion. In prostate cancer cells upregulation of DDR1 expression enhanced cell survival and invasion. Moreover, overexpression of DDR1 protected collagen-treated Hodgkin lymphoma and non small cell lung cancer cells from etoposide treatment. (Rammal, H. et al. Discoidin Domain Receptors: Potential Actors and Targets in Cancer. Front Pharmacol 2016, 7, 55).

On the other hand, silencing of DDR1 in breast cancer, Hodking lymphoma and colon cancer cells abolished the collagen-dependent cell survival and in non small cell lung cancer cells reduced invasiveness in collagen matrices. In addition, in thyroid cancer cells DDR1 silencing induced differentiation markers and reduced EMT and stemness markers through the IGF2/IR-A/DDR1 axis. Moreover, silencing of DDR1 in prostate cancer cell lines led to significant attenuation of EMT and migration via Pyk2 and MKK7 phosphoryation and induction of apoptosis via an increase in Bax expression and suppression of Bcl-2 expression. More importantly, DDR1 knockdown in xenograft models of colorectal, lung and pancreatic cancer reduces tumor growth and in a model of tumor metastasis to bone, lack of DDR1 showed decreased metastatic activity associated with reduced tumor burden and osteolytic lesions, suggesting impaired colonization ability that was highly dependent on the bone microenvironment (Henriet, E. et al., Multitasking discoidin domain receptors are involved in several and specific hallmarks of cancer. Cell Adh Migr 2018, 1-15.).

Several studies demonstrated that DDR1 expression at protein level also promoted the resistance of tumor cells to chemotherapeutic drugs like etoposide in lymphoma, breast and colon cancer cells, cisplastine in ovarian cancer and gefitinib in glioblastoma cells (both at the protein but also at RNA level). Moreover, in a DDR1 and 2 dependent manner, ECM mediates resistance to MAPK pathway inhibitor in BRAF-mutated melanoma. In addition, in different models of glioblastoma, such as stemlike and patient-derived cell cultures, DDR1 favours chemo and radio-resistance, mediated by a pathway involving AKT and mTOR. All these results indicate a potential role of DDR1 in chemoresistance and the possible application of DDR1/2-is in combination with standard of care treatments (Auguste, P et al., Meeting report - first discoidin domain receptors meeting. J Cell Sci 2020, 133(4))

In patients with metastatic CRC DDR1 expression level has been associated with shorter overall survival, and DDR1 phosphorylation was strongly increased in the corresponding metastatic lesions. Moreover, in patients with stage IV CRC DDR1 upregulation is an independent marker of poor prognosis. Furthermore, through tissue microarray in a cohort of renal clear cell carcinoma the expression level of DDR1 has been related with TNM stage, depth of tumor invasion and lymph node metastasis. In addition, in oral squamous cell carcinoma tissues overexpression of DDR1 has been statistically associated with angiolymphatic invasion, perineural invasion, and lymph node metastasis. Also, in human melanoma lesions by IHC, DDR1 has shown high expression that correlates with poor prognosis.

DDR2 mutations and overexpression are detected in different tumor types. Mutations are present in 3-4% of patients with lung squamous cell cancer and have been reported in other cancers at comparable frequencies including lung adenocarcinoma, cervical carcinoma, gastric carcinoma, bladder carcinoma, melanoma, colorectal cancer and head and neck cancer. Moreover, in a small cohort of patients with colorectal cancer, DDR2 levels in tumors are associated with high frequency of peritoneal dissemination and poor prognosis. Moreover, in high-grade serous ovarian cancer higher DDR2 expression has been associated with worse progression free survival.

The relevance of DDR2 in tumorigenesis has been shown mainly silencing or overexpressing DDR2. Down regulation of DDR2 protein levels in different tumor types such as hepatocellular carcinoma, papillary thyroid carcinoma, melanoma and breast cancer reduced tumor growth and/or invasion and migration *in vivo.* In fact, in CAFs, DDR2 controls tumor stiffness by reorganizing the collagen fibers, thereby facilitating integrin activation at the tumor-stromal boundary and favoring cell invasion. On the other hand, overexpression of DDR2 in HNSCC cell lines increases anchorage independent growth, invasion and migration *in vitro* and enhanced migration and metastasis *in vivo.* Overexpression of DDR2 had no effect on proliferation in the absence of collagen, but it led to induce SCLC cell invasion and migration *in vitro* and enhanced lung metastasis *in vivo.* Just one study showed that in breast cancer cells inhibition with WRG-28 (a small molecule that regulates allostericaly DDR2-collagen interactions) inhibited invasion and migration. The data mentioned above clearly pointed to the role of DDR2 in invasion, migration and consequently in metastasis and reinforce its therapeutic potential.

Both DDR1 and 2 have been implicated in immune evasion. It has been suggested that DDR1 promotes breast tumor growth by suppressing anti-tumor immunity and DDR2 has been postulated as an essential regulator of MSI+ colorectal cancer cell immune evasion. Moreover, DDR2 depletion has shown to increase sensitivity to anti-PD-1 treatment compared to monotherapy in murine models of bladder, breast and colon cancer, sarcoma, and melanoma.

DDRs have also been implicated in other pathologies that imply excessive extracellular matrix deposition and inflammatory cell recruitment such as skin hypertrophic scars, pulmonary fibrosis, cirrhotic liver and renal disease. Studies performed in mice lacking DDR1 suggest that promotes fibrosis and inflammation both in kidneys and lung by regulating inflammatory responses and ECM synthesis/deposition. Several publications also suggest that DDR2 could play a pathogenic role in early stages of liver fibrosis, and a different, if not opposing role, in later stages of chronic liver disease. Furthermore, knock-out mice showed that DDR1 has a role in mouse mammary gland development, specifically in stromal epithelial interaction during ductal morphogenesis and that DDR2 acts as an ECM sensor to modulate cell proliferation, required for bone formation. (Jing Guo et al., A patent review of discoidin domain receptor 1 (DDR1) modulators (2014-present) Expert Opin. Ther. Pat. 2020 May;30(5):341-350)

Moreover, DDR1 has been implicated in renal diseases in humans and de novo expression of DDR1 has been detected in glomeruli of patients suffering from lupus nephritis and Goodpasture's syndrome, two diseases targeting this structure. In fact, pharmacological inhibition of DDR1 both using a prophylactic or a therapeutic intervention regime showed positive results in different mouse models of glomerulonephritis.

Atherosclerosis is another disease in which DDR1 has been implicated. Mice lacking DDR1 are protected in a model of vascular injury. DDR2 has also been implicated in osteoarthritis (OA) and rheumatoid arthritis. DDR2 overexpression is one of the key early changes that are common to many different forms of osteoarthritis. Moreover, reduction of DDR2 expression in mouse models of OA attenuates cartilage degeneration.

The contribution of DDRs in tumor progression and especially in metastasis, lung and liver fibrosis, kidney diseases, atherosclerosis, osteoarthritis and rheumatoid arthritis clearly indicates that inhibition of these receptors might represent a promising therapeutic strategy.

Document US10,428,087B2 describes compounds with a pyrimidinone core which are useful for the treatment of cancer by modulating certain kinases. Document WO2010/062038A2 describes a family of compounds with a thienopyrimidine core which inhibit DDR1/2, and thus can be valuably used in the prevention or treatment of diseases such as a cancer.

### DESCRIPTION OF THE INVENTION

The present invention refers to a group of compounds with a thienopyrimidone core, and the method for obtaining them, which are inhibitors of DDR 1/2 in a nanomolar range. Therefore, these compounds are useful for the treatment of certain pathologies where DDR 1/2 are overexpressed, such as cancer, pulmonary fibrosis, cirrhotic liver, renal disease, osteoarthritis and rheumatoid arthritis.

Thus, in a first aspect, the invention refers to a compound of formula (I): 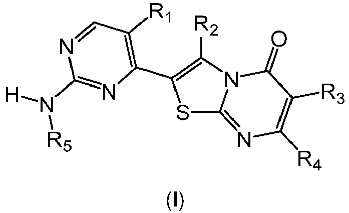 wherein:
R₁ is selected from H, halo, CN, O-alkyl C₁-C₆, alkyl C₁-C₆ optionally substituted by halo;
R₂ is selected from aryl optionally substituted by at least a Q₁ group, heteroaryl optionally substituted by at least a Q₂ group, alkyl C₁-C₆ optionally substituted by at least a Q₃ group, cycloalkyl C₃-C₆ optionally substituted by at least a Q₄ group, heterocycloalkyl C₃-C₆ optionally substituted by at least a Q₅ group, or CONRaRb being Ra and Rb independently selected from H or alkyl C₁-C₆;
R₃ and R₄ are independently selected from H, alkyl C₁-C₆ optionally substituted by at least a Q₆ group, cycloalkyl C₃-C₆ optionally substituted by at least a Q₇ group, heterocycloalkyl C₃-C₆ optionally substituted by at least a Q₈ group, COORc or CONRdRe, being Rc, Rd and Re independently selected from H or alkyl C₁-C₆;
R₅ is selected from aryl optionally substituted by at least a Q₉ group or heteroaryl optionally substituted by at least a Q₁₀ group;
groups Q₁ Q₂ Q₃ Q₄ Q₅ Q₆ Q₇ Q₈ Q₉ Q₁₀ are independently selected from halo, OH, CF₃, CN, SO₂NH₂, C(O)ORx, C(O)NRyRz, being Rx, Ry and Rz independently selected from H or alkyl C₁-C₄, O-alkyl C₁-C₆ optionally substituted by OH or alkyl C₁-C₄, alkyl C₁-C₆ optionally substituted by OH or alkyl C₁-C₄, cycloalkyl C₃-C₆ optionally substituted by OH or alkyl C₁-C₄, heterocycloalkyl C₃-C₆ optionally substituted by alkyl C₁-C₄ which may be substituted by OH;
or a pharmaceutically acceptable solvate or salt thereof.

In the present invention, the term "alkyl" refers to linear or branched hydrocarbonated chain radicals, with between 1 and 6 carbon atoms, preferably between 1 and 4, which bind to the rest of the molecule by means of a single bond, for example, propyl, ethyl, methyl, isopropyl, undecanoyl, heptadecanoyl, octadecanoyl, etc. These alkyl radicals may be optionally substituted in one or more positions by one or more groups, such as cycloalkyl, hydroxyl, amines, amides, oxo, cyano, halogens, aryl, etc.

In the present invention, the term "cycloalkyl" refers to cyclic hydrocarbonated chain radicals, preferably with between 3 and 6 carbon atoms, and more preferably 3, totally or partially saturated, and formed by only carbon and hydrogen atoms. They may be optionally substituted by one or more groups, such as alkyl, halogens, hydroxyl, amines, amides, cyano, etc. Examples of alkyl groups include, but are not limited to, cyclopropyl, cyclobutyl, cyclopentyl or cyclohexyl.

In the present invention, the term "heterocycloalkyl" refers to non-aromatic monocyclic or polycyclic ring comprising carbon and hydrogen atoms and at least one heteroatom (nitrogen, oxygen or sulfur). Preferably is a 4 to 8-member ring with one or more heteroatoms, more preferably is a 6-member ring with one or more heteroatoms. They may be optionally substituted by one or more groups, such as alkyl, halogens, hydroxyl, amines, amides, cyano, etc. Examples of heterocycloalkyl groups include, but are not limited to, piperazinyl, pyrrolidinyl, dioxanyl, morpholinyl, tetrahydrofuranyl, etc.

In the present invention, the term "aryl" refers to single or multiple aromatic rings, which have between 5 and 14 links wherefrom a proton has been eliminated from the ring. Preferably, the aryl group has between 5 and 8 carbon atoms. The aryl radicals may be optionally substituted by one or more substituents, such as alkyl, hydroxyl, amines, amide, cyano, halogens, etc. Examples of aryl groups include, but are not limited to, phenyl, naphthyl, diphenyl, indenyl, phenanthryl, anthracyl, etc.

In the present invention, the term "heteroaryl" refers to an aromatic ring having at least one heteroatom (nitrogen, oxygen or sulfur). The heteroaryl radicals may be optionally substituted by one or more substituents, such as alkyl, hydroxyl, amines, amide, cyano, halogens, etc.Examples of heteroaryl groups include, but are not limited to, pyridyl, furyl, imidazolyl, benzimidazolyl, pyrimidinyl, thienyl, quinolinyl, indolyl, thiazolyl, etc.

"Halo" or "halogen" refers to fluorine, chlorine, bromine or iodine.

Preferably, R₁ is selected from H or O-alkyl C₁-C₄ When R₁ is O-alkyl C₁-C₄, it is more preferably O-CH₃.

Preferably, R₂ is selected from aryl optionally substituted by at least a Q₁ group, alkyl C₁-C₆ optionally substituted by at least a Q₃ group, cycloalkyl C₃-C₆ optionally substituted by at least a Q₄ group, heterocycloalkyl C₃-C₆ optionally substituted by at least a Q₅ group. More preferably, R₂ is phenyl optionally substituted by at least one group selected from OH, halo, O-alkyl C₁-C₄ or CF₃.

Preferably, R₃ and R₄ are independently selected from H, alkyl C₁-C₄ or COORa, being Ra an alkyl C₁-C₄.

Preferably, R₅ is selected from phenyl optionally substituted by at least a Q₉ group, wherein Q₉ is selected from SO₂NH₂ or the following group: 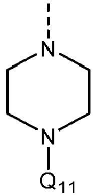 being Q₁₁ an alkyl C₁-C₄ optionally substituted by OH.

Preferably, the compound of formula (I) of the invention is selected from the following list:
- 3-Phenyl-2-(2-phenylamino-pyrimidin-4-yl)-thiazolo[3,2-a]pyrimidin-5-one (29)
- 3-[4-(5-Oxo-3-phenyl-5H-thiazolo[3,2-a]pyrimidin-2-yl)-pyrimidin-2-ylamino]-benzenesulfonamide (30)
- 2-(2-{3-[4-(2-Hydroxy-ethyl)-piperazin-1-yl]-phenylamino}-pyrimidin-4-yl)-3-phenyl-thiazolo[3,2-a]pyrimidin-5-one (31)
- 2-{2-[4-(4-Methyl-piperazin-1-yl)-phenylamino]-pyrimidin-4-yl}-3-phenyl-thiazolo[3,2-a]pyrimidin-5-one (34)
- 3-{4-[3-(2-Chloro-phenyl)-5-oxo-5H-thiazolo[3,2-a]pyrimidin-2-yl]-pyrimidin-2-ylamino}-benzenesulfonamide (37)
- 3-(2-Chloro-phenyl)-2-(2-{3-[4-(2-hydroxy-ethyl)-piperazin-1-yl]-phenylamino}-pyrimidin-4-yl)-thiazolo[3,2-a]pyrimidin-5-one (38)
- 3-(2-Chloro-phenyl)-2-{2-[4-(4-methyl-piperazin-1-yl)-phenylamino]-pyrimidin-4-yl}-thiazolo[3,2-a]pyrimidin-5-one (39)
- 3-{4-[3-(3-Chloro-phenyl)-5-oxo-5H-thiazolo[3,2-a]pyrimidin-2-yl]-pyrimidin-2-ylamino}-benzenesulfonamide (40)
- 3-(3-Chloro-phenyl)-2-(2-{3-[4-(2-hydroxy-ethyl)-piperazin-1-yl]-phenylamino}-pyrimidin-4-yl)-thiazolo[3,2-a]pyrimidin-5-one (41)
- 3-(3-Chloro-phenyl)-2-{2-[4-(4-methyl-piperazin-1-yl)-phenylamino]-pyrimidin-4-yl}-thiazolo[3,2-a]pyrimidin-5-one (42)
- 3-{4-[3-(4-Chloro-phenyl)-5-oxo-5H-thiazolo[3,2-a]pyrimidin-2-yl]-pyrimidin-2-ylamino}-benzenesulfonamide (43)
- 3-(4-Chloro-phenyl)-2-(2-{3-[4-(2-hydroxy-ethyl)-piperazin-1-yl]-phenylamino}-pyrimidin-4-yl)-thiazolo[3,2-a]pyrimidin-5-one (44)
- 3-(4-Chloro-phenyl)-2-{2-[4-(4-methyl-piperazin-1-yl)-phenylamino]-pyrimidin-4-yl}-thiazolo[3,2-a]pyrimidin-5-one (45)
- 3-{4-[3-(2-Methoxy-phenyl)-5-oxo-5H-thiazolo[3,2-a]pyrimidin-2-yl]-pyrimidin-2-ylamino}-benzenesulfonamide (46)
- 2-(2-{3-[4-(2-Hydroxy-ethyl)-piperazin-1-yl]-phenylamino}-pyrimidin-4-yl)-3-(2-methoxy-phenyl)-thiazolo[3,2-a]pyrimidin-5-one (47)
- 3-(2-Methoxy-phenyl)-2-{2-[4-(4-methyl-piperazin-1-yl)-phenylamino]-pyrimidin-4-yl}-thiazolo[3,2-a]pyrimidin-5-one (48)
- 3-{4-[3-(3-Methoxy-phenyl)-5-oxo-5H-thiazolo[3,2-a]pyrimidin-2-yl]-pyrimidin-2-ylamino}-benzenesulfonamide (49)
- 2-(2-{3-[4-(2-Hydroxy-ethyl)-piperazin-1-yl]-phenylamino}-pyrimidin-4-yl)-3-(3-methoxy-phenyl)-thiazolo[3,2-a]pyrimidin-5-one (50)
- 3-(3-Methoxy-phenyl)-2-{2-[4-(4-methyl-piperazin-1-yl)-phenylamino]-pyrimidin-4-yl}-thiazolo[3,2-a]pyrimidin-5-one (51)
- 3-{4-[3-(4-Methoxy-phenyl)-5-oxo-5H-thiazolo[3,2-a]pyrimidin-2-yl]-pyrimidin-2-ylamino}-benzenesulfonamide (52)
- 2-(2-{3-[4-(2-Hydroxy-ethyl)-piperazin-1-yl]-phenylamino}-pyrimidin-4-yl)-3-(4-methoxy-phenyl)-thiazolo[3,2-a]pyrimidin-5-one (53)
- 3-(4-Methoxy-phenyl)-2-{2-[4-(4-methyl-piperazin-1-yl)-phenylamino]-pyrimidin-4-yl}-thiazolo[3,2-a]pyrimidin-5-one (54)
- 3-(2-Hydroxy-phenyl)-2-{2-[4-(4-methyl-piperazin-1-yl)-phenylamino]-pyrimidin-4-yl}-thiazolo[3,2-a]pyrimidin-5-one (55)
- 3-(3-Hydroxy-phenyl)-2-{2-[4-(4-methyl-piperazin-1-yl)-phenylamino]-pyrimidin-4-yl}-thiazolo[3,2-a]pyrimidin-5-one (56)
- 3-(4-Hydroxy-phenyl)-2-{2-[4-(4-methyl-piperazin-1-yl)-phenylamino]-pyrimidin-4-yl}-thiazolo[3,2-a]pyrimidin-5-one (57)
- 3-(4-Fluoro-phenyl)-2-(2-{3-[4-(2-hydroxy-ethyl)-piperazin-1-yl]-phenylamino}-pyrimidin-4-yl)-thiazolo[3,2-a]pyrimidin-5-one (58)
- 3-{4-[3-(2,3-Dichloro-phenyl)-5-oxo-5H-thiazolo[3,2-a]pyrimidin-2-yl]-pyrimidin-2-ylamino}-benzenesulfonamide (59)
- 3-(2,3-Dichloro-phenyl)-2-(2-{3-[4-(2-hydroxy-ethyl)-piperazin-1-yl]-phenylamino}-pyrimidin-4-yl)-thiazolo[3,2-a]pyrimidin-5-one (60)
- 3-{4-[3-(3,4-Dichloro-phenyl)-5-oxo-5H-thiazolo[3,2-a]pyrimidin-2-yl]-pyrimidin-2-ylamino}-benzenesulfonamide (61)
- 3-(3-Chloro-4-methoxy-phenyl)-2-(2-{3-[4-(2-hydroxy-ethyl)-piperazin-1-yl]-phenylamino}-pyrimidin-4-yl)-thiazolo[3,2-a]pyrimidin-5-one (62)
- 3-(4-Fluoro-3-trifluoromethyl-phenyl)-2-(2-{3-[4-(2-hydroxy-ethyl)-piperazin-1-yl]-phenylamino}-pyrimidin-4-yl)-thiazolo[3,2-a]pyrimidin-5-one (63)
- 2-(2-{3-[4-(2-Hydroxy-ethyl)-piperazin-1-yl]-phenylamino}-pyrimidin-4-yl)-3-isopropyl-thiazolo[3,2-a]pyrimidin-5-one (64)
- 3-Cyclopropyl-2-(2-{3-[4-(2-hydroxy-ethyl)-piperazin-1-yl]-phenylamino}-pyrimidin-4-yl)-thiazolo[3,2-a]pyrimidin-5-one (65)
- 2-(2-{3-[4-(2-Hydroxy-ethyl)-piperazin-1-yl]-phenylamino}-pyrimidin-4-yl)-5-oxo-3-phenyl-5H-thiazolo[3,2-a]pyrimidine-6-carboxylic acid ethyl ester (71)
- 2-(2-{3-[4-(2-Hydroxy-ethyl)-piperazin-1-yl]-phenylamino}-pyrimidin-4-yl)-7-methyl-3-phenyl-thiazolo[3,2-a]pyrimidin-5-one (72)
- 2-(2-{3-[4-(2-Hydroxy-ethyl)-piperazin-1-yl]-phenylamino}-pyrimidin-4-yl)-7-isopropyl-3-phenyl-thiazolo[3,2-a]pyrimidin-5-one (73)
- 7-Ethyl-2-(2-{3-[4-(2-hydroxy-ethyl)-piperazin-1-yl]-phenylamino}-pyrimidin-4-yl)-3-phenyl-thiazolo[3,2-a]pyrimidin-5-one (74)
- 2-(2-{3-[4-(2-Hydroxy-ethyl)-piperazin-1-yl]-phenylamino}-pyrimidin-4-yl)-6,7-dimethyl-3-phenyl-thiazolo[3,2-a]pyrimidin-5-one (75)
- 2-(2-{3-[4-(2-Hydroxy-ethyl)-piperazin-1-yl]-phenylamino}-pyrimidin-4-yl)-6-isopropyl-7-methyl-3-phenyl-thiazolo[3,2-a]pyrimidin-5-one (76)
- 6-Ethyl-2-(2-{3-[4-(2-hydroxy-ethyl)-piperazin-1-yl]-phenylamino}-pyrimidin-4-yl)-7-methyl-3-phenyl-thiazolo[3,2-a]pyrimidin-5-one (77)

More preferably, the compound of formula (I) of the invention is selected from the following list:
- 3-Phenyl-2-(2-phenylamino-pyrimidin-4-yl)-thiazolo[3,2-a]pyrimidin-5-one (29)
- 3-[4-(5-Oxo-3-phenyl-5H-thiazolo[3,2-a]pyrimidin-2-yl)-pyrimidin-2-ylamino]-benzenesulfonamide (30)
- 2-(2-{3-[4-(2-Hydroxy-ethyl)-piperazin-1-yl]-phenylamino}-pyrimidin-4-yl)-3-phenyl-thiazolo[3,2-a]pyrimidin-5-one (31)
- 2-{2-[4-(4-Methyl-piperazin-1-yl)-phenylamino]-pyrimidin-4-yl}-3-phenyl-thiazolo[3,2-a]pyrimidin-5-one (34)
- 3-{4-[3-(2-Chloro-phenyl)-5-oxo-5H-thiazolo[3,2-a]pyrimidin-2-yl]-pyrimidin-2-ylamino}-benzenesulfonamide (37)
- 3-(2-Chloro-phenyl)-2-(2-{3-[4-(2-hydroxy-ethyl)-piperazin-1-yl]-phenylamino}-pyrimidin-4-yl)-thiazolo[3,2-a]pyrimidin-5-one (38)
- 3-(2-Chloro-phenyl)-2-{2-[4-(4-methyl-piperazin-1-yl)-phenylamino]-pyrimidin-4-yl}-thiazolo[3,2-a]pyrimidin-5-one (39)
- 3-{4-[3-(3-Chloro-phenyl)-5-oxo-5H-thiazolo[3,2-a]pyrimidin-2-yl]-pyrimidin-2-ylamino}-benzenesulfonamide (40)
- 3-(3-Chloro-phenyl)-2-(2-{3-[4-(2-hydroxy-ethyl)-piperazin-1-yl]-phenylamino}-pyrimidin-4-yl)-thiazolo[3,2-a]pyrimidin-5-one (41)
- 3-(3-Chloro-phenyl)-2-{2-[4-(4-methyl-piperazin-1-yl)-phenylamino]-pyrimidin-4-yl}-thiazolo[3,2-a]pyrimidin-5-one (42)
- 3-{4-[3-(4-Chloro-phenyl)-5-oxo-5H-thiazolo[3,2-a]pyrimidin-2-yl]-pyrimidin-2-ylamino}-benzenesulfonamide (43)
- 3-(4-Chloro-phenyl)-2-(2-{3-[4-(2-hydroxy-ethyl)-piperazin-1-yl]-phenylamino}-pyrimidin-4-yl)-thiazolo[3,2-a]pyrimidin-5-one (44)
- 3-(4-Chloro-phenyl)-2-{2-[4-(4-methyl-piperazin-1-yl)-phenylamino]-pyrimidin-4-yl}-thiazolo[3,2-a]pyrimidin-5-one (45)
- 3-{4-[3-(2-Methoxy-phenyl)-5-oxo-5H-thiazolo[3,2-a]pyrimidin-2-yl]-pyrimidin-2-ylamino}-benzenesulfonamide (46)
- 2-(2-{3-[4-(2-Hydroxy-ethyl)-piperazin-1-yl]-phenylamino}-pyrimidin-4-yl)-3-(2-methoxy-phenyl)-thiazolo[3,2-a]pyrimidin-5-one (47)
- 3-(2-Methoxy-phenyl)-2-{2-[4-(4-methyl-piperazin-1-yl)-phenylamino]-pyrimidin-4-yl}-thiazolo[3,2-a]pyrimidin-5-one (48)
- 3-{4-[3-(3-Methoxy-phenyl)-5-oxo-5H-thiazolo[3,2-a]pyrimidin-2-yl]-pyrimidin-2-ylamino}-benzenesulfonamide (49)
- 2-(2-{3-[4-(2-Hydroxy-ethyl)-piperazin-1-yl]-phenylamino}-pyrimidin-4-yl)-3-(3-methoxy-phenyl)-thiazolo[3,2-a]pyrimidin-5-one (50)
- 3-(3-Methoxy-phenyl)-2-{2-[4-(4-methyl-piperazin-1-yl)-phenylamino]-pyrimidin-4-yl}-thiazolo[3,2-a]pyrimidin-5-one (51)
- 3-{4-[3-(4-Methoxy-phenyl)-5-oxo-5H-thiazolo[3,2-a]pyrimidin-2-yl]-pyrimidin-2-ylamino}-benzenesulfonamide (52)
- 2-(2-{3-[4-(2-Hydroxy-ethyl)-piperazin-1-yl]-phenylamino}-pyrimidin-4-yl)-3-(4-methoxy-phenyl)-thiazolo[3,2-a]pyrimidin-5-one (53)
- 3-(4-Methoxy-phenyl)-2-{2-[4-(4-methyl-piperazin-1-yl)-phenylamino]-pyrimidin-4-yl}-thiazolo[3,2-a]pyrimidin-5-one (54)
- 3-(2-Hydroxy-phenyl)-2-{2-[4-(4-methyl-piperazin-1-yl)-phenylamino]-pyrimidin-4-yl}-thiazolo[3,2-a]pyrimidin-5-one (55)
- 3-(3-Hydroxy-phenyl)-2-{2-[4-(4-methyl-piperazin-1-yl)-phenylamino]-pyrimidin-4-yl}-thiazolo[3,2-a]pyrimidin-5-one (56)
- 3-(4-Hydroxy-phenyl)-2-{2-[4-(4-methyl-piperazin-1-yl)-phenylamino]-pyrimidin-4-yl}-thiazolo[3,2-a]pyrimidin-5-one (57)
- 3-(4-Fluoro-phenyl)-2-(2-{3-[4-(2-hydroxy-ethyl)-piperazin-1-yl]-phenylamino}-pyrimidin-4-yl)-thiazolo[3,2-a]pyrimidin-5-one (58)
- 3-{4-[3-(2,3-Dichloro-phenyl)-5-oxo-5H-thiazolo[3,2-a]pyrimidin-2-yl]-pyrimidin-2-ylamino}-benzenesulfonamide (59)
- 3-(2,3-Dichloro-phenyl)-2-(2-{3-[4-(2-hydroxy-ethyl)-piperazin-1-yl]-phenylamino}-pyrimidin-4-yl)-thiazolo[3,2-a]pyrimidin-5-one (60)
- 3-{4-[3-(3,4-Dichloro-phenyl)-5-oxo-5H-thiazolo[3,2-a]pyrimidin-2-yl]-pyrimidin-2-ylamino}-benzenesulfonamide (61)
- 3-(3-Chloro-4-methoxy-phenyl)-2-(2-{3-[4-(2-hydroxy-ethyl)-piperazin-1-yl]-phenylamino}-pyrimidin-4-yl)-thiazolo[3,2-a]pyrimidin-5-one (62)
- 2-(2-{3-[4-(2-Hydroxy-ethyl)-piperazin-1-yl]-phenylamino}-pyrimidin-4-yl)-3-isopropyl-thiazolo[3,2-a]pyrimidin-5-one (64)
- 2-(2-{3-[4-(2-Hydroxy-ethyl)-piperazin-1-yl]-phenylamino}-pyrimidin-4-yl)-7-methyl-3-phenyl-thiazolo[3,2-a]pyrimidin-5-one (72)
- 2-(2-{3-[4-(2-Hydroxy-ethyl)-piperazin-1-yl]-phenylamino}-pyrimidin-4-yl)-7-isopropyl-3-phenyl-thiazolo[3,2-a]pyrimidin-5-one (73)
- 7-Ethyl-2-(2-{3-[4-(2-hydroxy-ethyl)-piperazin-1-yl]-phenylamino}-pyrimidin-4-yl)-3-phenyl-thiazolo[3,2-a]pyrimidin-5-one (74)
- 2-(2-{3-[4-(2-Hydroxy-ethyl)-piperazin-1-yl]-phenylamino}-pyrimidin-4-yl)-6,7-dimethyl-3-phenyl-thiazolo[3,2-a]pyrimidin-5-one (75)
- 2-(2-{3-[4-(2-Hydroxy-ethyl)-piperazin-1-yl]-phenylamino}-pyrimidin-4-yl)-6-isopropyl-7-methyl-3-phenyl-thiazolo[3,2-a]pyrimidin-5-one (76)
- 6-Ethyl-2-(2-{3-[4-(2-hydroxy-ethyl)-piperazin-1-yl]-phenylamino}-pyrimidin-4-yl)-7-methyl-3-phenyl-thiazolo[3,2-a]pyrimidin-5-one (77)

The compounds of the present invention represented by the formula (I), and more specifically, the specific compounds belonging to this previously described general formula may include isomers, depending on the presence of multiple bonds (for example, Z, E), including optic isomers or enantiomers, depending on the presence of chiral centres. The individual isomers, enantiomers or diastereoisomers and the mixtures thereof fall within the scope of the present invention. The individual enantiomers or diastereoisomers, as well as their mixtures, can be separated by conventional techniques.

The compounds of the invention may be in crystalline form as free compounds or in solvate form, intending both forms to be within the scope of the present invention. In this sense, the term "solvate", as used herein, includes both pharmaceutically acceptable solvates, in other words, solvates of the compound of formula (I) which can be used to produce a medicament, as well as pharmaceutically unacceptable solvates, which can be useful to produce pharmaceutically acceptable solvates or salts. The nature of the pharmaceutically acceptable solvate is not critical on condition that it is pharmaceutically acceptable. In a particular embodiment, the solvate is a hydrate. The solvates can be obtained by conventional solvation methods known to technicians in the art.

For therapeutic application, the compounds of formula (I), their salts or solvates, will come preferably in a pharmaceutically acceptable or substantially pure form, in other words, having a pharmaceutically acceptable level of purity excluding standard drugs such as diluents and carriers, and not including material considered toxic at standard dose levels. The purity levels for the active principle are preferably higher than 50%, more preferably higher than 70%, more preferably higher than 90%. In a preferred embodiment, they are higher than 95% of the compound of formula (I), or the salts or solvates thereof

Another aspect of the invention refers to a compound of formula (I) as described above for use as a medicament.

Another aspect of the invention refers to a compound of formula (I) as described above for use in the prevention or treatment of a disease in which DDR1/2 is overexpressed, wherein said disease is selected from cancer, pulmonary fibrosis, cirrhotic liver, liver fibrosis, lupus nephritis, Goodpasture's syndrome, glomerulonephritis, osteoarthritis, rheumatoid arthritis and atherosclerosis.

Preferably, the cancer is selected from hepatocarcinoma, glioblastoma, gastric cancer, prostate cancer, Hodgkin lymphoma, lung cancer, breast cancer, colorectal cancer, pancreatic cancer, melanoma, ovarian cancer, papillary thyroid carcinoma, bladder cancer or sarcoma.

Another aspect of the invention refers to a composition comprising a compound of formula (I) as described above and a pharmaceutically acceptable excipient, diluent or carrier. Preferably, said composition further comprises an antitumoral compound which may be any chemotherapeutic agent authorized to be used in humans or in the process of clinical trials and/or pending of said authorization. Examples of said chemotherapeutic agents may be found, for example, in patent EP3341376.

The term "excipients, diluent or carriers" relates to molecular entities or substances through which the active ingredient is administered. Such pharmaceutical excipients, diluents or carriers can be sterile liquids, such as water and oils, including those of petroleum, animal, vegetable or synthetic origin, such as peanut oil, soybean oil, mineral oil, sesame oil and similar oils, excipients, disintegrating agents, humectants or dilutes. Suitable pharmaceutical excipients and carriers are known in the art by a skilled person.

The compounds of formula (I) for therapeutic use are prepared in solid form or aqueous suspension, in a pharmaceutically acceptable diluent. These preparations may be administered via any appropriate route of administration, wherefore said preparation will be formulated in the suitable pharmaceutical form for the selected route of administration. In a particular embodiment, the compound of formula (I) provided by this invention is administered orally, topically, rectally or parenterally (including subcutaneously, intraperitoneally, intradermally, intramuscularly, intravenously, etc.). A review of the different pharmaceutical forms for administering medicaments and the excipients required to obtain them can be found in the standard Pharmacopoeias of Europe and the US.

The compounds described in the present invention, their pharmaceutically acceptable salts, and solvates, as well as the pharmaceutical compositions containing them can be used in conjunction with other additional drugs in order to provide a combination therapy. Said additional drugs may form part of the same pharmaceutical composition or, alternatively, may be provided in the form of a separate composition for administration simultaneously or not with the administration of the pharmaceutical composition comprising a compound of formula (I), or pharmaceutically acceptable solvate or salt thereof.

Another additional aspect of the present invention refers to a method for treating a disease in which DDR 1/2 is/are overexpressed, comprising the administration of a therapeutically effective amount of a compound of formula (I) as described above.

In the sense used in this description, the expression "therapeutically effective amount" refers to the quantity of agent or compound capable of having an effect on the levels of phospho DDR 1/2 in primary cultured cells, calculated to produce the required effect in vivo and, in general, will be determined, among other aspects, by the inherent properties of the compounds, including the age, state of the patient, severity of the alteration or disorder, and route and frequency of administration. In general, the therapeutically effective amount of the compound of formula (I) to be administered will depend, among other factors, on the individual who is to be treated, the severity of the disease suffered by the individual, the selected form of administration, etc. For this reason, the doses mentioned in this invention must be considered solely as guides for the skilled person, who must adjust the doses according to the aforementioned variables. However, a compound of formula (I) may be administered one or more times a day, for example 1, 2, 3 or 4 times a day, in a typical total daily quantity comprised between 0.1 and 1,000 mg/kg body weight/day, preferably 10 mg/kg body mass/day.

The compound of the invention is compatible for use in protocols wherein the compounds of formula (I) or their mixtures are used alone or in combination with other treatments or medical procedures, such as radiotherapy or immunotherapy.

Another aspect of the invention refers to a method to obtain a compound of formula (I) as described above which comprises reacting a compound of formula (II): 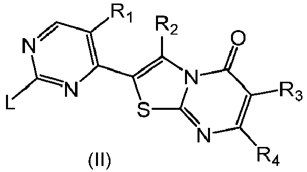 being R₁ to R₄ as defined above and L a leaving group, preferably Cl, with a compound of formula (III):

NH₂-R₅ (III)

being R₅ as defined above.

**Table 1. Chemical structure of the preferred compounds of formula (I)**

| **Compound Nr. and structure** | **Compound Nr. and structure** |
|---|---|
| 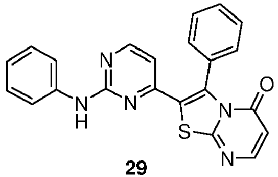 | 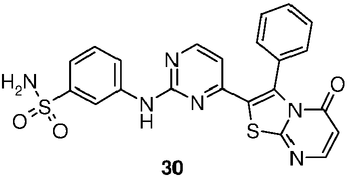 |
| 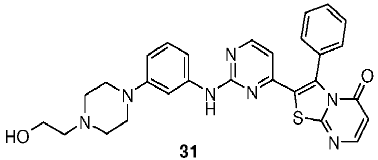 | 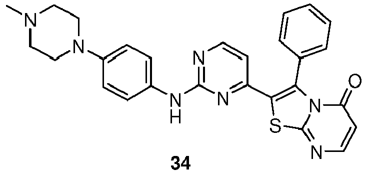 |
| 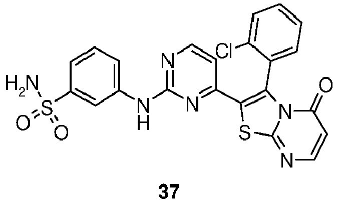 | 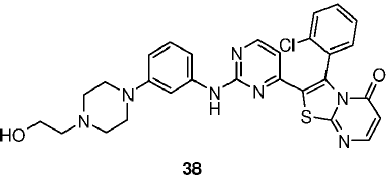 |
| 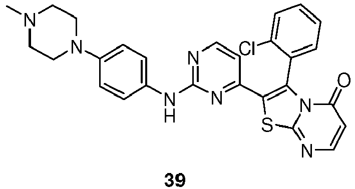 | 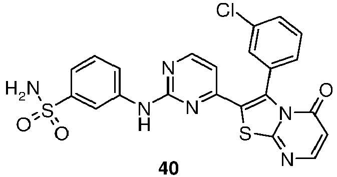 |
| 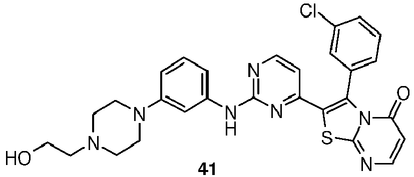 | 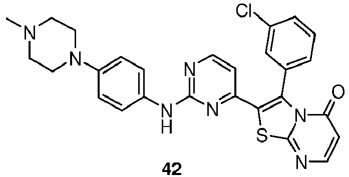 |
| 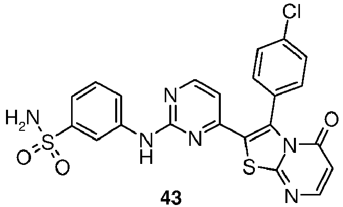 | 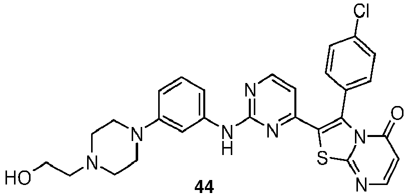 |
| 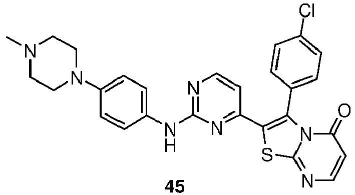 | 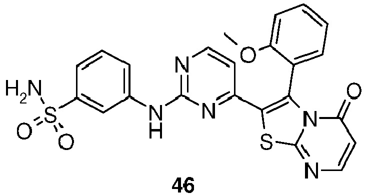 |
| 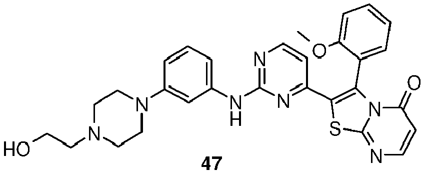 | 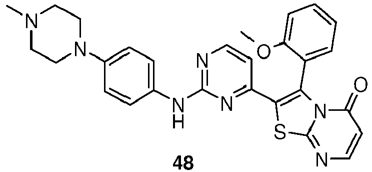 |
| 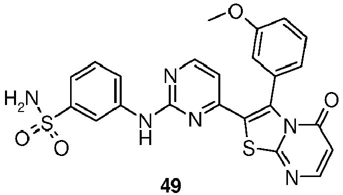 | 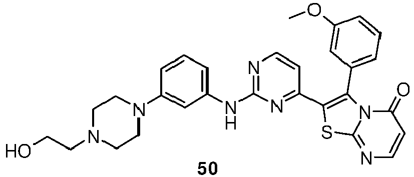 |
| 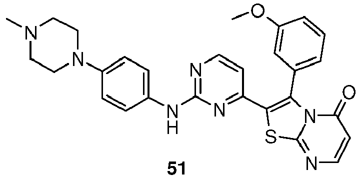 | 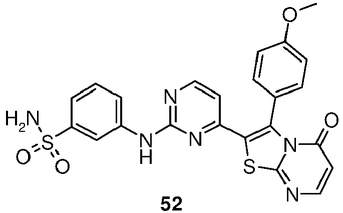 |
| 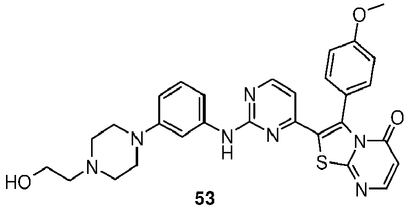 | 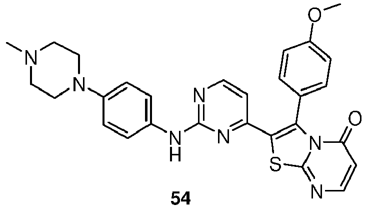 |
| 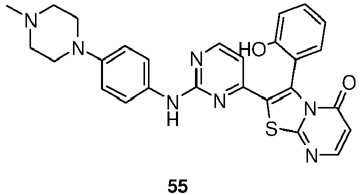 | 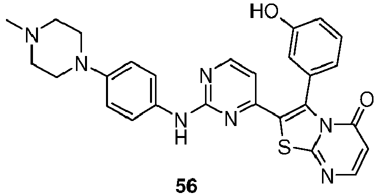 |
| 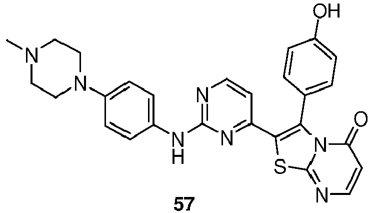 | 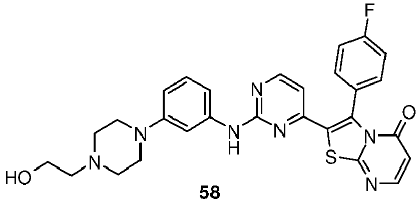 |
| 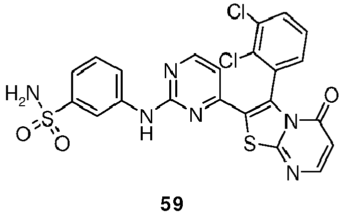 | 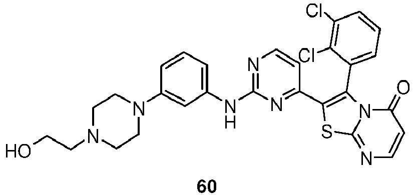 |
| 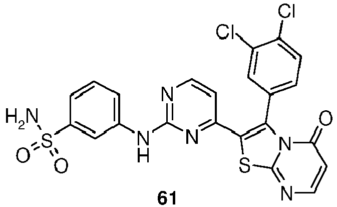 | 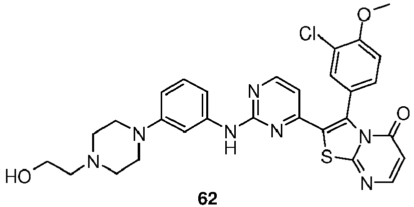 |
| 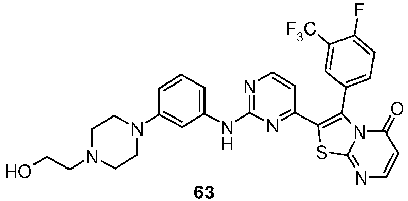 | 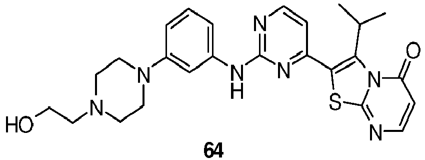 |
| 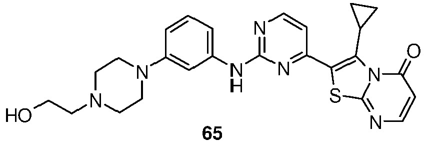 | 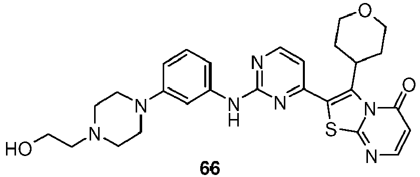 |
| 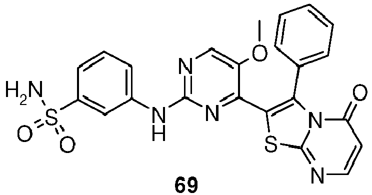 | 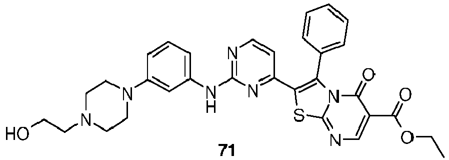 |
| 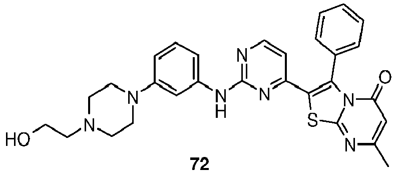 | 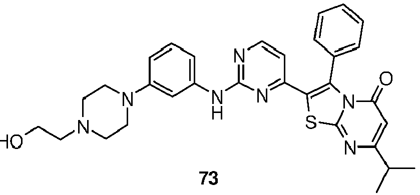 |
| 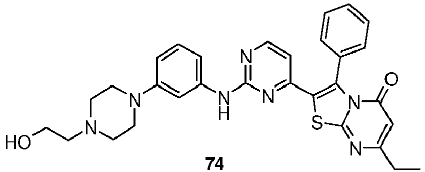 | 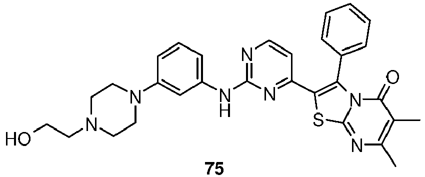 |
| 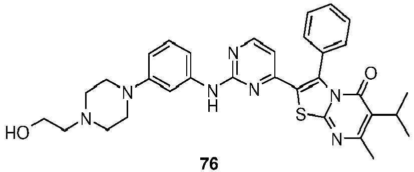 | 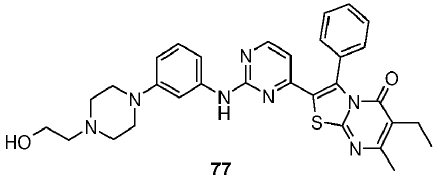 |

All individual features (e.g. preferred features) mentioned herein may be taken in isolation or in combination with any other feature (including a preferred feature) mentioned herein (hence, preferred features may be taken in conjunction with other preferred features, or independently of them).

Throughout the description and the claims, the word "comprises" and the variants thereof are not intended to exclude other technical characteristics, additives, components or steps. For persons skilled in the art, other objects, advantages and characteristics of the invention will arise partly from the description and partly from the practice of the invention. The following examples and figures are provided for illustrative purposes and are not intended to limit the scope of this invention.

### EXAMPLES

### A. General methods for the synthesis of the compounds of the invention

Method A: A mixture of **24a-r** or **25a-g** (1 eq), respective aniline (R₅-NH₂) (1.2 eq), Pd₂(dba)₃ (0.1 eq), Cs₂CO₃ (3 eq) and BINAP (0.2 eq) in dioxane (0.1 mmol/mL) was heated in a sealed tube at 110 ºC for 6 h under Ar atmosphere. Solvent was removed at reduced pressure. Water and NH₄Cl (sat) were added until pH 7-8. Aqueous phase was extracted with CHCl₃:iPrOH (1:1). Organic layers were mixed, dried with Na₂SO₄ and filtered. Solvent was removed and desired product was purified by flash chromatography. Method B: To a solution of **48, 51** or **54** (1 eq) in DCM (0.04 mmol/mL) was added (CH₃)₂S·BF₃ (10 eq) at 0 ºC. The reaction mixture was stirred at 0 ºC for 4h 30 min. Then, NaHCO₃ (sat) was added and the mixture was extracted with CHCl₃:iPrOH (1:1). The organic layers were dried with Na₂SO₄, filtered and evaporated. The residue was purified by flash chromatography.

General Chemical Procedure: Chemicals were purchased from Aldrich Chemical Company Ltd., Apollo Scientific Ltd and TCI Europe N.V. unless otherwise stated, commercial chemicals and solvents were used without further purification. Anhydrous DMF, DCM, toluene, MeOH, dioxane, acetonitrile, DPE, TEA, DIPEA, DIPA and DME were purchased from Aldrich in Sure Seal^{™} bottle and kept under nitrogen. All reactions were monitored by thin layer chromatography (TLC) and HPLC-MS. Thin layer chromatography (TLC) was performed on 20 mm precoated plates of silica gel (Merck, silica gel 60F254); visualization was achieved using ultraviolet light (254 nm). The HPLC measurements were performed using a HP 1100 from Agilent Technologies comprising a pump (binary) with degasser, an autosampler, a column oven, a diode-array detector (DAD) and a column Gemini-NX C18 (100 x 2.0 mm; 5 mm particle size). Eluent A, water with 0.1% formic acid; eluent B: acetonitrile with 0.1% formic acid. Gradient 5-100% of B within 8 min (LC-MS 1 method) or 4 min (LC-MS 2 method) or gradient 0-30% of B within 8 min (LC-MS 3 method) at 50 ºC, DAD. Flow from the column was split to a MS spectrometer. The MS detector was configured with an electrospray ionization source or API/APCI. Nitrogen was used as the nebulizer gas. Data acquisition was performed with ChemStation LC/MSD quad, software. Purities of all reported compounds were greater than 90% based on HPLC chromatograms obtained on an Agilent HP 1100 LCMS system. All final compounds were purified to have purity higher than 90% by reverse phase high performance liquid chromatography (HPLC), or normal phase by flash chromatography in silica gel employing Biotage apparatus medium pressure liquid chromatography system SP4 or Isolera^{™} Prime, using ExtraBond Flash SLL with spacer SI (cartridges of 12 g, 20 g, 40 g, 80 g or 120 g). Proton (¹H) and carbon (¹³C) NMR spectra were recorded on Bruker Avance II 300 and/or Bruker Avance II 700 spectrometers using DMSO-*d*6 or CDCl₃-*d1* as solvent. Chemical shifts are expressed in parts per million (ppm) (δ relative to residual solvent peak for 1H). Multiplicities are indicated by s (singlet), d (doublet), t (triplet), q (quartet), m (multiplet), br (broad) or combination thereof.

### Example 1. 3-Phenyl-2-(2-phenylamino-pyrimidin-4-yl)-thiazolo[3,2-a]pyrimidin-5-one (29)

From 2-(2-chloro-pyrimidin-4-yl)-3-phenyl-thiazolo [3,2-a]pyrimidin-5-one (**24a**) (1 eq, 15 mg, 0.044 mmol) and aniline (1.5 eq, 6 µL, 0.066 mmol) as starting materials following synthetic method A. Purified by flash chromatography (DCM:MeOH 99:1 to 92:8). Product **29** was a white solid. (5 mg, Yield = 29%). LC-MS 1 method. Rt= 4.474 min, m/z = 398 [M+H]⁺. ¹H-NMR (300MHz, DMSO-*d*6): δ 9.84 (s, 1H), 8.22 (d, J = 5.3 Hz, 1H), 7.96 (d, J = 6.5 Hz, 1H), 7.73 (d, J = 7.6 Hz, 2H), 7.60-7.46 (m, 5H), 7.35 (t, J = 7.6, 2H), 7.01 (t, J = 7.3 Hz, 1H), 6.12 (d, J = 6.5 Hz, 1H), 5.63 (d, J = 5.3 Hz, 1H). ¹³C-NMR (75MHz, DMSO-d6): δ 162.66, 159.37, 158.87, 158.60, 155.81, 152.38, 139.87, 137.39, 131.54, 129.54, 129.48 (2C), 128.61 (2C), 128.47 (2C), 125.22, 122.14, 119.29 (2C), 108.08, 107.60.

### Example 2. 3-[4-(5-Oxo-3-phenyl-5H-thiazolo[3,2-a]pyrimidin-2-yl)-pyrimidin-2-ylamino]-benzenesulfonamide (30)

From 2-(2-chloro-pyrimidin-4-yl)-3-phenyl-thiazolo[3,2-a]pyrimidin-5-one (**24a**) (1 eq, 50 mg, 0.147 mmol) and 3-aminobenzenesulfonamide (1.5 eq, 38 mg, 0.220 mmol) as starting materials following synthetic method A. Purified by flash chromatography (DCM:MeOH 98.5:1.5 to 90:10). Product **30** was a yellow solid. (3.5 mg, Yield = 5%). LC-MS 1 method. Rt= 4.478 min, m/z = 477 [M+H]⁺. ¹H-NMR (300MHz, DMSO-*d*6): δ 10.17 (s, 1H), 8.30 (t, J = 1.8 Hz, 1H), 8.27 (d, J = 5.3 Hz, 1H), 7.97 (d, J = 6.5 Hz, 1H), 7.90 (ddd, J = 8.1, 2.1, 1.1 Hz, 1H), 7.61-7.52 (m, 5H), 7.53-7.42 (m, 2H), 7.35 (s, 2H), 6.12 (d, J = 6.5 Hz, 1H), 5.70 (d, J = 5.3 Hz, 1H). ¹³C-NMR (75MHz, DMSO-*d*6): δ 162.81, 159.22, 159.01, 158.70, 156.15, 152.35, 144.64, 140.34, 137.65, 131.53, 129.70, 129.49 (2C), 129.25, 128.53 (2C), 124.99, 122.07, 119.03, 116.00, 108.77, 107.45.

### Example 3. 2-(2-{3-[4-(2-Hydroxy-ethyl)-piperazin-1-yl]-phenylamino}-pyrimidin-4-yl)-3-phenyl-thiazolo[3,2-a]pyrimidin-5-one (31)

From 2-(2-chloro-pyrimidin-4-yl)-3-phenyl-thiazolo[3,2-a]pyrimidin-5-one (**24a**) (1 eq, 20 mg, 0.059 mmol) and 2-[4-(3-amino-phenyl)-piperazin-1-yl]-ethanol (**28b**) (1.5 eq, 19 mg, 0.088 mmol) as starting materials following synthetic method A. Purified by reverse phase HPLC. Product **31** was a yellow solid. (5 mg, Yield = 16%). LC-MS 1 method. Rt= 3.397 min, m/z = 526 [M+H]⁺. ¹H-NMR (300MHz, DMSO-*d*6): δ 9.65 (s, 1H), 8.20 (d, J = 5.26 Hz, 1H), 7.96 (d, J = 6.47 Hz, 1H), 7.60-7.45 (m, 5H), 7.35 (br s, 1H), 7.21-7.13 (m, 2H), 6.60 (br d, J = 6.98 Hz, 1H), 6.11 (d, J = 6.48 Hz, 1H), 5.62 (d, J = 5.24 Hz, 1H), 3.54 (br, 2H), 3.16 (m, 4H), 2.59 (m, 4H), 2.45 (t, J = 6.27 Hz, 2H).

### Example 4. 2-{2-[4-(4-Methyl-piperazin-1-yl)-phenylamino]-pyrimidin-4-yl}-3-phenyl-thiazolo[3,2-a]pyrimidin-5-one (34)

From 2-(2-chloro-pyrimidin-4-yl)-3-phenyl-thiazolo[3,2-a]pyrimidin-5-one (**24a**) (1 eq, 60 mg, 0.176 mmol) and 4-(4-methylpiperazino)aniline (1.5 eq, 51 mg, 0.264 mmol) as starting materials following synthetic method A. Purified by reverse phase HPLC. Product **34** was a white solid. (8 mg, Yield = 9.2%). LC-MS 1 method. Rt= 4.232 min, m/z = 496 [M+H]⁺. ¹H-NMR (300MHz, DMSO-*d*6): δ 9.58 (s, 1H), 8.14 (d, J = 5.2 Hz, 1H), 7.95 (d, J = 6.5 Hz, 1H), 7.60-7.44 (m, 7H), 6.94 (d, J = 9.0 Hz, 2H), 6.10 (d, J = 6.5 Hz, 1H), 5.54 (d, J = 5.2 Hz, 1H), 3.10 (br, 4H), 2.47 (br, 4H), 2.24 (s, 3H). ¹³C-NMR (75MHz, DMSO-*d*6): δ 162.61, 159.52, 158.87, 158.69,152.35, 146.60, 137.11, 131.80, 131.56, 131.49, 129.61, 129.49 (2C), 128.35 (2C), 125.45, 120.60 (2C), 115.75 (2C), 107.57, 107.17, 54.64 (2C), 48.62 (2C), 45.69.

### Example 5. 3-[4-[3-(2-Chloro-phenyl)-5-oxo-5H-thiazolo[3,2-a]pyrimidin-2-yl]-pyrimidin-2-ylamino}-benzenesulfonamide (37)

From 3-(2-chloro-phenyl)-2-(2-chloro-pyrimidin-4-yl)-thiazolo[3,2-a]pyrimidin-5-one (**24b**) (1 eq, 69 mg, 0.184 mmol) and 3-aminobenzenesulfonamide (1.5 eq, 47 mg, 0.276 mmol) as starting materials following synthetic method A. Purified by flash chromatography (DCM:MeOH 98:2 to 92:8). Product **37** was a pale yellow solid. (19 mg, Yield = 20%). LC-MS 1 method. Rt= 4.806 min, m/z = 511 [M+H]⁺. ¹H-NMR (300MHz, DMSO-*d*6): δ 10.23 (s, 1H), 8.35 (d, J = 5.3 Hz, 1H), 8.32 (t, J = 1.8 Hz, 1H), 8.01 (d, J = 6.5 Hz, 1H), 7.92-7.85 (m, 1H), 7.72-7.54 (m, 4H), 7.54-7.45 (m, 2H), 7.36 (s, 2H), 6.17 (d, J = 6.5 Hz, 1H), 5.75 (d, J = 5.2 Hz, 1H). ¹³C-NMR (75MHz, DMSO-*d*6): δ 162.36, 159.62, 159.31, 158.40, 155.37, 152.70, 144.69, 140.17, 133.76, 133.09, 131.83, 131.41, 130.61, 129.31, 129.25, 127.59, 126.19, 122.15, 119.21, 116.05, 108.01, 107.34.

### Example 6. 3-(2-Chloro-phenyl)-2-(2-{3-[4-(2-hydroxy-ethyl)-piperazin-1-yl]-phenylamino}-pyrimidin-4-yl)-thiazolo[3,2-a]pyrimidin-5-one (38)

From 3-(2-chloro-phenyl)-2-(2-chloro-pyrimidin-4-yl)-thiazolo[3,2-a]pyrimidin-5-one (**24b**) (1 eq, 180 mg, 0.480 mmol) and 2-[4-(3-amino-phenyl)-piperazin-1-yl]-ethanol (**28b**) (1.3 eq, 138 mg, 0.624 mmol) as starting materials following synthetic method A. Purified by flash chromatography (DCM:MeOH 98:2 to 93:7). Product **38** was a white solid. (17 mg, Yield = 6.3%). LC-MS 1 method. Rt= 3.435 min, m/z = 560 [M+H]⁺. ¹H-NMR (300MHz, DMSO-*d*6): δ 9.72 (s, 1H), 8.28 (d, J = 5.2 Hz, 1H), 8.01 (d, J = 6.5 Hz, 1H), 7.70-7.58 (m, 3H), 7.49 (td, J = 7.4, 1.4 Hz, 1H), 7.34 (s, 1H), 7.19 (m, 2H), 6.67-6.56 (m, 1H), 6.17 (d, J = 6.5 Hz, 1H), 5.66 (d, J = 5.2 Hz, 1H), 4.50 (br, 1H), 3.55 (dd, J = 10.8, 5.7 Hz, 2H), 3.17 (br, 4H), 2.61 (br, 4H), 2.46 (br, 2H). ¹³C-NMR (75MHz, DMSO-*d*6): δ 162.26, 159.56, 159.50, 158.38, 155.06, 152.73, 151.48, 140.39, 133.54, 133.11, 131.81, 131.43, 130.63, 129.22, 129.00, 127.57, 126.34, 110.46, 109.77, 107.41, 107.17, 106.68, 60.22, 58.42, 53.12 (2C), 48.36 (2C).

### Example 7. 3-(2-Chloro-phenyl)-2-{2-[4-(4-methyl-piperazin-1-yl)-phenylamino]-pyrimidin-4-yl}-thiazolo[3,2-a]pyrimidin-5-one (39)

From 3-(2-chloro-phenyl)-2-(2-chloro-pyrimidin-4-yl)-thiazolo[3,2-a]pyrimidin-5-one (**24b**) (1 eq, 116 mg, 0.309 mmol) and 4-(4-methylpiperazino)aniline (1.3 eq, 77 mg, 0.402 mmol) as starting materials following synthetic method A. Purified by flash chromatography (DCM:MeOH 98:2 to 85:15). Product **39** was a white solid. (8 mg, Yield = 5%). LC-MS 1 method. Rt= 3.200 min, m/z = 530 [M+H]⁺. ¹H-NMR (300MHz, DMSO-*d*6): δ 9.64 (s, 1H), 8.22 (d, J = 5.2 Hz, 1H), 8.00 (d, J = 6.5 Hz, 1H), 7.75 - 7.43 (m, 6H), 6.94 (d, J = 9.0 Hz, 2H), 6.16 (d, J = 6.5 Hz, 1H), 5.60 (d, J = 5.2 Hz, 1H), 3.16-3.03 (m, 4H), 2.48-2.42 (m, 4H), 2.22 (s, 3H). ¹³C-NMR (75MHz, DMSO-*d*6): δ 162.30, 159.57, 159.43, 158.37, 155.05, 152.65, 146.72, 133.35, 133.10, 131.76, 131.52,131.43, 130.65, 129.18, 127.52, 126.63, 120.73 (2C), 115.71 (2C), 107.34, 106.51, 54.71 (2C), 48.68 (2C), 45.80.

### Example 8. 3-{4-[3-(3-Chloro-phenyl)-5-oxo-5H-thiazolo[3,2-a]pyrimidin-2-yl]-pyrimidin-2-ylamino}-benzenesulfonamide (40)

From 3-(3-chloro-phenyl)-2-(2-chloro-pyrimidin-4-yl)-thiazolo[3,2-a]pyrimidin-5-one (**24c**) (1 eq, 94 mg, 0.251 mmol) and 3-aminobenzenesulfonamide (1.5 eq, 65 mg, 0.376 mmol) as starting materials following synthetic method A. Purified by flash chromatography (DCM:MeOH 98:2 to 92:8). Product **40** was a pale yellow solid. (20 mg, Yield = 16%). LC-MS 1 method. Rt= 4.945 min, m/z = 511 [M+H]⁺.
¹H-NMR (300MHz, DMSO-*d*6): δ 10.20 (s, 1H), 8.35 (d, J = 5.3 Hz, 1H), 8.31 (t, J = 1.8 Hz, 1H), 7.98 (d, J = 6.5 Hz, 1H), 7.93-7.85 (m, 1H), 7.76-7.70 (m, 1H), 7.69-7.60 (m, 1H), 7.60-7.51 (m, 3H), 7.51-7.44 (m, 1H), 7.35 (s, 2H), 6.14 (d, J = 6.5 Hz, 1H), 5.79 (d, J = 5.3 Hz, 1H). ¹³C-NMR (75MHz, DMSO-*d*6): δ 162.56, 159.34, 159.30, 158.70, 155.83, 152.51, 144.69, 140.24, 135.64, 133.50, 132.95, 130.25, 129.68, 129.41, 129.31, 128.41, 125.57, 122.04, 119.13, 115.99, 108.73, 107.49.

### Example 9. 3-(3-Chloro-phenyl)-2-(2-{3-[4-(2-hydroxy-ethyl)-piperazin-1-yl]-phenylamino}-pyrimidin-4-yl)-thiazolo[3,2-a]pyrimidin-5-one (41)

From 3-(3-chloro-phenyl)-2-(2-chloro-pyrimidin-4-yl)-thiazolo[3,2-a]pyrimidin-5-one (**24c**) (1 eq, 150 mg, 0.400 mmol) and 2-[4-(3-amino-phenyl)-piperazin-1-yl]-ethanol (**28b**) (1.3 eq, 115 mg, 0.520 mmol) as starting materials following synthetic method A. Purified by flash chromatography (DCM:MeOH 100:0 to 85:15). Product **41** was a white solid. (33 mg, Yield = 15%). LC-MS 1 method. Rt= 3.510 min, m/z = 560 [M+H]⁺. ¹H-NMR (300MHz, DMSO-*d*6): δ 9.69 (s, 1H), 8.27 (d, J = 5.2 Hz, 1H), 7.97 (d, J = 6.5 Hz, 1H), 7.76-7.68 (m, 1H), 7.63 (m, 1H), 7.59-7.49 (m, 2H), 7.35 (s, 1H), 7.22 - 7.12 (m, 2H), 6.68-6.54 (m, 1H), 6.13 (d, J = 6.5 Hz, 1H), 5.70 (d, J = 5.2 Hz, 1H), 4.48 (br, 1H), 3.55 (dd, J = 10.8, 5.8 Hz, 2H), 3.16 (s, 4H), 2.59 (s, 4H), 2.45 (d, J = 6.2 Hz, 2H). ¹³C-NMR (75MHz, DMSO-*d*6): δ 162.45, 159.51, 159.16, 158.66, 155.45, 152.49, 151.51, 140.47, 135.37, 133.50, 132.91, 130.21, 129.62, 129.40, 128.98, 128.41, 125.76, 110.33, 109.65, 107.87, 107.52, 106.55, 60.28, 58.50, 53.18 (2C), 48.43 (2C).

### Example 10. 3-(3-Chloro-phenyl)-2-{2-[4-(4-methyl-piperazin-1-yl)-phenylamino]-pyrimidin-4-yl}-thiazolo[3,2-a]pyrimidin-5-one (42)

From 3-(3-chloro-phenyl)-2-(2-chloro-pyrimidin-4-yl)-thiazolo[3,2-a]pyrimidin-5-one (**24c**) (1 eq, 150 mg, 0.400 mmol) and 4-(4-methylpiperazino)aniline (1.3 eq, 99 mg, 0.520 mmol) as starting materials following synthetic method A. Purified by flash chromatography (DCM:MeOH 100:0 to 85:15). Product **42** was a white solid. (37 mg, Yield = 18%). LC-MS 1 method. Rt= 4.367 min, m/z = 530 [M+H]⁺. ¹H-NMR (300MHz, DMSO-*d*6): δ 9.61 (s, 1H), 8.21 (d, J = 5.2 Hz, 1H), 7.96 (d, J = 6.5 Hz, 1H), 7.70 (d, J = 1.1 Hz, 1H), 7.67-7.59 (m, 1H), 7.57-7.49 (m, 4H), 6.93 (d, J = 9.0 Hz, 2H), 6.12 (d, J = 6.5 Hz, 1H), 5.63 (d, J = 5.2 Hz, 1H), 3.15-3.02 (m, 4H), 2.48-2.43 (m, 4H), 2.23 (s, 3H). ¹³C-NMR (75MHz, DMSO-*d*6): δ 162.49, 159.52, 159.09, 158.66, 155.42, 152.41, 146.61, 135.17, 133.52, 132.87, 131.65, 130.16, 129.57, 129.40, 128.41, 126.07, 120.61 (2C), 115.72 (2C), 107.47, 107.24, 54.69 (2C), 48.67 (2C), 45.77.

### Example 11. 3-{4-[3-(4-Chloro-phenyl)-5-oxo-5H-thiazolo[3,2-a]pyrimidin-2-yl]-pyrimidin-2-ylamino}-benzenesulfonamide (43)

From 3-(4-chloro-phenyl)-2-(2-chloro-pyrimidin-4-yl)-thiazolo[3,2-a]pyrimidin-5-one (**24d**) (1 eq, 68 mg, 0.181 mmol) and 3-aminobenzenesulfonamide (1.2 eq, 37 mg, 0.217 mmol) as starting materials following synthetic method A. Purified by flash chromatography (DCM:MeOH 100:0 to 97:3). Product **43** was a pale yellow solid. (5 mg, Yield = 5.4%). LC-MS 1 method. Rt= 5.005 min, m/z = 511 [M+H]⁺. ¹H-NMR (300MHz, DMSO-*d*6): δ 10.19 (s, 1H), 8.35 (d, J = 5.3 Hz, 1H), 8.30 (t, J = 1.9 Hz, 1H), 7.97 (d, J = 6.5 Hz, 1H), 7.89 (ddd, J = 8.0, 2.1, 1.2 Hz, 1H), 7.60 (m, 4H), 7.54 (t, J = 7.9 Hz, 1H), 7.50-7.45 (m, 1H), 7.35 (s, 2H), 6.13 (d, J = 6.5 Hz, 1H), 5.83 (d, J = 5.3 Hz, 1H). ¹³C-NMR (75MHz, DMSO-*d*6): δ 162.64, 159.36, 159.31, 158.79, 155.90, 152.47,144.70, 140.26, 136.20, 134.43, 131.53 (2C), 130.42, 129.31, 128.54 (2C), 125.40, 122.07, 119.14, 115.99, 108.82, 107.51.

### Example 12. 3-(4-Chloro-phenyl)-2-(2-{3-[4-(2-hydroxy-ethyl)-piperazin-1-yl]-phenylamino}-pyrimidin-4-yl)-thiazolo[3,2-a]pyrimidin-5-one (44)

From 3-(4-chloro-phenyl)-2-(2-chloro-pyrimidin-4-yl)-thiazolo[3,2-a]pyrimidin-5-one (**24d**) (1 eq, 70 mg, 0.187 mmol) and 2-[4-(3-amino-phenyl)-piperazin-1-yl]-ethanol (**28b**) (1.2 eq, 50 mg, 0.224 mmol) as starting materials following synthetic method A. Purified by flash chromatography (DCM:MeOH 100:0 to 85:15). Product **44** was a white solid. (29 mg, Yield = 28%). LC-MS 1 method. Rt= 3.545 min, m/z = 560 [M+H]⁺. ¹H-NMR (300MHz, DMSO-*d*6): δ 9.67 (s, 1H), 8.26 (d, J = 5.2 Hz, 1H), 7.96 (d, J = 6.5 Hz, 1H), 7.58 (s, 4H), 7.35 (s, 1H), 7.16 (q, J = 8.4 Hz, 2H), 6.60 (d, J = 7.3 Hz, 1H), 6.11 (d, J = 6.5 Hz, 1H), 5.72 (d, J = 5.2 Hz, 1H), 4.46 (t, J = 5.0 Hz, 1H), 3.54 (dd, J = 11.0, 5.9 Hz, 2H), 3.21-3.09 (m, 4H), 2.63-2.54 (m, 4H), 2.45 (t, J = 6.2 Hz, 2H). ¹³C-NMR (75MHz, DMSO-*d*6): δ 162.49, 159.47, 159.13, 158.70, 155.48, 152.42, 151.51, 140.47, 135.86, 134.36, 131.51 (2C), 130.40, 128.94, 128.46 (2C), 125.59, 110.27, 109.59, 107.92, 107.51, 106.50, 60.32, 58.54, 53.20 (2C), 48.47 (2C).

### Example 13. 3-(4-Chloro-phenyl)-2-{2-[4-(4-methyl-piperazin-1-yl)-phenylamino]-pyrimidin-4-yl}-thiazolo[3,2-a]pyrimidin-5-one (45)

From 3-(4-chloro-phenyl)-2-(2-chloro-pyrimidin-4-yl)-thiazolo[3,2-a]pyrimidin-5-one (**24d**) (1 eq, 70 mg, 0.187 mmol) and 4-(4-methylpiperazino)aniline (1.2 eq, 43 mg, 0.224 mmol) as starting materials following synthetic method A. Purified by flash chromatography (DCM:MeOH 97:3 to 85:15). Product **45** was a white solid. (18 mg, Yield = 18%). LC-MS 1 method. Rt= 3.496 min, m/z = 530 [M+H]⁺. ¹H-NMR (300MHz, DMSO-*d*6): δ 9.59 (s, 1H), 8.21 (d, J = 5.2 Hz, 1H), 7.95 (d, J = 6.5 Hz, 1H), 7.57 (s, 4H), 7.54 (d, J = 9.1 Hz, 2H), 6.93 (d, J = 9.1 Hz, 2H), 6.11 (d, J = 6.5 Hz, 1H), 5.67 (d, J = 5.2 Hz, 1H), 3.15-3.03 (m, 4H), 2.47 (m, 4H), 2.24 (s, 3H). ¹³C-NMR (75MHz, DMSO-*d*6): δ 162.65, 162.56, 159.51, 159.09, 158.74, 153.33, 152.38, 146.59, 134.36, 131.69 (2C), 131.53, 130.43, 128.45 (2C), 120.63 (2C), 115.75 (2C), 114.80, 107.49, 107.34, 54.67 (2C), 48.64 (2C), 45.71.

### Example 14. 3-{4-[3-(2-Methoxy-phenyl)-5-oxo-5H-thiazolo[3,2-a]pyrimidin-2-yl]-pyrimidin-2-ylamino}-benzenesulfonamide (46)

From 2-(2-chloro-pyrimidin-4-yl)-3-(2-methoxy-phenyl)-thiazolo[3,2-a]pyrimidin-5-one (**24e**) (1 eq, 78 mg, 0.210 mmol) and 3-aminobenzenesulfonamide (1.3 eq, 47 mg, 0.273 mmol) as starting materials following synthetic method A. Purified by flash chromatography (DCM:MeOH 100:0 to 97:3). Product **46** was a pale yellow solid. (28 mg, Yield = 26%). LC-MS 1 method. Rt= 4.640 min, m/z = 507 [M+H]+. ¹H-NMR (300MHz, DMSO-*d*6): δ 10.18 (s, 1H), 8.34-8.28 (m, 2H), 7.97 (d, J = 6.5 Hz, 1H), 7.89 (ddd, J = 8.0, 2.1, 1.2 Hz, 1H), 7.61-7.51 (m, 2H), 7.50-7.45 (m, 1H), 7.39 (dd, J = 7.5, 1.7 Hz, 1H), 7.35 (s, 2H), 7.19 (d, J = 8.0 Hz, 1H), 7.07 (td, J = 7.5, 0.9 Hz, 1H), 6.13 (d, J = 6.5 Hz, 1H), 5.88 (d, J = 5.3 Hz, 1H), 3.73 (s, 3H). ¹³C-NMR (75MHz, DMSO-*d*6): δ 162.63, 159.27, 159.22, 158.45, 157.39, 156.01, 152.37, 144.69, 140.29, 134.67, 131.82, 130.38, 129.31, 124.95, 122.08, 120.68, 120.14, 119.11, 115.99, 111.42, 108.38, 107.42, 55.84.

### Example 15. 2-(2-{3-[4-(2-Hydroxy-ethyl)-piperazin-1-yl]-phenylamino}-pyrimidin-4-yl)-3-(2-methoxy-phenyl)-thiazolo[3,2-a]pyrimidin-5-one (47)

From 2-(2-chloro-pyrimidin-4-yl)-3-(2-methoxy-phenyl)-thiazolo[3,2-a]pyrimidin-5-one (**24e**) (1 eq, 100 mg, 0.270 mmol) and 2-[4-(3-amino-phenyl)-piperazin-1-yl]-ethanol (**28b**) (1.3 eq, 78 mg, 0.351 mmol) as starting materials following synthetic method A. Purified by flash chromatography (DCM:MeOH 100:0 to 85:15). Product **47** was a white solid. (25 mg, Yield = 17%). LC-MS 1 method. Rt= 3.307 min, m/z = 556 [M+H]⁺. ¹H-NMR (300MHz, DMSO-*d*6): δ 9.66 (s, 1H), 8.24 (d, J = 5.2 Hz, 1H), 7.96 (d, J = 6.5 Hz, 1H), 7.62-7.50 (m, 1H), 7.44-7.31 (m, 2H), 7.25-7.11 (m, 3H), 7.06 (t, J = 7.5 Hz, 1H), 6.60 (dt, J = 3.2, 2.0 Hz, 1H), 6.12 (d, J = 6.5 Hz, 1H), 5.80 (d, J = 5.2 Hz, 1H), 4.46 (s, 1H), 3.72 (s, 3H), 3.54 (d, J = 2.9 Hz, 2H), 3.22-3.09 (m, 4H), 2.64-2.54 (m, 4H), 2.45 (t, J = 6.3 Hz, 2H). ¹³C-NMR (75MHz, DMSO-*d*6): δ 162.51, 159.50, 159.08, 158.39, 157.39, 155.66, 152.35, 151.52, 140.48, 134.41, 131.75, 130.37, 128.95, 125.07, 120.62, 120.15, 111.37, 110.33, 109.61, 107.51, 107.44, 106.57, 60.31, 58.53, 55.81, 53.19 (2C), 48.46 (2C).

### Example 16. 3-(2-Methoxy-phenyl)-2-{2-[4-(4-methyl-piperazin-1-yl)-phenylamino]-pyrimidin-4-yl}-thiazolo[3,2-a]pyrimidin-5-one (48)

From 2-(2-chloro-pyrimidin-4-yl)-3-(2-methoxy-phenyl)-thiazolo[3,2-a]pyrimidin-5-one (**24e**) (1 eq, 100 mg, 0.270 mmol) and 4-(4-methylpiperazino)aniline (1.3 eq, 67 mg, 0.351 mmol) as starting materials following synthetic method A. Purified by flash chromatography (DCM:MeOH 97:3 to 0:100). Product **48** was a white solid. (30 mg, Yield = 21%). LC-MS 1 method. Rt= 3.296 min, m/z = 526 [M+H]⁺. ¹H-NMR (300MHz, DMSO-*d*6): δ 9.61 (s, 1H), 8.18 (d, J = 5.2 Hz, 1H), 7.95 (d, J = 6.5 Hz, 1H), 7.61-7.52 (m, 3H), 7.37 (dd, J = 7.5, 1.7 Hz, 1H), 7.17 (d, J = 8.1 Hz, 1H), 7.05 (td, J = 7.5, 0.7 Hz, 1H), 6.96 (d, J = 9.1 Hz, 2H), 6.11 (d, J = 6.5 Hz, 1H), 5.74 (d, J = 5.3 Hz, 1H), 3.72 (s, 3H), 3.22 (s, 4H), 2.80 (s, 4H), 2.45 (s, 3H). ¹³C-NMR (75MHz, DMSO-*d*6): δ 162.55, 159.49, 159.02, 158.41, 157.38, 155.62, 152.28, 145.93, 134.24, 132.13, 131.72, 130.38, 125.38, 120.63 (2C), 120.60, 120.18, 116.02 (2C), 111.37, 107.40, 106.97, 55.82, 53.66 (2C), 47.63 (2C), 44.22.

### Example 17. 3-{4-[3-(3-Methoxy-phenyl)-5-oxo-5H-thiazolo[3,2-a]pyrimidin-2-yl]-pyrimidin-2-ylamino}-benzenesulfonamide (49)

From 2-(2-chloro-pyrimidin-4-yl)-3-(3-methoxy-phenyl)-thiazolo[3,2-a]pyrimidin-5-one (**24f**) (1 eq, 120 mg, 0.324 mmol) and 3-aminobenzenesulfonamide (1.5 eq, 84 mg, 0.485 mmol) as starting materials following synthetic method A. Purified by flash chromatography (Cyclohexane:(EtOAc:EtOH 3:1, 2% NH₄OH) 90:10 to 30:70). Product **49** was a pale yellow solid. (19 mg, Yield = 12%). LC-MS 1 method. Rt= 4.688 min, m/z = 507 [M+H]⁺. ¹H-NMR (300MHz, DMSO-*d*6): δ 10.15 (s, 1H), 8.30 (d, J = 5.2 Hz, 2H), 7.95 (d, J = 6.5 Hz, 1H), 7.93-7.87 (m, 1H), 7.54 (t, J = 7.9 Hz, 1H), 7.44 (dd, J = 16.6, 8.5 Hz, 2H), 7.34 (s, 2H), 7.18-7.07 (m, 3H), 6.12 (d, J = 6.5 Hz, 1H), 5.82 (d, J = 5.3 Hz, 1H), 3.77 (s, 3H). ¹³C-NMR (75MHz, DMSO-*d*6): δ 162.67, 159.22, 159.12, 159.06, 158.52, 156.04, 152.30, 144.68, 140.29, 137.24, 132.64, 129.62, 129.27, 124.93, 122.02, 121.71, 119.07, 115.97, 115.31, 114.99, 108.74, 107.51, 55.30.

### Example 18. 2-(2-{3-[4-(2-Hydroxy-ethyl)-piperazin-1-yl]-phenylamino}-pyrimidin-4-yl)-3-(3-methoxy-phenyl)-thiazolo[3,2-a]pyrimidin-5-one (50)

From 2-(2-chloro-pyrimidin-4-yl)-3-(3-methoxy-phenyl)-thiazolo[3,2-a]pyrimidin-5-one (**24f**) (1 eq, 120 mg, 0.324 mmol) and 2-[4-(3-amino-phenyl)-piperazin-1-yl]-ethanol (**28b**) (1.3 eq, 86 mg, 0.388 mmol) as starting materials following synthetic method A. Purified by flash chromatography (Cyclohexane:(EtOAc:EtOH 3:1 2% NH₄OH) 70:30 to 0:100). Product **50** was a white solid. (38 mg, Yield = 21%). LC-MS 1 method. Rt= 3.370 min, m/z = 556 [M+H]⁺. ¹H-NMR (300MHz, DMSO-*d*6): δ 9.63 (s, 1H), 8.22 (d, J = 5.2 Hz, 1H), 7.95 (d, J = 6.5 Hz, 1H), 7.41 (t, J = 7.8 Hz, 1H), 7.35 (s, 1H), 7.22-7.07 (m, 5H), 6.59 (d, J = 7.6 Hz, 1H), 6.10 (d, J = 6.5 Hz, 1H), 5.73 (d, J = 5.2 Hz, 1H), 4.45 (br, 1H), 3.77 (s, 3H), 3.55 (s, 2H), 3.16 (s, 4H), 2.58 (s, 4H), 2.45 (t, J = 6.2 Hz, 2H). ¹³C-NMR (75MHz, DMSO-*d*6): δ 162.55, 159.44, 159.08, 158.92, 158.46, 155.65, 152.29, 151.52, 140.51, 136.95, 132.65, 129.55, 128.92, 125.12, 121.73, 115.25, 114.98, 110.27, 109.54, 107.87, 107.53, 106.50, 60.33, 58.56, 55.28, 53.20 (2C), 48.49 (2C).

### Example 19. 3-(3-Methoxy-phenyl)-2-{2-[4-(4-methyl-piperazin-1-yl)-phenylamino]-pyrimidin-4-yl}-thiazolo[3,2-a]pyrimidin-5-one (51)

From 2-(2-chloro-pyrimidin-4-yl)-3-(3-methoxy-phenyl)-thiazolo[3,2-a]pyrimidin-5-one (**24f**) (1 eq, 100 mg, 0.270 mmol) and 4-(4-methylpiperazino)aniline (1.2 eq, 62 mg, 0.324 mmol) as starting materials following synthetic method A. Purified by flash chromatography (DCM:MeOH 99.5:0.5 to 91.5:8.5). Product **51** was a white solid. (55 mg, Yield = 39%). LC-MS 1 method. Rt= 3.218 min, m/z = 526 [M+H]⁺. ¹H-NMR (300MHz, DMSO-*d*6): δ 9.58 (s, 1H), 8.16 (dd, J = 5.2, 1.2 Hz, 1H), 7.93 (dd, J = 6.5, 1.0 Hz, 1H), 7.54 (d, J = 9.0 Hz, 2H), 7.41 (t, J = 7.8 Hz, 1H), 7.15-7.06 (m, 3H), 6.92 (d, J = 8.9 Hz, 2H), 6.09 (dd, J = 6.5, 0.7 Hz, 1H), 5.66 (d, J = 5.2 Hz, 1H), 3.76 (s, 3H), 3.16-3.03 (m, 4H), 2.51 (m, 4H), 2.25 (s, 3H). ¹³C-NMR (75MHz, DMSO-*d*6): δ 162.61, 159.46, 159.08, 158.90, 158.50, 155.62, 152.25, 146.49, 136.79, 132.70, 131.81, 129.56, 125.47, 121.75 (2C), 120.57, 115.77 (2C), 115.22, 115.00, 107.52, 107.27, 55.30, 54.59 (2C), 48.57 (2C), 45.60.

### Example 20. 3-{4-[3-(4-Methoxy-phenyl)-5-oxo-5H-thiazolo[3,2-a]pyrimidin-2-yl]-pyrimidin-2-ylamino}-benzenesulfonamide (52)

From 2-(2-chloro-pyrimidin-4-yl)-3-(4-methoxy-phenyl)-thiazolo[3,2-a]pyrimidin-5-one (**24g**) (1 eq, 98 mg, 0.264 mmol) and 3-aminobenzenesulfonamide (1.5 eq, 68 mg, 0.396 mmol) as starting materials following synthetic method A. Purified by flash chromatography (Cyclohexane:(EtOAc:EtOH 3:1, 2% NH₄OH) 70:30 to 0:100). Product **52** was a pale yellow solid. (3 mg, Yield = 2%). LC-MS 1 method. Rt= 4.695 min, m/z = 507 [M+H]⁺. ¹H-NMR (300MHz, DMSO-*d*6): δ 10.14 (s, 1H), 8.31 (d, J = 5.3 Hz, 1H), 8.29 (d, J = 1.8 Hz, 1H), 7.94 (d, J = 6.5 Hz, 1H), 7.93-7.88 (m, 1H), 7.54 (t, J = 7.9 Hz, 1H), 7.49-7.47 (m, 1H), 7.47 - 7.42 (m, 2H), 7.34 (s, 2H), 7.06 (d, J = 8.8 Hz, 2H), 6.10 (d, J = 6.5 Hz, 1H), 5.83 (d, J = 5.3 Hz, 1H), 3.86 (s, 3H). ¹³C-NMR (75MHz, DMSO-*d*6): δ 162.72, 160.07, 159.23, 159.01, 158.78, 156.32, 152.24, 144.67, 140.31, 137.81, 130.99 (2C), 129.27, 124.98, 123.28, 122.01, 119.05, 115.96, 113.85 (2C), 108.82, 107.57, 55.27.

### Example 21. 2-(2-{3-[4-(2-Hydroxy-ethyl)-piperazin-1-yl]-phenylamino}-pyrimidin-4-yl)-3-(4-methoxy-phenyl)-thiazolo[3,2-a]pyrimidin-5-one (53)

From 2-(2-chloro-pyrimidin-4-yl)-3-(4-methoxy-phenyl)-thiazolo[3,2-a]pyrimidin-5-one (**24g**) (1 eq, 110 mg, 0.297 mmol) and 2-[4-(3-amino-phenyl)-piperazin-1-yl]-ethanol (**28b**) (1.5 eq, 98 mg, 0.445 mmol) as starting materials following synthetic method A. Purified by flash chromatography (EtOAc:(EtOAc:MeOH:NH₃ 7N 8:1:1) 80:20 to 0:100). Product **53** was a white solid. (22 mg, Yield = 13%). LC-MS 1 method. Rt= 3.360 min, m/z = 556 [M+H]⁺. ¹H-NMR (300MHz, DMSO-*d*6): δ 9.65 (s, 1H), 8.23 (d, J = 5.2 Hz, 1H), 7.94 (d, J = 6.4 Hz, 1H), 7.44 (d, J = 8.5 Hz, 2H), 7.35 (s, 1H), 7.27-7.09 (m, 2H), 7.04 (d, J = 8.6 Hz, 2H), 6.59 (d, J = 7.2 Hz, 1H), 6.09 (d, J = 6.4 Hz, 1H), 5.73 (d, J = 5.2 Hz, 1H), 3.85 (s, 3H), 3.54 (m, 2H), 3.16 (br, 4H), 2.57 (br, 4H), 2.44 (m, 2H). ¹³C-NMR (75MHz, DMSO-*d*6): δ 162.60, 160.03, 159.46, 158.90, 158.75, 155.94, 152.25, 151.54, 140.54, 137.54, 130.99 (2C), 128.95, 125.16, 123.31, 113.81 (2C), 110.26, 109.54, 107.93, 107.62, 106.49, 60.34, 58.56, 55.27, 53.22 (2C), 48.50 (2C).

### Example 22. 3-(4-Methoxy-phenyl)-2-{2-[4-(4-methyl-piperazin-1-yl)-phenylamino]-pyrimidin-4-yl}-thiazolo[3,2-a]pyrimidin-5-one (54)

From 2-(2-chloro-pyrimidin-4-yl)-3-(4-methoxy-phenyl)-thiazolo[3,2-a]pyrimidin-5-one (**24g**) (1 eq, 70 mg, 0.189 mmol) and 4-(4-methylpiperazino)aniline (1.5 eq, 54 mg, 0.283 mmol) as starting materials following synthetic method A. Purified by flash chromatography (DCM:MeOH 100:0 to 92:8). Product **54** was a white solid. (44 mg, Yield = 44%). LC-MS 1 method. Rt= 3.227 min, m/z = 526 [M+H]⁺. ¹H-NMR (300MHz, DMSO-*d*6): δ 9.57 (s, 1H), 8.18 (d, J = 5.2 Hz, 1H), 7.93 (d, J = 6.5 Hz, 1H), 7.55 (d, J = 9.0 Hz, 2H), 7.43 (d, J = 8.7 Hz, 2H), 7.04 (d, J = 8.7 Hz, 2H), 6.94 (d, J = 9.0 Hz, 2H), 6.09 (d, J = 6.5 Hz, 1H), 5.68 (d, J = 5.2 Hz, 1H), 3.85 (s, 3H), 3.10 (br, 4H), 2.48 (br, 4H), 2.25 (s, 3H). ¹³C-NMR (75MHz, DMSO-*d*6): δ 162.64, 160.00, 159.46, 158.84, 158.74,155.91, 152.17, 146.50, 137.35, 131.78, 130.98 (2C), 125.45, 123.33, 120.56 (2C), 115.75 (2C), 113.76 (2C), 107.56, 107.31, 55.24, 54.62 (2C), 48.60 (2C), 45.65.

### Example 23. 3-(2-Hydroxy-phenyl)-2-{2-[4-(4-methyl-piperazin-1-yl)-phenylamino]-pyrimidin-4-yl}-thiazolo[3,2-a]pyrimidin-5-one (55)

From 3-(2-Methoxy-phenyl)-2-{2-[4-(4-methyl-piperazin-1-yl)-phenylamino]-pyrimidin-4-yl}-thiazolo[3,2-a]pyrimidin-5-one (**48**) (1 eq, 29 mg, 0.055 mmol) following synthetic method B. Purified by flash chromatography (Cyclohexane:(EtOAc:EtOH 3:1, 4% NH₄OH) 60:40 to 10:90). Product **55** was a white solid. (7 mg, Yield = 25%). LC-MS 1 method. Rt= 3.028 min, m/z = 512 [M+H]⁺. ¹H-NMR (300MHz, DMSO-*d*6): δ 9.83 (s, 1H), 9.57 (s, 1H), 8.20 (d, J = 5.2 Hz, 1H), 7.95 (d, J = 6.5 Hz, 1H), 7.55 (d, J = 9.0 Hz, 2H), 7.42-7.34 (m, 1H), 7.29 (dd, J = 7.6, 1.6 Hz, 1H), 6.92 (m, 4H), 6.11 (d, J = 6.5 Hz, 1H), 5.82 (d, J = 5.2 Hz, 1H), 3.15-3.03 (m, 4H), 2.48-2.42 (m, 4H), 2.22 (s, 3H). ¹³C-NMR (75MHz, DMSO-*d*6): δ 162.76, 159.50, 158.99, 158.50, 155.81, 155.47, 152.21, 146.62, 134.87, 131.72, 131.30, 130.36, 125.44, 120.61 (2C), 119.12, 118.64, 115.74, 115.64 (2C), 107.40, 106.92, 54.74 (2C), 48.73 (2C), 45.83.

### Example 24. 3-(3-Hydroxy-phenyl)-2-{2-[4-(4-methyl-piperazin-1-yl)-phenylamino]-pyrimidin-4-yl}-thiazolo[3,2-a]pyrimidin-5-one (56)

From 3-(3-Methoxy-phenyl)-2-{2-[4-(4-methyl-piperazin-1-yl)-phenylamino]-pyrimidin-4-yl}-thiazolo[3,2-a]pyrimidin-5-one (**51**) (1 eq, 33 mg, 0.063 mmol) following synthetic method B. Purified by flash chromatography (DCM:MeOH 97:3 to 85:15). Product **56** was a white solid. (23 mg, Yield = 72%). LC-MS 1 method. Rt= 2.998 min, m/z = 512 [M+H]⁺. ¹H-NMR (300MHz, DMSO-*d*6): δ 9.72 (s, 1H), 9.56 (s, 1H), 8.19 (d, J = 5.2 Hz, 1H), 7.93 (d, J = 6.5 Hz, 1H), 7.54 (d, J = 9.1 Hz, 2H), 7.29 (t, J = 7.8 Hz, 1H), 6.92 (m, 5H), 6.10 (d, J = 6.5 Hz, 1H), 5.71 (d, J = 5.2 Hz, 1H), 3.13 - 3.07 (m, 4H), 2.52 (s, 4H), 2.26 (s, 3H). ¹³C-NMR (75MHz, DMSO-*d*6): δ 162.68, 159.48, 158.87, 158.49, 157.22, 155.74, 152.24, 146.50, 137.15, 132.51, 131.81, 129.56, 125.17, 120.60 (2C), 120.11, 116.67, 116.24, 115.79 (2C), 107.50, 107.33, 54.60 (2C), 48.58 (2C), 45.60.

### Example 25. 3-(4-Hydroxy-phenyl)-2-{2-[4-(4-methyl-piperazin-1-yl)-phenylamino]-pyrimidin-4-yl}-thiazolo[3,2-a]pyrimidin-5-one (57)

From 3-(4-Methoxy-phenyl)-2-{2-[4-(4-methyl-piperazin-1-yl)-phenylamino]-pyrimidin-4-yl}-thiazolo[3,2-a]pyrimidin-5-one (**54**) (1 eq, 31 mg, 0.059 mmol) following synthetic method B. Purified by flash chromatography (DCM:MeOH 96:4 to 85:15). Product **57** was a white solid. (4 mg, Yield = 13%). LC-MS 1 method. Rt= 2.914 min, m/z = 512 [M+H]⁺. ¹H-NMR (300MHz, DMSO-*d*6): δ 9.90 (s, 1H), 9.58 (s, 1H), 8.21 (d, J = 5.3 Hz, 1H), 7.93 (d, J = 6.5 Hz, 1H), 7.56 (d, J = 9.1 Hz, 2H), 7.29 (d, J = 8.6 Hz, 2H), 6.95 (d, J = 9.1 Hz, 2H), 6.86 (d, J = 8.6 Hz, 2H), 6.10 (d, J = 6.5 Hz, 1H), 5.73 (d, J = 5.2 Hz, 1H), 3.14 (br, 4H), 2.64 (br, 4H), 2.35 (s, 3H). ¹³C-NMR (75MHz, DMSO-*d*6): δ 162.89, 159.66, 158.97, 158.83, 158.62, 156.19, 152.23, 146.45, 137.97, 132.17, 130.99 (2C), 122.95, 121.74, 120.73 (2C), 116.03 (2C), 115.38 (2C), 107.67, 107.61, 54.50 (2C), 49.64 (2C), 47.84.

### Example 26. 3-(4-Fluoro-phenyl)-2-(2-{3-[4-(2-hydroxy-ethyl)-piperazin-1-yl]-phenylamino}-pyrimidin-4-yl)-thiazolo[3,2-a]pyrimidin-5-one (58)

From 2-(2-chloro-pyrimidin-4-yl)-3-(4-fluoro-phenyl)-thiazolo[3,2-a]pyrimidin-5-one (**24h**) (1 eq, 100 mg, 0.279 mmol) and 2-[4-(3-amino-phenyl)-piperazin-1-yl]-ethanol (**28b**) (1.1 eq, 68 mg, 0.307 mmol) as starting materials following synthetic method A. Purified by flash chromatography (Cyclohexane:EtOAc 100:0 to 0:100). Product **58** was a white solid. (15 mg, Yield = 10%). LC-MS 1 method. Rt= 3.321 min, m/z = 544 [M+H]⁺. ¹H-NMR (700MHz, DMSO-*d*6): δ 9.67 (s, 1H), 8.26 (d, J = 5.2 Hz, 1H), 7.97 (d, J = 6.4 Hz, 1H), 7.64-7.59 (m, 2H), 7.35 (m, 3H), 7.17 (m, 2H), 6.60 (d, J = 7.0 Hz, 1H), 6.12 (d, J = 6.4 Hz, 1H), 5.69 (d, J = 5.2 Hz, 1H), 3.54 (t, J = 6.3 Hz, 2H), 3.18-3.12 (m, 4H), 2.59-2.56 (m, 4H), 2.44 (t, J = 6.3 Hz, 2H).

### Example 27. 3-{4-[3-(2,3-Dichloro-phenyl)-5-oxo-5H-thiazolo[3,2-a]pyrimidin-2-yl]-pyrimidin-2-ylamino}-benzenesulfonamide (59)

From 2-(2-chloro-pyrimidin-4-yl)-3-(2,3-dichloro-phenyl)-thiazolo[3,2-a]pyrimidin-5-one (**24i**) (1 eq, 160 mg, 0.391 mmol) and 3-aminobenzenesulfonamide (1 eq, 67 mg, 0.391 mmol) as starting materials following synthetic method A. Purified by flash chromatography (DCM:MeOH 100:0 to 90:10). Product **59** was a pale yellow solid. (7 mg, Yield = 3.3%). LC-MS 1 method. Rt= 5.216 min, m/z = 545 [M+H]⁺. ¹H-NMR (300MHz, DMSO-*d*6): δ 10.25 (s, 1H), 8.40 (d, J = 5.2 Hz, 1H), 8.32 (s, 1H), 8.03 (d, J = 6.5 Hz, 1H), 7.98-7.79 (m, 2H), 7.67 (d, J = 7.2 Hz, 1H), 7.62-7.43 (m, 3H), 7.36 (s, 2H), 6.19 (d, J = 6.5 Hz, 1H), 5.83 (d, J = 5.2 Hz, 1H). ¹³C-NMR (75MHz, DMSO-*d*6): δ 162.27, 159.95, 159.36, 158.46, 155.08, 152.87, 144.71, 140.16, 132.85, 132.82, 132.21, 131.91, 131.48, 130.21, 129.35, 128.78, 126.55, 122.19, 119.27, 116.07, 108.15, 107.35.

### Example 28. 3-(2,3-Dichloro-phenyl)-2-(2-{3-[4-(2-hydroxy-ethyl)-piperazin-1-yl]-phenylamino}-pyrimidin-4-yl)-thiazolo[3,2-a]pyrimidin-5-one (60)

From 2-(2-chloro-pyrimidin-4-yl)-3-(2,3-dichloro-phenyl)-thiazolo[3,2-a]pyrimidin-5-one (**24i**) (1 eq, 95 mg, 0.232 mmol) and 2-[4-(3-amino-phenyl)-piperazin-1-yl]-ethanol (**28b**) (1.1 eq, 56 mg, 0.255 mmol) as starting materials following synthetic method A. Purified by flash chromatography (DCM:MeOH 97:3 to 93:7). Product **60** was a white solid. (14 mg, Yield = 10%). LC-MS 1 method. Rt= 3.622 min, m/z = 596 [M+H]⁺. ¹H-NMR (300MHz, DMSO-*d*6): δ 9.75 (s, 1H), 8.33 (d, J = 5.2 Hz, 1H), 8.03 (d, J = 6.5 Hz, 1H), 7.88 (dd, J = 8.1, 1.4 Hz, 1H), 7.67 (dd, J = 7.7, 1.4 Hz, 1H), 7.52 (t, J = 7.9 Hz, 1H), 7.34 (s, 1H), 7.25-7.08 (m, 2H), 6.61 (m, 1H), 6.18 (d, J = 6.5 Hz, 1H), 5.74 (d, J = 5.2 Hz, 1H), 3.54 (t, J = 6.2 Hz, 2H), 3.16 (br, 4H), 2.59 (br, 4H), 2.45 (t, J = 6.3 Hz, 2H). ¹³C-NMR (75MHz, DMSO-*d*6): δ 162.15, 159.79, 159.58, 158.41, 154.77, 152.86, 151.56, 140.36, 132.86, 132.58, 132.16, 131.87, 131.50, 130.20, 129.01, 128.75, 126.69, 110.43, 109.81, 107.37, 107.28, 106.68, 60.32, 58.52, 53.20 (2C), 48.43 (2C).

### Example 29. 3-{4-[3-(3,4-Dichloro-phenyl)-5-oxo-5H-thiazolo[3,2-a]pyrimidin-2-yl]-pyrimidin-2-ylamino}-benzenesulfonamide (61)

From 2-(2-chloro-pyrimidin-4-yl)-3-(3,4-dichloro-phenyl)-thiazolo[3,2-a]pyrimidin-5-one (**24j**) (1 eq, 200 mg, 0.488 mmol) and 3-aminobenzenesulfonamide (1.2 eq, 101 mg, 0.586 mmol) as starting materials following synthetic method A. Purified by flash chromatography (DCM:MeOH 100:0 to 85:15). Product **61** was a pale yellow solid. (16 mg, Yield = 6%). LC-MS 1 method. Rt= 5.232 min, m/z = 545 [M+H]⁺.
¹H-NMR (300MHz, DMSO-*d*6): δ 10.20 (s, 1H), 8.39 (d, J = 4.8 Hz, 1H), 8.31 (s, 1H), 8.02-7.91 (m, 2H), 7.89 (d, J = 7.7 Hz, 1H), 7.80 (d, J = 8.2 Hz, 1H), 7.63-7.43 (m, 3H), 7.36 (s, 2H), 6.14 (d, J = 6.3 Hz, 1H), 5.92 (d, J = 4.8 Hz, 1H). ¹³C-NMR (75MHz, DMSO-*d*6): δ 162.49, 159.59, 159.33, 158.84, 155.61, 152.58, 144.72, 140.24, 134.47, 132.53, 132.06, 131.71, 131.12, 130.62, 130.13, 129.33, 126.01, 122.06, 119.17, 116.01, 108.92, 107.54.

### Example 30. 3-(3-Chloro-4-methoxy-phenyl)-2-(2-{3-[4-(2-hydroxy-ethyl)-piperazin-1-yl]-phenylamino}-pyrimidin-4-yl)-thiazolo[3,2-a]pyrimidin-5-one (62)

From 3-(3-chloro-4-methoxy-phenyl)-2-(2-chloro-pyrimidin-4-yl)-thiazolo[3,2-a]pyrimidin-5-one (**24k**) (1 eq, 140 mg, 0.345 mmol) and 2-[4-(3-amino-phenyl)-piperazin-1-yl]-ethanol (**28b**) (1.1 eq, 84 mg, 0.380 mmol) as starting materials following synthetic method A. Purified by flash chromatography (DCM:MeOH 100:0 to 85:15). Product **62** was a white solid. (25 mg, Yield = 12%). LC-MS 1 method. Rt= 3.486 min, m/z = 590 [M+H]⁺. ¹H-NMR (300MHz, DMSO-*d*6): δ 9.67 (s, 1H), 8.28 (d, J = 5.2 Hz, 1H), 7.95 (d, J = 6.5 Hz, 1H), 7.68 (d, J = 2.1 Hz, 1H), 7.49 (dd, J = 8.5, 2.1 Hz, 1H), 7.35 (s, 1H), 7.25 (d, J = 8.7 Hz, 1H), 7.20 (d, J = 8.1 Hz, 1H), 7.18-7.12 (m, 1H), 6.59 (d, J = 7.6 Hz, 1H), 6.11 (d, J = 6.5 Hz, 1H), 5.80 (d, J = 5.2 Hz, 1H), 4.49 (br, 1H), 3.95 (s, 3H), 3.55 (t, J = 6.1 Hz, 2H), 3.22-3.10 (m, 4H), 2.66-2.54 (m, 4H), 2.46 (d, J = 6.1 Hz, 2H). ¹³C-NMR (75MHz, DMSO-*d*6): δ 162.46, 159.48, 159.12, 158.77, 155.67, 155.33, 152.33, 151.48, 140.51, 135.78, 131.01, 129.92, 128.96, 125.74, 124.22, 120.78, 112.38, 110.30, 109.60, 107.96, 107.60, 106.51, 60.25, 58.45, 56.29, 53.15 (2C), 48.40 (2C).

### Example 31. 3-(4-Fluoro-3-trifluoromethyl-phenyl)-2-(2-{3-[4-(2-hydroxy-ethyl)-piperazin-1-yl]-phenylamino}-pyrimidin-4-yl)-thiazolo[3,2-a]pyrimidin-5-one (63)

From 2-(2-chloro-pyrimidin-4-yl)-3-(4-fluoro-3-trifluoromethyl-phenyl)-thiazolo[3,2-a]pyrimidin-5-one (**24l**) (1 eq, 150 mg, 0.418 mmol) and 2-[4-(3-amino-phenyl)-piperazin-1-yl]-ethanol (**28b**) (1.1 eq, 102 mg, 0.460 mmol) as starting materials following synthetic method A. Purified by flash chromatography (DCM:MeOH 100:0 to 90:10). Product **63** was a white solid. (22 mg, Yield = 9%). LC-MS 1 method. Rt= 3.663 min, m/z = 612 [M+H]⁺. ¹H-NMR (700MHz, DMSO-*d*6): δ 9.70 (s, 1H), 8.30 (d, J = 5.2 Hz, 1H), 8.14-8.11 (m, 1H), 7.99 (t, J = 5.5 Hz, 2H), 7.70-7.66 (m, 1H), 7.35 (s, 1H), 7.20-7.15 (m, 2H), 6.63-6.59 (m, 1H), 6.13 (d, J = 6.4 Hz, 1H), 5.74 (d, J = 5.2 Hz, 1H), 4.49 (br, 1H), 3.54 (d, J = 3.8 Hz, 2H), 3.16 (s, 4H), 2.61-2.58 (m, 4H), 2.46 (t, J = 6.2 Hz, 2H).

### Example 32. 2-(2-{3-[4-(2-Hydroxy-ethyl)-piperazin-1-yl]-phenylamino}-pyrimidin-4-yl)-3-isopropyl-thiazolo[3,2-a]pyrimidin-5-one (64)

From 2-(2-chloro-pyrimidin-4-yl)-3-isopropyl-thiazolo[3,2-a]pyrimidin-5-one (**24m**) (1 eq, 130 mg, 0.424 mmol) and 2-[4-(3-amino-phenyl)-piperazin-1-yl]-ethanol (**28b**) (1.2 eq, 113 mg, 0.509 mmol) as starting materials following synthetic method A. Purified by flash chromatography (DCM:MeOH 99:1 to 80:20). Product **64** was a white solid. (76 mg, Yield = 36%). LC-MS 1 method. Rt= 3.180 min, m/z = 492 [M+H]⁺. ¹H-NMR (300MHz, DMSO-*d*6): δ 9.74 (s, 1H), 8.62 (d, J = 5.0 Hz, 1H), 7.95 (d, J = 6.4 Hz, 1H), 7.38 (s, 1H), 7.22 (d, J = 8.0 Hz, 1H), 7.13 (t, J = 8.1 Hz, 1H), 7.05 (d, J = 5.0 Hz, 1H), 6.58 (d, J = 8.1 Hz, 1H), 6.28 (d, J = 6.3 Hz, 1H), 5.75 (s, 1H), 4.48 (br, 1H), 4.05-3.90 (m, 1H), 3.55 (d, J = 6.8 Hz, 3H), 3.14 (br, 4H), 2.57 (br, 4H), 2.44 (t, J = 6.1 Hz, 2H), 1.44 (s, 3H), 1.41 (s, 3H). ¹³C-NMR (75MHz, DMSO-*d*6): δ 163.48, 159.85, 159.76, 159.61, 156.94, 151.83, 151.49, 143.69, 140.57, 128.94, 122.89, 111.37, 110.34, 109.62, 107.82, 106.60, 60.29, 58.48, 53.17 (2C), 48.40 (2C), 29.45, 20.85 (2C).

### Example 33. 3-Cyclopropyl-2-(2-{3-[4-(2-hydroxy-ethyl)-piperazin-1-yl]-phenylamino}-pyrimidin-4-yl)-thiazolo[3,2-a]pyrimidin-5-one (65)

From 2-(2-chloro-pyrimidin-4-yl)-3-cyclopropyl-thiazolo[3,2-a]pyrimidin-5-one (**24n**) (1 eq, 55 mg, 0.180 mmol) and 2-[4-(3-amino-phenyl)-piperazin-1-yl]-ethanol (**28b**) (1.2 eq, 48 mg, 0.217 mmol) as starting materials following synthetic method A. Purified by flash chromatography (DCM:MeOH 98.5:1.5 to 80:20). Product **65** was a white solid. (9 mg, Yield = 10%). LC-MS 3 method. Rt= 6.191 min, m/z = 490 [M+H]⁺. ¹H-NMR (300MHz, DMSO-*d*6): δ 9.69 (s, 1H), 8.63 (d, J = 5.1 Hz, 1H), 7.92 (d, J = 6.5 Hz, 1H), 7.42 (s, 1H), 7.38 (d, J = 5.1 Hz, 1H), 7.20 (d, J = 8.1 Hz, 1H), 7.14 (t, J = 7.9 Hz, 1H), 6.58 (d, J = 7.8 Hz, 1H), 6.23 (d, J = 6.5 Hz, 1H), 4.50 (br, 1H), 3.54 (d, J = 3.9 Hz, 2H), 3.16 (br, 4H), 2.59 (br, 4H), 2.54 (m, 1H), 2.45 (m, 2H), 1.14-1.04 (m, 2H), 0.59 (q, J = 5.5 Hz, 2H). ¹³C-NMR (75MHz, DMSO-*d*6): δ 162.67, 159.55, 159.43, 159.02, 158.89, 155.92, 152.20, 151.53, 140.74, 128.97, 125.40, 111.31, 110.23, 109.47, 107.92, 106.41, 60.28, 58.47, 55.00, 53.18 (2C), 48.45 (2C), 11.92, 11.31.

### Example 34. 2-(2-{3-[4-(2-Hydroxy-ethyl)-piperazin-1-yl]-phenylamino}-pyrimidin-4-yl)-3-(tetrahydropyran-4-yl)-thiazolo[3,2-a]pyrimidin-5-one (66)

From 2-(2-chloro-pyrimidin-4-yl)-3-(tetrahydro-pyran-4-yl)-thiazolo[3,2-a]pyrimidin-5-one (**24o**) (1 eq, 170 mg, 0.487 mmol) and 2-[4-(3-amino-phenyl)-piperazin-1-yl]-ethanol (**28b**) (1.2 eq, 129 mg, 0.585 mmol) as starting materials following synthetic method A. Purified by reverse phase HPLC. Product **66** was a white solid. (25.2 mg, Yield = 10%). LC-MS 1 method. Rt= 2.908 min, m/z = 534 [M+H]⁺. ¹H-NMR (300MHz, DMSO-*d*6): δ 9.77 (s, 1H), 8.64 (d, J = 5.0 Hz, 1H), 7.96 (d, J = 6.4 Hz, 1H), 7.38 (s, 1H), 7.22 (d, J = 8.1 Hz, 1H), 7.14 (d, J = 8.1 Hz, 1H), 7.09 (d, J = 4.9 Hz, 1H), 6.59 (d, J = 8.0 Hz, 1H), 6.29 (d, J = 6.4 Hz, 1H), 4.47 (t, J = 5.0 Hz, 1H), 3.95 (m, 1H), 3.88 (dd, J = 11.2, 3.5 Hz, 2H), 3.53 (dd, J = 11.3, 5.9 Hz, 2H), 3.30 (d, J = 11.5 Hz, 2H), 3.13 (br, 4H), 2.56 (br, 4H), 2.43 (t, J = 6.2 Hz, 2H), 2.30 (dd, J = 17.9, 5.4 Hz, 2H), 1.68 (d, J = 11.3 Hz, 2H). ¹³C-NMR (75MHz, DMSO-*d*6): δ 163.23, 160.11, 159.87, 159.79, 157.23, 151.88, 151.49, 141.08, 140.53, 128.95, 123.63, 111.96, 110.40, 109.69, 107.91, 106.67, 67.54 (2C), 60.32, 58.52, 53.19 (2C), 48.41 (2C), 37.64, 30.53 (2C).

### Example 35. 3-[5-Methoxy-4-(5-oxo-3-phenyl-5H-thiazolo[3,2-a]pyrimidin-2-yl)-pyrimidin-2-ylamino]-benzenesulfonamide (69)

From 2-(2-chloro-5-methoxy-pyrimidin-4-yl)-3-phenyl-thiazolo[3,2-a]pyrimidin-5-one (**24r**) (1 eq, 28 mg, 0.076 mmol) and 3-aminobenzenesulfonamide (1.5 eq, 20 mg, 0.113 mmol) as starting materials following synthetic method A. Purified by flash chromatography (Cyclohexane:EtOAc 100:0 to 0:100 and then EtOAc:MeOH 100:0 to 80:20). Product **69** was a yellow solid. (5 mg, Yield = 13%). LC-MS 1 method. Rt= 4.515 min, m/z = 507 [M+H]⁺. ¹H-NMR (300MHz, DMSO-*d*6): δ 9.93 (s, 1H), 8.37 (s, 1H), 8.19 (s, 1H), 8.00 (d, J = 6.4 Hz, 1H), 7.75 (d, J = 7.8 Hz, 1H), 7.44 (t, J = 7.9 Hz, 1H), 7.33 (dd, J = 18.9, 6.0 Hz, 8H), 6.20 (d, J = 6.5 Hz, 1H), 3.37 (s, 3H).

### Example 36. 2-(2-{3-[4-(2-Hydroxy-ethyl)-piperazin-1-yl]-phenylamino}-pyrimidin-4-yl)-5-oxo-3-phenyl-5H-thiazolo[3,2-a]pyrimidine-6-carboxylic acid ethyl ester (71)

From 2-(2-chloro-pyrimidin-4-yl)-5-oxo-3-phenyl-5H-thiazolo[3,2-a]pyrimidine-6-carboxylic acid ethyl ester (**25a**) (1 eq, 213 mg, 0.516 mmol) and 2-[4-(3-amino-phenyl)-piperazin-1-yl]-ethanol (**28b**) (1.2 eq, 137 mg, 0.619 mmol) as starting materials following synthetic method A. Purified by flash chromatography (DCM:MeOH 98:2 to 80:20). Product **71** was a pale brown solid. (10 mg, Yield = 3.2%). LC-MS 1 method. Rt= 3.531 min, m/z = 598 [M+H]⁺. ¹H-NMR (700MHz, DMSO-*d*6): δ 9.69 (s, 1H), 8.58 (s, 1H), 8.21 (d, J = 5.3 Hz, 1H), 7.58 (m, 1H), 7.56-7.51 (m, 4H), 7.33 (s, 1H), 7.17 (m, 2H), 6.60 (d, J = 6.9 Hz, 1H), 5.58 (d, J = 5.2 Hz, 1H), 4.18 (q, J = 7.1 Hz, 2H), 3.55 (t, J = 6.3 Hz, 2H), 3.16 (s, 4H), 2.60-2.56 (m, 4H), 2.45 (t, J = 6.3 Hz, 2H), 1.21 (t, J = 7.1 Hz, 3H).

### Example 37. 2-(2-{3-[4-(2-Hydroxy-ethyl)-piperazin-1-yl]-phenylamino}-pyrimidin-4-yl)-7-methyl-3-phenyl-thiazolo[3,2-a]pyrimidin-5-one (72)

From 2-(2-chloro-pyrimidin-4-yl)-7-methyl-3-phenyl-thiazolo[3,2-a]pyrimidin-5-one (**25b**) (1 eq, 37 mg, 0.104 mmol) and 2-[4-(3-amino-phenyl)-piperazin-1-yl]-ethanol (**28b**) (1.1 eq, 25 mg, 0.115 mmol) as starting materials following synthetic method A. Purified by reverse phase HPLC. Product **72** was a yellow solid. (7 mg, Yield = 12%). LC-MS 1 method. Rt= 3.377 min, m/z = 540 [M+H]⁺. ¹H-NMR (700MHz, DMSO-*d*6): δ 9.64 (s, 1H), 8.18 (d, J = 5.1 Hz, 1H), 7.59-7.46 (m, 5H), 7.36 (s, 1H), 7.20 (d, J = 7.8 Hz, 1H), 7.16 (t, J = 8.0 Hz, 1H), 6.59 (d, J = 7.5 Hz, 1H), 5.99 (s, 1H), 5.60 (d, J = 5.2 Hz, 1H), 4.45 (br, 1H), 3.55 (t, J = 6.1 Hz, 2H), 3.16 (br, 4H), 2.58 (br, 4H), 2.45 (t, J = 6.1 Hz, 2H), 2.26 (s, 3H).

### Example 38. 2-(2-{3-[4-(2-Hydroxy-ethyl)-piperazin-1-yl]-phenylamino}-pyrimidin-4-yl)-7-isopropyl-3-phenyl-thiazolo[3,2-a]pyrimidin-5-one (73)

From 2-(2-chloro-pyrimidin-4-yl)-7-isopropyl-3-phenyl-thiazolo[3,2-a]pyrimidin-5-one (**25c**) (1 eq, 103 mg, 0.269 mmol) and 2-[4-(3-amino-phenyl)-piperazin-1-yl]-ethanol (**28b**) (1.1 eq, 65 mg, 0.296 mmol) as starting materials following synthetic method A. Purified by flash chromatography (DCM-MeOH 100:0 to 80:20). Product **73** was a yellow solid. (23 mg, Yield = 15%). LC-MS 1 method. Rt= 3.780 min, m/z =568 [M+H]⁺. ¹H-NMR (700MHz, DMSO-*d*6): δ 9.67 (s, 1H), 8.19 (d, J = 5.3 Hz, 1H), 7.57-7.53 (m, 2H), 7.53 (d, J = 1.6 Hz, 2H), 7.51 (d, J = 7.3 Hz, 2H), 7.49 (d, J = 1.4 Hz, 1H), 7.19-7.11 (m, 2H), 6.59 (dd, J = 8.1, 1.4 Hz, 1H), 5.97 (s, 1H), 5.60 (d, J = 5.2 Hz, 1H), 4.46 (br, 1H), 3.54 (t, J = 6.3 Hz, 2H), 3.18 (br, 4H), 2.79 (dt, J = 13.6, 6.8 Hz, 1H), 2.62-2.58 (br, 4H), 2.45 (t, J = 6.3 Hz, 2H), 1.21 (s, 3H), 1.20 (s, 3H).

### Example 39. 7-Ethyl-2-(2-{3-[4-(2-hydroxy-ethyl)-piperazin-1-yl]-phenylamino}-pyrimidin-4-yl)-3-phenyl-thiazolo[3,2-a]pyrimidin-5-one (74)

From 2-(2-chloro-pyrimidin-4-yl)-7-ethyl-3-phenyl-thiazolo[3,2-a]pyrimidin-5-one (**25d**) (1 eq, 73 mg, 0.198 mmol) and 2-[4-(3-amino-phenyl)-piperazin-1-yl]-ethanol (**28b**) (1.1 eq, 48 mg, 0.218 mmol) as starting materials following synthetic method A. Purified by flash chromatography (Cyclohexane:EtOAc 100:0 to 0:100 and then EtOAc:MeOH 100:0 to 80:20). Product **74** was a yellow solid. (15 mg, Yield = 14%). LC-MS 1 method. Rt= 3.617 min, m/z =554 [M+H]⁺. ¹H-NMR (700MHz, DMSO-*d*6): 5 9.66 (s, 1H), 8.19 (d, J = 5.3 Hz, 1H), 7.55 (m, 1H), 7.54-7.51 (m, 3H), 7.50 (d, J = 7.3 Hz, 1H), 7.42 (s, 1H), 7.19-7.15 (m, 2H), 6.61-6.58 (m, 1H), 5.98 (s, 1H), 5.60 (d, J = 5.3 Hz, 1H), 3.55 (t, J = 6.3 Hz, 2H), 3.17 (br, 4H), 2.61-2.57 (br, 4H), 2.55 (dd, J = 15.2, 7.6 Hz, 2H), 2.45 (t, J = 6.3 Hz, 2H), 1.20 (t, J = 7.5 Hz, 3H).

### Example 40. 2-(2-{3-[4-(2-Hydroxy-ethyl)-piperazin-1-yl]-phenylamino}-pyrimidin-4-yl)-6,7-dimethyl-3-phenyl-thiazolo[3,2-a]pyrimidin-5-one (75)

From 2-(2-chloro-pyrimidin-4-yl)-6,7-dimethyl-3-phenyl-thiazolo[3,2-a]pyrimidin-5-one (**25e**) (1 eq, 68 mg, 0.184 mmol) and 2-[4-(3-amino-phenyl)-piperazin-1-yl]-ethanol (**28b**) (1.2 eq, 49 mg, 0.221 mmol) as starting materials following synthetic method A. Purified by flash chromatography (DCM:MeOH 100:0 to 80:20). Product **75** was a yellow solid. (10 mg, Yield = 10%). LC-MS 1 method. Rt= 3.579 min, m/z =554 [M+H]⁺. ¹H-NMR (700MHz, DMSO-*d*6): δ 9.64 (s, 1H), 8.18 (d, J = 5.3 Hz, 1H), 7.60-7.46 (m, 5H), 7.37 (s, 1H), 7.21 (d, J = 8.0 Hz, 1H), 7.16 (t, J = 8.1 Hz, 1H), 6.60 (dd, J = 8.2, 1.6 Hz, 1H), 5.60 (d, J = 5.3 Hz, 1H), 4.41 (br, 1H), 3.56 (d, J = 6.2 Hz, 2H), 3.17 (br, 4H), 2.63-2.57 (br, 4H), 2.46 (t, J = 6.3 Hz, 2H), 2.30 (s, 3H), 1.87 (s, 3H).

### Example 41. 2-(2-{3-[4-(2-Hydroxy-ethyl)-piperazin-1-yl]-phenylamino}-pyrimidin-4-yl)-6-isopropyl-7-methyl-3-phenyl-thiazolo[3,2-a]pyrimidin-5-one (76)

From 2-(2-chloro-pyrimidin-4-yl)-6-isopropyl-7-methyl-3-phenyl-thiazolo[3,2-a]pyrimidin-5-one (**25f**) (1 eq, 266 mg, 0.670 mmol) and 2-[4-(3-amino-phenyl)-piperazin-1-yl]-ethanol (**28b**) (1.05 eq, 156 mg, 0.704 mmol) as starting materials following synthetic method A. Purified by reverse phase HPLC. Product **76** was a yellow solid. (2 mg, Yield = 1%). LC-MS 1 method. Rt= 3.995 min, m/z =582 [M+H]⁺. ¹H-NMR (700MHz, DMSO-*d*6): δ 9.64 (s, 1H), 8.18 (d, J = 5.3 Hz, 1H), 7.60-7.47 (m, 5H), 7.37 (s, 1H), 7.24-7.10 (m, 2H), 6.59 (d, J = 7.3 Hz, 1H), 5.55 (d, J = 5.3 Hz, 1H), 4.47 (t, J = 5.3 Hz, 1H), 3.55 (dd, J = 11.7, 6.0 Hz, 2H), 3.16 (br, 4H), 3.00 (m, 1H), 2.58 (m, 4H), 2.44 (d, J = 6.4 Hz, 2H), 2.35 (s, 3H), 1.15 (s, 3H), 1.13 (s, 3H).

### Example 42. 6-Ethyl-2-(2-{3-[4-(2-hydroxy-ethyl)-piperazin-1-yl]-phenylamino}-pyrimidin-4-yl)-7-methyl-3-phenyl-thiazolo[3,2-a]pyrimidin-5-one (77)

From 2-(2-Chloro-pyrimidin-4-yl)-6-ethyl-7-methyl-3-phenyl-thiazolo[3,2-a]pyrimidin-5-one (**25g**) (1 eq, 93 mg, 0.243 mmol) and 2-[4-(3-amino-phenyl)-piperazin-1-yl]-ethanol (**28b**) (1.1 eq, 59 mg, 0.267 mmol) as starting materials following synthetic method A. Purified by reverse phase HPLC. Product **77** was a yellow solid. (5 mg, Yield = 4%). LC-MS 1 method. Rt= 3.730 min, m/z =568 [M+H]⁺. ¹H-NMR (300MHz, DMSO-*d*6): δ 9.70 (s, 1H), 8.19 (d, J = 5.3 Hz, 1H), 7.61-7.46 (m, 5H), 7.34 (s, 1H), 7.33 (d, J = 9.1 Hz, 1H), 7.23 (t, J = 8.0 Hz, 1H), 6.68 (d, J = 8.2 Hz, 1H), 5.61 (d, J = 5.2 Hz, 1H), 5.41 (br, 1H), 3.79 (br, 2H), 3.24 (br, 4H), 2.5 (br, 6H), 2.40-2.32 (m, 2H), 2.32 (s, 3H), 0.94 (t, J = 7.4 Hz, 3H).

### General procedure for the synthesis of intermediates 18

To a solution of 2-5 g (1 eq) of 2-chloro-4-methylpyrimidine, 4-methyl-2-(methylthio)pyrimidine, or 2-chloro-5-methoxy-4-methyl-pyrimidine in THF anhydrous (0.352 mmol/mL) was added the respective ester derivative (1-1.2 eq). Three cycles of vacuum and filling with Ar were carried out. The rounded bottom flask was placed in ice bath (0 ºC) and then 2 eq of LiHDMS 1.0M in THF were added dropwise to the previous solution. Once the LiHDMS was added the ice bath was removed. The reaction mixture was stirred at RT for 16-24 h. Then, the reaction was quenched with NH₄Cl (sat) until pH 7-8 and deionized water. The aqueous phase was extracted with EtOAc. The organic phase was dried with Na₂SO₄ and the solvent was removed under reduced pressure. The enolate and keto's form of the product were observed by ¹H-NMR. Obtained solids or oil were used in next reaction steps without further purification.

### 2-(2-Chloro-pyrimidin-4-yl)-1-phenyl-ethanone (18a).

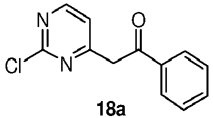

From 2-chloro-4-methylpyrimidine (1 eq, 2 g, 15.557 mmol) and methyl benzoate (2 eq, 3.893 mL, 31.114 mmol) as starting materials. **18a** was a brown oil (3.5 g, Yield = 97%). LC-MS 2 method. Rt= 3.651 and 4.541 min, m/z = 233 [M+H]⁺. Ketone: ¹H-NMR (300MHz, DMSO-*d*6): δ 8.75 (d, J = 5.0 Hz, 1H), 8.07-8.01 (m, 2H), 7.60 (d, J = 5.0 Hz, 1H), 7.74-7.65 (m, 1H), 7.61-7.52 (m, 2H), 4.70 (s, 2H). Enol: ¹H-NMR (300MHz, DMSO-*d*6): δ 13.51 (s, 1H), 8.60 (d, J = 5.4 Hz, 1H), 7.92-7.85 (m, 2H), 7.56-7.52 (m, 3H), 7.36 (d, J = 5.4 Hz, 1H), 6.57 (s, 1H).

### 1-(2-Chloro-phenyl)-2-(2-chloro-pyrimidin-4-yl)-ethanone (18b)

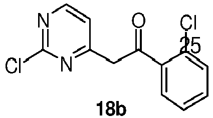

From 2-chloro-4-methylpyrimidine (1 eq, 2 g, 15.557 mmol) and 2-chloro-benzoic acid methyl ester (1.5 eq, 3.981 g, 23.335 mmol) as starting materials. **18b** was a brown oil (3.72 g, Yield = 90%). LC-MS 2 method. Rt= 4.110 and 4.303 min, m/z = 267 [M+H]⁺. Ketone: ¹H-NMR (300MHz, DMSO-*d*6): δ 8.76 (d, J = 4.9 Hz, 1H), 7.87 (d, J =7.4 Hz, 1H), 7.62 - 7.44 (m, 4H), 4.62 (s, 2H). Enol: ¹H-NMR (300MHz, DMSO-*d*6): δ 12.96 (s, 1H), 8.62 (d, J = 5.3 Hz, 1H), 7.78 (d, 7.4 Hz, 1H) 7.62 - 7.44 (m, 4H), 5.91 (s, 1H).

### 1-(3-Chloro-phenyl)-2-(2-chloro-pyrimidin-4-yl)-ethanone (18c)

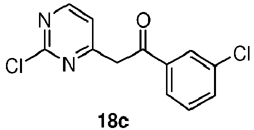

From 2-chloro-4-methylpyrimidine (1 eq, 2 g, 15.557 mmol) and 3-chloro-benzoic acid methyl ester (1.5 eq, 3.981 g, 23.335 mmol) as starting materials. **18c** was a brown oil (3.42 g, Yield = 82%). LC-MS 2 method. Rt= 4.179 and 4.811 min, m/z = 267 [M+H]⁺. Ketone: ¹H-NMR (300MHz, DMSO-*d*6): δ 8.75 (d, J = 5.0 Hz, 1H), 8.05 (s, 1H), 8.00 (d, J =7.8 Hz, 1H), 7.76 (dd, J = 8.20, 1.1 Hz, 1H), 7.64-7.55 (m, 2H), 4.72 (s, 2H). Enol: ¹H-NMR (300MHz, DMSO-*d*6): δ 13.47 (s, 1H), 8.63 (d, J = 5.3 Hz, 1H), 7.92 (s, 1H), 7.86 (d, J = 7.3 Hz, 1H), 7.59-7.56 (m, 2H), 7.37 (d, J = 5.4 Hz, 1H), 6.68 (s, 1H).

### 1-(4-Chloro-phenyl)-2-(2-chloro-pyrimidin-4-yl)-ethanone (18d)

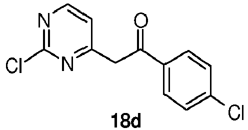

From 2-chloro-4-methylpyrimidine (1 eq, 2 g, 15.557 mmol) and 4-chlorobenzoic acid methyl ester (1.3 eq, 3.450 g, 20.224 mmol) as starting materials. **18d** was a brown oil (3.94g, Yield = 95%). LC-MS 2 method. Rt= 4.124 and 5.016 min, m/z = 267 [M+H]⁺.

Ketone: ¹H-NMR (300MHz, DMSO-*d*6): δ 8.75 (d, J = 5.0 Hz, 1H), 8.04 (d, J = 8.6 Hz, 2H), 7.64 (d, J =8.6 Hz, 2H), 7.59 (d, J = 5.0 Hz, 1H), 4.69 (s, 2H). Enol: ¹H-NMR (300MHz, DMSO-*d*6): δ 13.48z (s, 1H), 8.58 (d, J = 5.4 Hz, 1H), 7.89 (d, J = 8.6 Hz, 2H), 7.57 (d, J = 8.6 Hz, 2H), 7.36 (d, J = 5.4 Hz, 1H), 6.56 (s, 1H).

### 2-(2-Chloro-pyrimidin-4-yl)-1-(2-methoxy-phenyl)-ethanone (18e)

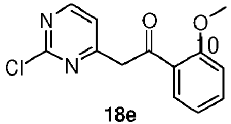

From 2-chloro-4-methylpyrimidine (1 eq, 2.33 g, 18.147 mmol) and 2-methoxybenzoic acid methyl ester (1.3 eq, 3.920 g, 23.591 mmol) as starting materials. **18e** was a brown oil (4.74 g, Yield = 99%). LC-MS 2 method. Rt= 4.165 and 5.140 min, m/z = 263 [M+H]⁺. Ketone: ¹H-NMR (300MHz, DMSO-*d*6): δ 8.71 (d, J = 5.0 Hz, 1H), 7.75-7.42 (m, 4H), 7.55 (d, J = 5.0 Hz, 1Hz), 4.51 (s, 2H), 3.87 (s, 3H). Enol: ¹H-NMR (300MHz, DMSO-*d*6): δ 13.38 (s, 1H), 8.54 (d, J = 5.4 Hz, 1H), 7.75-7.42 (m, 4H), 7.39 (d, J = 5.4 Hz, 1H), 6.47 (s, 1H), 3.89 (s, 3H).

### 2-(2-Chloro-pyrimidin-4-yl)-1-(3-methoxy-phenyl)-ethanone (18f)

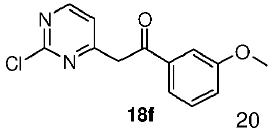

From 2-chloro-4-methylpyrimidine (1 eq, 2 g, 15.6 mmol) and 3-methoxybenzoic acid methyl ester (1 eq, 2.592 g, 15.6 mmol) as starting materials. **18f** was a brown oil (4.0 g, Yield = 98%). LC-MS 2 method. Rt= 4.154 min, m/z = 263 [M+H]⁺. Ketone: ¹H-NMR (300MHz, DMSO-*d*6): δ 8.75 (d, J = 5.0 Hz, 1H), 7.64 (ddd, J = 7.7, 1.5, 1.0 Hz, 1H), 7.59 (d, J = 5.0 Hz, 1H), 7.52-7.49 (m, 2H), 7.27 (ddd, J = 8.2, 2.7, 0.9 Hz, 1H), 4.69 (s, 2H), 3.83 (s, 1H). Enol: ¹H-NMR (300MHz, DMSO-*d*6): δ 13.50 (s, 1H), 8.60 (d, J = 5.4 Hz, 1H), 7.46-7.43 (m, 3H), 7.35 (d, J = 5.4 Hz, 1H), 7.11 (ddd, J = 7.8, 2.6, 1.3 Hz, 1H), 6.59 (s, 1H), 3.83 (s, 1H).

### 2-(2-Chloro-pyrimidin-4-yl)-1-(4-methoxy-phenyl)-ethanoneethanone (18g)

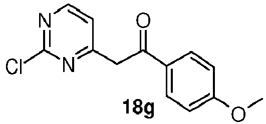

From 2-chloro-4-methylpyrimidine (1 eq, 4.2 g, 32.669 mmol) and 4-methoxy-benzoic acid methyl ester (1 eq, 5.429 g, 32.669 mmol) as starting materials. **18g** was a brown oil (7.58 g, Yield = 88%). LC-MS 2 method. Rt= 3.959 and 4.893 min, m/z = 263 [M+H]⁺. Ketone: ¹H-NMR (300MHz, DMSO-*d*6): δ 8.74 (d, J = 5.0 Hz, 1H), 8.01 (d, J = 8.9 Hz, 2H), 7.59 (d, J = 5.0 Hz, 1H), 7.08 (d, J =8.9 Hz, 2H), 4.62 (s, 2H), 3.86 (s, 1H). Enol: ¹H-NMR (300MHz, DMSO-*d*6): δ 13.58 (s, 1H), 8.71 (d, J = 5.1 Hz, 1H), 7.94 (d, J = 8.6 Hz, 2H), 7.48 (d, J = 5.1 Hz, 2H), 7.07 (d, J = 8.6 Hz, 1H), 6.47 (s, 1H), 3.85 (s, 1H).

### 2-(2-Chloro-pyrimidin-4-yl)-1-(4-fluoro-phenyl)-ethanone (18h)

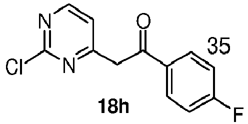

From 2-chloro-4-methylpyrimidine (1 eq, 5 g, 38.9 mmol) and 4-fluoro-benzoic acid methyl ester (1 eq, 5.991 g, 38.9 mmol) as starting materials. **18h** was a brown oil (9.7 g, Yield = 100%). LC-MS 2 method. Rt= 4.242 and 4.942 min, m/z = 251 [M+H]⁺. Ketone: ¹H-NMR (300MHz, DMSO-*d*6): δ 8.67 (d, J = 5.0 Hz, 1H), 8.05 (m, 2H), 7.53 (d, J = 5.0 Hz, 1H), 7.29 (m, 2H), 4.62 (s, 2H).

### 2-(2-Chloro-pyrimidin-4-yl)-1-(2,3-dichloro-phenyl)-ethanone (18i)

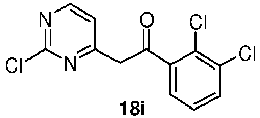

From 2-chloro-4-methylpyrimidine (1 eq, 4.284 g, 33.32 mmol) and 2,3-dichloro-benzoic acid methyl ester (1.1 eq, 7.515 g, 36.652 mmol) as starting materials. **18i** was a brown oil (10 g, Yield = 99%). LC-MS 2 method. Rt= 4.697 and 4.971 min, m/z = 303 [M+H]⁺.

### 2-(2-Chloro-pyrimidin-4-yl)-1-(3,4-dichloro-phenyl)-ethanone (18j)

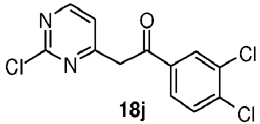

From 2-chloro-4-methylpyrimidine (1 eq, 2.623 g, 20.4 mmol) and 3,4-dichloro-benzoic acid methyl ester (1 eq, 4.187 g, 20.4 mmol) as starting materials. **18j** was a brown oil (5.94 g, Yield = 96%). LC-MS 2 method. Rt= 4.779 and 4.957 min, m/z = 303 [M+H]⁺. Ketone: ¹H-NMR (300MHz, DMSO-*d*6): δ 8.76 (d, J = 5.1 Hz, 1H), 8.26 (d, J = 2.0 Hz, 1H), 7.99 (dd, J = 8.4, 2.0 Hz, 1H), 7.87 (d, J = 8.4 Hz, 1H), 7.59 (d, J = 5.1 Hz, 1H), 4.73 (s, 2H).

### 1-(3-Chloro-4-methoxy-phenyl)-2-(2-chloro-pyrimidin-4-yl)-ethanone (18k)

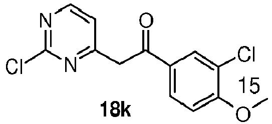

From 2-chloro-4-methylpyrimidine (1 eq, 5 g, 38.9 mmol) and 3-chloro-4-methoxy-benzoic acid methyl ester (1 eq, 7.804 g, 38.9 mmol) as starting materials. **18k** was a brown oil (10.5 g, Yield = 91%).

### 2-(2-Chloro-pyrimidin-4-yl)-1-(4-fluoro-3-trifluoromethyl-phenyl)-ethanone (18l)

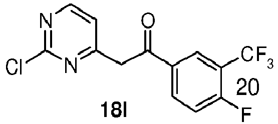

From 2-chloro-4-methylpyrimidine (1 eq, 3.555 g, 27.65 mmol) and 4-fluoro-3-trifluoromethyl-benzoic acid methyl ester (1 eq, 6.142 g, 27.65 mmol) as starting materials. **18l** was a brown oil (8.236 g, Yield = 94%). LC-MS 2 method. Rt= 4.659 and 4.975 min, m/z = 285 [M+H]⁺. Ketone: ¹H-NMR (300MHz, DMSO-*d*6): δ 8.76 (d, J = 5.0 Hz, 1H), 8.45 - 8.38 (m, 1H), 8.36 (d, J = 7.0 Hz, 1H), 7.75 (t, J = 10 Hz, 1H), 7.58 (d, J = 5.0 Hz, 1H), 4.78 (s, 2H).

### 3-Methyl-1-(2-methylsulfanyl-pyrimidin-4-yl)-butan-2-one (18m)

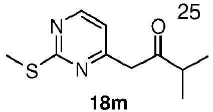

From 4-methyl-2-(methylthio)pyrimidine (1 eq, 5 g, 35.661 mmol) and ethyl isobutyrate (1 eq, 4.789 mL, 35.661 mmol) as starting materials. **18m** was a black oil (7.5 g, Yield = 100%). LC-MS 2 method. Rt= 3.902 and 4.959 min, m/z = 211 [M+H]⁺. Ketone: ¹H-NMR (700MHz, DMSO-*d*6): δ 8.55 (d, J = 5.0 Hz, 1H), 7.09 (d, J = 5.0 Hz, 1H), 4.00 (s, 2H), 2.75 (hept, J = 6.9 Hz, 1H), 2.47 (s, 3H), 1.07 (s, 3H), 1.06 (s, 3H).
Enol: ¹H-NMR (700MHz, DMSO-*d*6): δ 13.89 (s, 1H), 8.40 (d, J = 5.4 Hz, 1H), 6.86 (d, J = 5.4 Hz, 1H), 5.50 (s, 1H), 2.87 (hept, J = 6.9 Hz, 1H), 2.52 (s, 3H), 1.13 (s, 3H), 1.12 (s, 3H).

### 1-Cyclopropyl-2-(2-methylsulfanyl-pyrimidin-4-yl)-ethanone (18n)

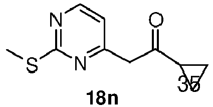

From 4-methyl-2-(methylthio)pyrimidine (1 eq, 5 g, 35.661 mmol) and ethyl cyclopropanecarboxylate (1 eq, 4.240 mL, 35.661 mmol) as starting materials. **18n** was a black oil (7.4 g, Yield = 100%). LC-MS 2 method. Rt= 3.455 and 5.075 min, m/z = 209 [M+H]⁺.
Ketone: ¹H-NMR (700MHz, DMSO-*d*6): δ 8.55 (d, J = 5.0 Hz, 1H), 7.12 (d, J = 5.1 Hz, 1H), 4.05 (s, 2H), 2.48 (s, 3H), 2.13 (tt, J = 7.7, 4.6 Hz, 1H), 0.95 - 0.92 (m, 2H), 0.92 - 0.88 (m, 2H).

### 2-(2-Methylsulfanyl-pyrimidin-4-yl)-1-(tetrahydro-pyran-4-yl)-ethanone (18o)

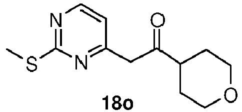

From 4-methyl-2-(methylthio)pyrimidine (1 eq, 5 g, 35.661 mmol) and methyl tetrahydro-2H-pyran-4-carboxylate (1 eq, 4.760 mL, 35.661 mmol) as starting materials. **18o** was a black oil (8.9 g, Yield = 100%). LC-MS 2 method. Rt= 3.355 and 4.357 min, m/z = 253 [M+H]⁺. Ketone: ¹H-NMR (700MHz, DMSO-*d*6): δ 8.55 (d, J = 5.1 Hz, 1H), 7.09 (d, J = 5.1 Hz, 1H), 4.01 (s, 2H), 3.86 (ddd, J = 11.2, 4.0, 2.2 Hz, 2H), 3.32 (dd, J = 11.6, 2.1 Hz, 2H), 2.78 (tt, J = 11.5, 3.8 Hz, 1H), 2.47 (s, 3H), 1.83 - 1.75 (m, 2H), 1.54 - 1.43 (m, 2H). Enol: 1H-NMR (700MHz, DMSO-d6): δ 13.92, (s, 1H), 8.41 (d, J = 5.4 Hz, 1H), 6.87 (d, J = 5.4 Hz, 1H), 5.52 (s, 1H), 3.90 (dd, J = 11.1, 3.3 Hz, 2H), 3.32 (m, 2H), 2.78 (m, 1H), 2.52 (s, 3H), 1.72 (m, 2H), 1.59 (m, 2H).

### 2-(2-Chloro-5-methoxy-pyrimidin-4-yl)-1-phenyl-ethanone (18r)

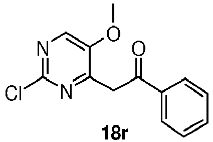

From 2-chloro-5-methoxy-4-methylpyridine (1 eq, 1 g, 6.306 mmol) and methyl benzoate (1.1 eq, 0.868 mL, 6.936 mmol) as starting materials. **18r** was a yellow solid (1.52 g, Yield = 92%). LC-MS 2 method. Rt= 4.155 and 5.088 min, m/z = 263 [M+H]⁺. Ketone: ¹H-NMR (300MHz, DMSO-*d*6): δ 8.52 (s, 1H), 8.02 (dd, J = 15 7.8, 1.1 Hz, 2H), 7.69 (tt, J = 7.8, 1.1 Hz, 1H), 7.57 (t, J = 7.8 Hz, 2H), 4.58 (s, 2H), 3.87 (s, 3H). Enol: ¹H-NMR (700MHz, DMSO-*d*6): δ 13.77 (s, 1H), 8.36 (s, 1H), 7.90 - 7.85 (m, 2H), 7.54-7.48 (m, 3H), 6.47 (s, 1H), 3.97 (s, 3H).

### Bromination Method of Intermediates 18 to render brominated intermediates 19

The respective **18a-o** or **18r** (1 eq) was dissolved in DCM (0.060 mmol/mL). The resulting solution was placed in ice bath (0 ºC) and N-bromosuccinimide (1.1 eq) was added. The reaction mixture was stirred for 3 h at RT. Then, water was added, and the organic phase was washed three times with water. Aqueous phase was extracted with DCM and organic layers were mixed and dried with Na₂SO₄. Solvent was removed and brominated product **19** was directly used without further purification in next reaction step. Quantitative yield was assumed.

### General Method for AminoThiazol formation: Intermediates 20 and 23

The respective brominated residue **(19a-r)** (1 eq) was taken up in dioxane (0.028 mmol/mL). Thiourea (1 eq) and Na₂CO₃ (1 eq) were added. The reaction mixture was heated at 50 °C for 18 h. Then, the reaction mixture was cooled at room temperature, diluted in water, and extracted with EtOAc or CHCl₃:iPrOH (1:1). The combined organic layers were dried over Na₂SO₄ and filtered, and the solvent was removed under reduced pressure. The residue was purified by flash chromatography.

### 4-Isopropyl-5-(2-methylsulfanyl-pyrimidin-4-yl)-thiazol-2-ylamine (20m)

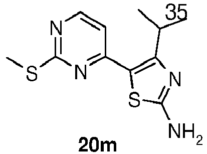

From 1-bromo-3-methyl-1-(2-methylsulfanyl-pyrimidin-4-yl)-butan-2-one **(19m)** (1 eq, 10.313 g, 35.661 mmol). Product was used in the next step without further purification. **20m** was a pale yellow solid (9.3 g, Yield = 97%). LC-MS 2 method. Rt= 3.421 min, m/z = 267 [M+H]⁺. ¹H-NMR (700MHz, DMSO-*d*6): δ 8.39 (d, J = 5.5 Hz, 1H), 7.60 (s, 2H), 7.02 (d, J = 5.5 Hz, 1H), 3.68 (hept, J = 6.7 Hz, 1H), 2.49 (s, 3H), 1.21 (s, 3H), 1.20 (s, 3H).

### 4-Cyclopropyl-5-(2-methylsulfanyl-pyrimidin-4-yl)-thiazol-2-ylamine (20n)

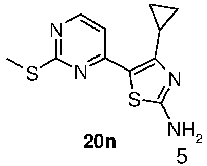

From 2-Bromo-1-cyclopropyl-2-(2-methylsulfanyl-pyrimidin-4-yl)-ethanone **(19n)** (1 eq, 10.241 g, 35.661 mmol). Product was used in the next step without further purification. **20n** was a pale yellow solid (10 g, Yield = 100%). LC-MS 2 method. Rt= 3.406 min, m/z = 265 [M+H]⁺. ¹H-NMR (700MHz, DMSO-*d*6): δ 8.38 (d, J = 5.5 Hz, 1H), 7.64 (s, 2H), 7.22 (d, J = 5.5 Hz, 1H), 2.67-2.60 (m, 1H), 2.56 (s, 3H), 1.00-0.92 (m, 4H). ¹³C-NMR (75MHz, DMSO-*d*6) δ 179.57, 170.71, 169.49, 158.55, 156.92, 116.07, 111.56, 29.60, 13.56, 12.61, 9.04.

### 5-(2-Methylsulfanyl-pyrimidin-4-yl)-4-(tetrahydro-pyran-4-yl)-thiazol-2-ylamine (20o)

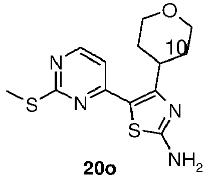

From 2-bromo-2-(2-methylsulfanyl-pyrimidin-4-yl)-1-(tetrahydro-pyran-4-yl)-ethanone (**19o**) (1 eq, 11.812 g, 35.661 mmol). Product was used in the next step without further purification. **20o** was a pale yellow solid (8.17 g, Yield = 74%). LC-MS 2 method. Rt= 3.509 min, m/z = 309 [M+H]⁺. ¹H-NMR (700MHz, DMSO-*d*6): δ 8.39 (d, J = 5.5 Hz, 1H), 7.69 (s, 2H), 7.01 (d, J = 5.5 Hz, 1H), 3.91 (dd, J = 11.0, 3.8 Hz, 2H), 3.67 (ddd, J = 11.6, 7.9, 3.7 Hz, 1H), 3.42 (t, J = 11.2 Hz, 2H), 2.50 (s, 3H), 1.85 (qd, J = 12.8, 4.4 Hz, 2H), 1.63 (dd, J = 12.9, 1.6 Hz, 2H).

### 5-(2-Chloro-pyrimidin-4-yl)-4-phenyl-thiazol-2-ylamine (23a)

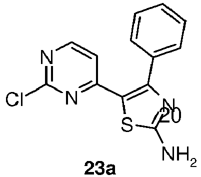

From 2-bromo-2-(2-chloro-pyrimidin-4-yl)-1-phenyl-ethanone **(19a)** (1 eq, 4.702 g, 15.09 mmol). Purified by flash chromatography (Cyclohexane:AcOEt 80:20 to 40:60). **23a** was a pale yellow solid (2.4 g, Yield = 55%). LC-MS 1 method. Rt= 4.302 min, m/z = 289 [M+H]⁺. ¹H-NMR (300MHz, DMSO-*d*6): δ 8.25 (d, J = 5.6 Hz, 1H), 7.96 (s, 2H), 7.57-7.42 (s, 5H), 6.75 (d, J = 5.6 Hz, 1H). ¹³C-NMR (75MHz, DMSO-*d*6): δ 170.48, 161.38, 159.72, 158.46, 156.64, 135.38, 129.35, 128.89 (2C), 128.77 (2C), 116.88, 113.87.

### 4-(2-Chloro-phenyl)-5-(2-chloro-pyrimidin-4-yl)-thiazol-2-ylamine (23b)

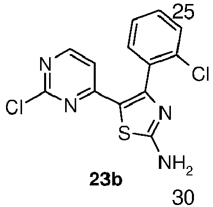

From 2-bromo-1-(2-chloro-phenyl)-2-(2-chloro-pyrimidin-4-yl)-ethanone **(19b)** (1 eq, 4.819 g, 13.927 mmol). Purified by flash chromatography (DCM:MeOH 100:0 to 90:10). **23b** was a pale yellow solid (1.61 g, Yield = 36%). LC-MS 2 method. Rt= 4.059-4.306 min, m/z = 323 [M+H]⁺. ¹H-NMR (300MHz, DMSO-*d*6): δ 8.28 (d, J = 5.6 Hz, 1H), 8.03 (s, 2H), 7.64 (dd, J = 8.2, 0.8 Hz, 1H), 7.58-7.45 (m, 3H), 6.34 (d, J = 5.6 Hz, 1H). ¹³C-NMR (75MHz, DMSO-*d*6): δ 170.59, 160.52, 159.78, 159.03, 153.31, 134.71, 131.84, 131.04, 130.94, 130.03, 128.03, 118.62, 112.65.

### 4-(3-Chloro-phenyl)-5-(2-chloro-pyrimidin-4-yl)-thiazol-2-ylamine (23c)

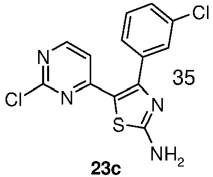

From 2-bromo-1-(3-chloro-phenyl)-2-(2-chloro-pyrimidin-4-yl)-ethanone **(19c)** (1 eq, 1.943 g, 5.616 mmol). Purified by flash chromatography (DCM:MeOH 100:0 to 90:10). **23c** was a pale yellow solid (1.23 g, Yield = 68%). LC-MS 2 method. Rt= 4.194 min, m/z = 323 [M+H]⁺. ¹H-NMR (300MHz, DMSO-*d*6): δ 8.32 (d, J = 5.5 Hz, 1H), 7.98 (s, 2H), 7.65-7.35 (m, 4H), 6.83 (d, J = 5.5 Hz, 1H). ¹³C-NMR (75 MHz, DMSO-*d*6) δ 170.43, 161.05, 159.75, 158.83, 154.32, 137.32, 133.42, 130.73, 129.19, 128.60, 127.58, 117.39, 114.28.

### 4-(4-Chloro-phenyl)-5-(2-chloro-pyrimidin-4-yl)-thiazol-2-ylamine (23d)

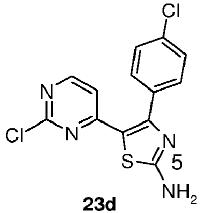

From 2-bromo-1-(4-chloro-phenyl)-2-(2-chloro-pyrimidin-4-yl)-ethanone **(19d)** (1 eq, 5.109 g, 14.765 mmol). Purified by flash chromatography (DCM:MeOH 99:1 to 94:6). **23d** was a pale yellow solid (1.84 g, Yield = 38%). LC-MS 2 method. Rt= 4.225 min, m/z = 323 [M+H]⁺. ¹H-NMR (300MHz, DMSO-*d*6): δ 8.31 (d, J = 5.5 Hz, 1H), 7.97 (s, 2H), 7.63-7.45 (m, 4H), 6.84 (d, J = 5.5 Hz, 1H). ¹³C-NMR (75MHz, DMSO-*d*6): δ 170.44, 161.16, 159.74, 158.82, 154.82, 134.10, 133.93, 130.76 (2C), 128.92 (2C), 117.14, 114.24.

### 5-(2-Chloro-pyrimidin-4-yl)-4-(2-methoxy-phenyl)-thiazol-2-ylamine (23e)

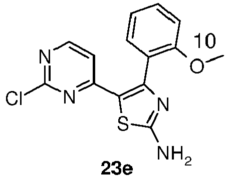

From 2-bromo-2-(2-chloro-pyrimidin-4-yl)-1-(2-methoxy-phenyl)-ethanone **(19e)** (1 eq, 6.165 g, 18.048 mmol). Purified by flash chromatography (DCM:MeOH 99:1 to 94:6). **23e** was a pale yellow solid (1.72 g, Yield = 30%). LC-MS 2 method. Rt= 4.194 min, m/z = 319 [M+H]⁺. ¹H-NMR (300MHz, DMSO-*d*6): δ 8.24 (d, J = 5.6 Hz, 1H), 7.89 (s, 2H), 7.48 (ddd, J = 7.9, 7.4, 1.8 Hz, 1H), 7.31 (dd, J = 7.5, 1.7 Hz, 1H), 7.15 (d, J = 7.9 Hz, 1H), 7.06 (td, J = 7.5, 0.9 Hz, 1H), 6.52 (d, J = 5.6 Hz, 1H), 3.64 (s, 3H). ¹³C-NMR (75MHz, DMSO-*d*6): δ 170.29, 161.13, 159.50, 158.37, 156.25, 153.37, 130.84, 130.34, 124.44, 120.85, 118.11, 112.89, 111.87, 55.36.

### 5-(2-Chloro-pyrimidin-4-yl)-4-(3-methoxy-phenyl)-thiazol-2-ylamine (23f)

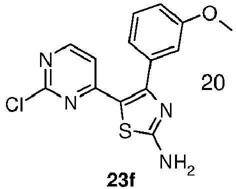

From 2-bromo-2-(2-chloro-pyrimidin-4-yl)-1-(3-methoxy-phenyl)-ethanone **(19f)** (1 eq, 3.689 g, 10.80 mmol). Product was used after workup without further purification. **23f** was a pale yellow solid (3.07 g, Yield = 89%). LC-MS 2 method. Rt= 4.123 min, m/z = 319 [M+H]⁺. ¹H-NMR (300MHz, DMSO-*d*6): δ 8.27 (d, J = 5.6 Hz, 1H), 7.96 (s, 2H), 7.38 (dd, J = 8.8, 7.4 Hz, 1H), 7.11 - 6.98 (m, 3H), 6.80 (d, J = 5.6 Hz, 1H), 3.76 (s, 3H).¹³C-NMR (75MHz, DMSO-*d*6: δ 170.44, 161.32, 159.67, 159.43, 158.44, 156.33, 136.66, 130.03, 120.92, 116.97, 115.34, 114.02, 113.66, 55.25.

### 5-(2-Chloro-pyrimidin-4-yl)-4-(4-methoxy-phenyl)-thiazol-2-ylamine (23g)

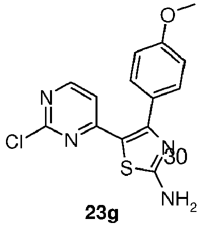

From 2-bromo-2-(2-chloro-pyrimidin-4-yl)-1-(4-methoxy-phenyl)-ethanone **(19g)** (1 eq, 9.857 g, 28.855 mmol). Purified by flash chromatography (DCM:MeOH 100:0 to 90:10). **23g** was a pale yellow solid (2.7 g, Yield = 29%). LC-MS 2 method. Rt= 4.087 min, m/z = 319 [M+H]⁺. ¹H-NMR (300MHz, DMSO-*d*6): δ 8.26 (d, J = 5.5 Hz, 1H), 7.92 (s, 2H), 7.64 (d, J = 8.4 Hz, 1H), 7.45 (d, J = 8.3 Hz, 2H), 7.01 (d, J = 8.3 Hz, 2H), 6.86 (d, J = 5.5 Hz, 1H), 3.81 (s, 3H). ¹³C-NMR (75MHz, DMSO-*d*6): δ 170.37, 161.65, 160.02, 159.72, 158.39, 156.66, 130.32 (2C), 127.43, 116.19, 114.23 (2C), 113.90, 55.28.

### 5-(2-Chloro-pyrimidin-4-yl)-4-(4-fluoro-phenyl)-thiazol-2-ylamine (23h)

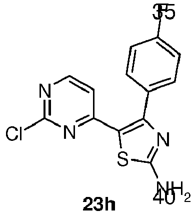

From 2-bromo-2-(2-chloro-pyrimidin-4-yl)-1-(4-fluoro-phenyl)-ethanone **(19h)** (1 eq, 11.728 g, 36.3 mmol). Purified by flash chromatography (Cyclohexane:(EtOAc:EtOH 3:1) 3:1). **23h** was a pale yellow solid (4.41 g, Yield = 40%). LC-MS 2 method. Rt= 4.163 min, m/z = 307 [M+H]⁺. ¹H-NMR (300MHz, DMSO-*d*6): δ 8.29 (d, J = 5.6 Hz, 1H), 7.96 (s, 2H), 7.57 (dd, J = 8.8, 5.6 Hz, 2H), 7.30 (t, J = 8.9 Hz, 2H), 6.80 (d, J = 5.6 Hz, 1H). ¹³C-NMR (75MHz, DMSO-*d*6): δ 170.83, 160.4 (d, J = 195 Hz), 160.16, 155.74, 132.17, 131.64 (2C), 131.52, 117.34, 116.44, 116.16, 114.49 (2C).

### 5-(2-Chloro-pyrimidin-4-yl)-4-(2,3-dichloro-phenyl)-thiazol-2-ylamine (23i)

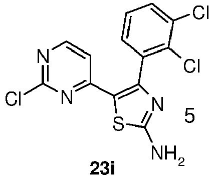

From 2-bromo-2-(2-chloro-pyrimidin-4-yl)-1-(2,3-dichloro-phenyl)-ethanone **(19i)** (1 eq, 12.677 g, 33.32 mmol). Purified by flash chromatography (DCM:MeOH 98.5:1.5 to 90:10). **23i** was a pale yellow solid (2.9 g, Yield = 24%). LC-MS 2 method. Rt= 4.491 min, m/z = 357 [M+H]⁺. ¹H-NMR (300MHz, DMSO-*d*6): δ 8.32 (d, J = 5.5 Hz, 1H), 8.05 (s, 2H), 7.80 (dd, J = 7.7, 2.0 Hz, 1H), 7.54 - 7.43 (m, 2H), 6.41 (d, J = 5.5 Hz, 1H), 3.16 (s, 1H). ¹³C-NMR (75MHz, DMSO-*d*6): δ 170.61, 160.19, 159.77, 159.36, 152.47, 137.08, 132.55, 131.39, 130.23, 129.57, 129.08, 118.62, 112.94.

### 5-(2-Chloro-pyrimidin-4-yl)-4-(3,4-dichloro-phenyl)-thiazol-2-ylamine (23j)

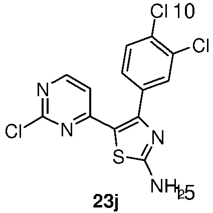

From 2-bromo-2-(2-chloro-pyrimidin-4-yl)-1-(3,4-dichloro-phenyl)-ethanone **(19j)** (1 eq, 5.94 g, 19.60 mmol). Purified by flash chromatography (Cyclohexane:EtOAc 2:1) **23j** was a pale yellow solid (4.19 g, Yield = 60%). LC-MS 2 method. Rt= 4.656 min, m/z = 357 [M+H]⁺. ¹H-NMR (300MHz, DMSO-*d*6): δ 8.35 (d, J = 5.5 Hz, 1H), 7.99 (s, 2H), 7.81 (d, J = 2.0 Hz, 1H), 7.71 (d, J = 8.3 Hz, 1H), 7.52 (dd, J = 8.3, 2.0 Hz, 1H), 6.94 (d, J = 5.5 Hz, 1H).

### 4-(3-Chloro-4-methoxy-phenyl)-5-(2-chloro-pyrimidin-4-yl)-thiazol-2-ylamine (23k)

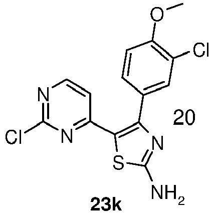

From 2-bromo-2-(2-chloro-pyrimidin-4-yl)-1-(3-chloro-4-methoxy-phenyl)-ethanone **(19k)** (1 eq, 13.3 g, 35.47 mmol). Purified by flash chromatography (Cyclohexane:EtOAc 2:1) **23k** was a pale yellow solid (3.53 g, Yield = 28%). LC-MS 2 method. Rt= 4.854 min, m/z = 353 [M+H]⁺. ¹H-NMR (300MHz, DMSO-*d*6): δ 8.31 (d, J = 5.6 Hz, 1H), 7.94 (s, 2H), 7.59 (d, J = 2.1 Hz, 1H), 7.49 (dd, J = 8.5, 2.1 Hz, 1H), 7.22 (d, J = 8.6 Hz, 1H), 6.93 (d, J = 5.6 Hz, 1H), 3.92 (s, 3H). ¹³C-NMR (75MHz, DMSO-*d*6): δ 170.76, 161.80, 160.17, 159.08, 155.61, 155.04, 130.66, 129.50, 128.73, 121.64, 117.10, 114.58, 113.30, 56.72.

### 5-(2-Chloro-pyrimidin-4-yl)-4-(4-fluoro-3-trifluoromethyl-phenyl)-thiazol-2-ylamine (231)

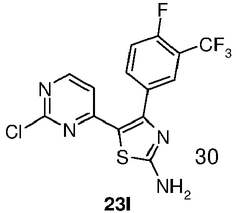

From 2-bromo-2-(2-chloro-pyrimidin-4-yl)-1-(4-fluoro-3-trifluoromethyl-phenyl)-ethanone (**19l**) (1 eq, 9.787 g, 24.617 mmol). Purified by flash chromatography (Cyclohexane:(EtOAc:EtOH 3:1) 3:1). **23l** was a pale yellow solid (3.2 g, Yield = 35%). LC-MS 2 method. Rt= 4.890 min, m/z = 375 [M+H]⁺. ¹H-NMR (300MHz, DMSO-*d*6): δ 8.35 (d, J = 5.5 Hz, 1H), 8.02 (s, 2H), 7.95 - 7.88 (m, 1H), 7.62-7.55 (m, 1H), 7.40 - 6.99 (m, 1H), 6.95 (d, J = 5.5 Hz, 1H).

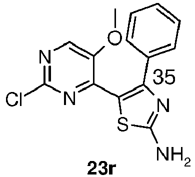

### 5-(2-Chloro-5-methoxy-pyrimidin-4-yl)-4-phenyl-thiazol-2-ylamine (23r)

From 2-bromo-2-(2-chloro-5-methoxy-pyrimidin-4-yl)-1-phenyl-ethanone **(19r)** (1 eq, 1.4 g, 4.098 mmol). Crude was filtered and solid was washed with ACN yielding the desired product pure. **23r** was a pale yellow solid (1.2 g, Yield = 92%). LC-MS 2 method. Rt= 3.830 min, m/z = 319 [M+H]⁺. ¹H-NMR (700MHz, DMSO-*d*6): δ 8.36 (s, 1H), 7.53-7.12 (m, 5H), 3.41 (s, 3H).

### General Procedure for the synthesis of Intermediates 21

To a solution of **20m-o** (1 eq) in DCM (0.1 mmol/mL) was added m-CPBA (2.5 eq) at 0 ºC. The reaction mixture was stirred at RT for 6-16 h. Solvent was removed under reduced pressure. Solid was triturated with deionized water in order to remove the resulting acid derived from m-CPBA. The obtained solid was used in the following steps without further purification.

### 4-Isopropyl-5-(2-methanesulfonyl-pyrimidin-4-yl)-thiazol-2-ylamine (21m)

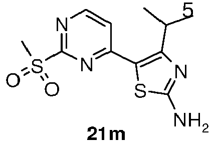

From 4-isopropyl-5-(2-methylsulfanyl-pyrimidin-4-yl)-thiazol-2-ylamine **(20m)** (1 eq, 3.5 g, 13.139 mmol). Product **21m** was a white solid (3.3 g, Yield = 84%). LC-MS 2 method. Rt= 1.835-3.067 min, m/z = 299 [M+H]⁺. ¹H-NMR (700MHz, DMSO-*d*6): δ 8.73 (d, J = 5.6 Hz, 1H), 7.95 (s, 2H), 7.50 (d, J = 5.6 Hz, 1H), 3.74 (m, 1H), 3.37 (s, 3H), 1.23 (s, 3H), 1.22 (s, 3H).

### 4-Cyclopropyl-5-(2-methanesulfonyl-pyrimidin-4-yl)-thiazol-2-ylamine (21n)

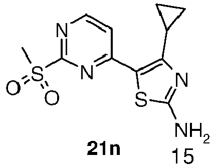

From 4-cyclopropyl-5-(2-methylsulfanyl-pyrimidin-4-yl)-thiazol-2-ylamine **(20n)** (1 eq, 3.5 g, 13.239 mmol). Product **21n** was a white solid (1.08 g, Yield = 28%). LC-MS 2 method. Rt= 2.463 and 3.191 min, m/z = 297 [M+H]⁺. ¹H-NMR (300MHz, DMSO-*d*6): δ 8.71 (d, J = 5.7 Hz, 1H), 7.93 (s, 2H), 7.66 (d, J = 5.7 Hz, 1H), 3.36 (s, 3H), 2.84-2.76 (m, 1H), 1.07-0.96 (m, 4H). ¹³C-NMR (75MHz, DMSO-*d*6): δ 170.40, 165.19, 162.59, 159.50, 157.76, 118.06, 114.59, 38.82, 12.81, 9.60 (2C).

### 5-(2-Methanesulfonyl-pyrimidin-4-yl)-4-(tetrahydro-pyran-4-yl)-thiazol-2-ylamine (21o)

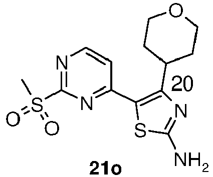

From 5-(2-methylsulfanyl-pyrimidin-4-yl)-4-(tetrahydro-pyran-4-yl)-thiazol-2-ylamine (**20o**) (1 eq, 3 g, 9.727 mmol). Product **21o** was a white solid (1.29 g, Yield = 39%). LC-MS 2 method. Rt= 2.943 min, m/z = 341 [M+H]⁺. ¹H-NMR (700MHz, DMSO-*d*6): δ 8.74 (d, J = 5.6 Hz, 1H), 7.99 (s, 2H), 7.48 (d, J = 5.6 Hz, 1H), 3.91 (dd, J = 10.9, 3.9 Hz, 2H), 3.83-3.71 (m, 1H), 3.45 (t, J = 11.1 Hz, 2H), 3.38 (s, 3H), 1.83 (qd, J = 12.8, 4.3 Hz, 2H), 1.72-1.62 (m, 2H).

### General Procedure for the synthesis of Intermediates 22

Product **21m-o** was suspended in HCl (37%) in a sealed pressure tube. Mixture was heated at 90 ºC for 1 h. Mixture was diluted with MeOH and then solvent was removed under reduced pressure. Rinse with MeOH was repeated three times. Solid was dried at 40 ºC in vacuo for 24 h. Remaining water in the solid was removed adding toluene and concentrating in vacuum three times.

### 4-(2-Amino-4-isopropyl-thiazol-5-yl)-pyrimidin-2-ol (22m)

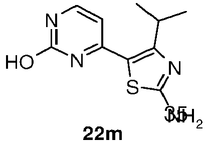

From 4-isopropyl-5-(2-methanesulfonyl-pyrimidin-4-yl)-thiazol-2-ylamine **(21m)** (1 eq, 3.3 g, 11.059 mmol). Product **22m** was a yellow solid (2.6 g, Yield = 100%). LC-MS 2 method. Rt= 0.384-0.782 min, m/z = 237 [M+H]⁺. ¹H-NMR (300MHz, DMSO-*d*6): δ 9.03 (s, 1H), 7.99 (d, J = 6.0 Hz, 1H), 6.61 (d, J = 6.0 Hz, 1H), 3.47 (m, 1H), 1.26 (m, 6H).

### 4-(2-Amino-4-cyclopropyl-thiazol-5-yl)-pyrimidin-2-ol (22n)

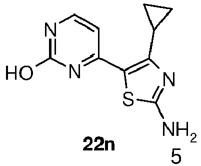

From 4-cyclopropyl-5-(2-methanesulfonyl-pyrimidin-4-yl)-thiazol-2-ylamine (**21n**) (1 eq, 1.08 g, 3.648 mmol). Product **22n** was a yellow solid (854 mg, Yield = 100%). LC-MS 2 method. Rt= 1.005 min, m/z = 235 [M+H]⁺. ¹H-NMR (300MHz, DMSO-*d*6): δ 8.00 (d, J = 6.9 Hz, 1H), 6.89 (d, J = 6.9 Hz, 1H), 2.37 (s, 1H), 1.18-1.02 (m, 4H).

### 4-[2-Amino-4-(tetrahydro-pyran-4-yl)-thiazol-5-yl]-pyrimidin-2-ol (22o)

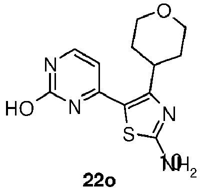

From 5-(2-Methanesulfonyl-pyrimidin-4-yl)-4-(tetrahydro-pyran-4-yl)-thiazol-2-ylamine (**21o**) (1 eq, 1.18 g, 3.466 mmol). Product **22o** was a yellow solid (963 mg, Yield = 100%). LC-MS 2 method. Rt= 0.391 and 0.734 min, m/z = 279 [M+H]⁺. ¹H-NMR (700MHz, DMSO-*d*6): δ 8.94 (s, 1H), 8.00 (d, J = 6.7 Hz, 1H), 6.65 (d, J = 6.7 Hz, 1H), 3.72-3.67 (m, 1H), 3.46 (m, 4H), 1.87 (m, 2H), 1.73-1.66 (m, 2H).

### General Procedure for the synthesis of Intermediates 23

Phosphorus oxychloride (0.147 mmol/mL) and **22m-o** were heated at 90 ºC for 3h 30 min. The reaction was poured carefully into ice H₂O, quenched with Na₂CO₃, and aqueous phase extracted with CHCl₃:iPrOH (1:1). The organic layer was dried with Na₂SO₄ and concentrated in vacuo.

### 5-(2-Chloro-pyrimidin-4-yl)-4-isopropyl-thiazol-2-ylamine (23m)

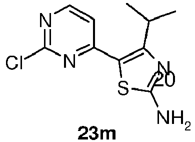

From 4-(2-Amino-4-isopropyl-thiazol-5-yl)-pyrimidin-2-ol **(22m)** (1 eq, 2.6 g, 11.059 mmol). Purified by flash chromatography (DCM:(DCM:MeOH:NH3 7N 9:0.5:0.5) from 100:0 to 0:100) Product **23m** was a yellow solid (600 mg, Yield = 21%). LC-MS 2 method. Rt= 3.428 min, m/z = 255 [M+H]⁺. ¹H-NMR (300MHz, DMSO-*d*6): δ 8.48 (d, J = 5.6 Hz, 1H), 7.87 (s, 2H), 7.34 (d, J = 5.6 Hz, 1H), 3.67-3.55 (m, 1H), 1.21 (s, 3H), 1.19 (s, 3H). ¹³C-NMR (75MHz, DMSO-*d*6): δ 170.41, 165.43, 160.89, 159.65, 159.48, 115.12, 114.24, 29.29, 21.92 (2C).

### 5-(2-Chloro-pyrimidin-4-yl)-4-cyclopropyl-thiazol-2-ylamine (23n)

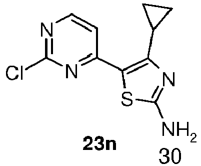

From 4-(2-Amino-4-cyclopropyl-thiazol-5-yl)-pyrimidin-2-ol **(22n)** (1 eq, 854 mg, 3.648 mmol). Product was directly used in the next step. Product **23n** was a yellow solid (614 g, Yield = 67%). LC-MS 2 method. Rt= 3.386 min, m/z = 253 [M+H]⁺. ¹H-NMR (300MHz, DMSO-*d*6): δ 8.46 (d, J = 5.6 Hz, 1H), 7.86 (s, 2H), 7.52 (d, J = 5.6 Hz, 1H), 2.64 (m, 1H), 1.03-0.95 (m, 4H).

### 5-(2-Chloro-pyrimidin-4-yl)-4-(tetrahydro-pyran-4-yl)-thiazol-2-ylamine (23o)

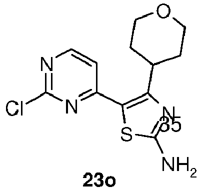

From 4-[2-Amino-4-(tetrahydro-pyran-4-yl)-thiazol-5-yl]-pyrimidin-2-ol (**22o**) (1 eq, 965 mg, 3.466 mmol). Product was directly used in the next step. Product **23o** was a yellow solid (879 mg, Yield = 86%). LC-MS 2 method. Rt= 3.479 min, m/z = 297 [M+H]⁺. ¹H-NMR (700MHz, DMSO-*d*6): δ 8.49 (d, J = 5.6 Hz, 1H), 7.89 (s, 2H), 7.34 (d, J = 5.6 Hz, 1H), 7.02 (d, J = 5.5 Hz, 1H), 3.92 (dd, J = 10.9, 4.0 Hz, 2H), 3.53 (tt, J = 11.5, 3.6 Hz, 1H), 3.48-3.41 (m, 2H), 1.83 (ddd, J = 16.5, 12.7, 4.3 Hz, 2H), 1.66 (dd, J = 13.1, 1.7 Hz, 2H).

### General Procedure for the synthesis of intermediates 24

To a solution of **23a-s** (1 eq) in ACN (0.145 mmol/mL) was added Meldrum's acid (1.1 eq) at room temperature followed by trimethylorthoformate (1.2 eq). The resulting reaction mixture was heated at 90 ºC for 16 h in a round bottom flask and reflux. The mixture was cooled, precipitated with diethyl ether and filtered off. The solid was washed with diethyl ether and used in following steps without further purification. A round bottom flask with DPE (as less volume as possible, enough to cover the whole solid) was heated at 200 ºC until solvent started melting. Respective derivative of **23a-s,** obtained in previous step, was added in portions wise (gas evolution reaction), and then a balloon with Ar was placed on the septum of the reflux flask in order to leave CO₂ and acetone generated get out of system. The reaction mixture was stirred for 30 min-2 h at 200 ºC. Crude was left to cool at room temperature. The product was directly purified by flash chromatography.

### 2-(2-Chloro-pyrimidin-4-yl)-3-phenyl-thiazolo[3,2-a]pyrimidin-5-one (24a)

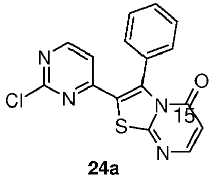

From 5-(2-chloro-pyrimidin-4-yl)-4-phenyl-thiazol-2-ylamine **(23a)** (1 eq, 1.285 g, 4.464 mmol). Purified by flash chromatography (DCM:MeOH 99.5:0.5 to 97:3). Product **24a** was a pale brown solid. (440 mg, Yield = 29%). LC-MS 2 method. Rt= 4.141 min, m/z = 341 [M+H]⁺. ¹H-NMR (300MHz, DMSO-*d*6): δ 8.53 (d, J = 5.4 Hz, 1H), 7.96 (d, J = 6.5 Hz, 1H), 7.65-7.47 (m, 5H), 6.21 (d, J = 5.4 Hz, 1H), 6.14 (d, J = 6.5 Hz, 1H). ¹³C-NMR (75MHz, DMSO-*d*6): δ 162.40, 160.83, 159.82, 158.85, 158.54, 152.46, 139.60, 130.91, 129.94, 129.26 (2C), 128.69 (2C), 122.30, 116.06, 107.84.

### 3-(2-Chloro-phenyl)-2-(2-chloro-pyrimidin-4-yl)-thiazolo[3,2-a]pyrimidin-5-one (24b)

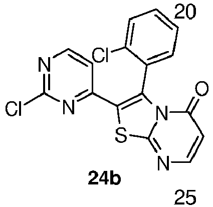

From 4-(2-Chloro-phenyl)-5-(2-chloro-pyrimidin-4-yl)-thiazol-2-ylamine **(23b)** (1 eq, 802 mg, 2.491 mmol). Purified by flash chromatography (DCM:MeOH 100:0 to 98:2). Product **24b** was a pale brown solid. (198 mg, Yield = 21%). LC-MS 2 method. Rt= 4.538 min, m/z = 375 [M+H]⁺. ¹H-NMR (300MHz, DMSO-*d*6): δ 8.63 (d, J = 5.4 Hz, 1H), 8.02 (d, J = 6.5 Hz, 1H), 7.71-7.63 (m, 4H), 6.33 (d, J = 5.4 Hz, 1H), 6.20 (d, J = 6.5 Hz, 1H). ¹³C-NMR (75MHz, DMSO-*d*6): δ 162.05, 161.58, 160.02, 158.28, 158.02, 152.84, 135.68, 132.94, 132.13, 131.20, 130.03, 129.44, 127.81, 123.43, 115.71, 107.69.

### 3-(3-Chloro-phenyl)-2-(2-chloro-pyrimidin-4-yl)-thiazolo[3,2-a]pyrimidin-5-one (24c)

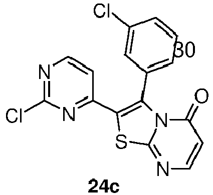

From 4-(3-Chloro-phenyl)-5-(2-chloro-pyrimidin-4-yl)-thiazol-2-ylamine **(23c)** (1 eq, 1.218 g, 3.784 mmol). Purified by flash chromatography (Cyclohexane:EtOAc 100:0 to 25:75). Product **24c** was a pale brown solid. (636 mg, Yield = 45%). LC-MS 2 method. Rt= 4.559 min, m/z = 375 [M+H]⁺. ¹H-NMR (300MHz, DMSO-*d*6): δ 8.61 (d, J = 5.4 Hz, 1H), 7.99 (d, J = 6.5 Hz, 1H), 7.56-7.53 (m, 4H), 6.38 (d, J = 5.4 Hz, 1H), 6.16 (d, J = 6.5 Hz, 1H). ¹³C-NMR (75MHz, DMSO-*d*6) δ 162.27, 161.20, 159.89, 158.60, 158.52, 152.58, 137.57, 133.16, 132.85, 130.48, 129.97, 129.24, 128.17, 122.84, 116.28, 107.84.

### 3-(4-Chloro-phenyl)-2-(2-chloro-pyrimidin-4-yl)-thiazolo[3,2-a]pyrimidin-5-one (24d)

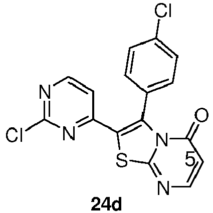

From 4-(4-Chloro-phenyl)-5-(2-chloro-pyrimidin-4-yl)-thiazol-2-ylamine **(23d)** (1 eq, 1.833 g, 5.693 mmol). Purified by flash chromatography (DCM:MeOH 99.5:0.5 to 97:3). Product **24d** was a pale brown solid. (215 mg, Yield = 10%). LC-MS 2 method. Rt= 4.566 min, m/z = 375 [M+H]⁺. ¹H-NMR (300MHz, DMSO-*d*6): δ 8.61 (d, J = 5.4 Hz, 1H), 7.97 (d, J = 6.5 Hz, 1H), 7.60 (m, 4H), 6.40 (d, J = 5.4 Hz, 1H), 6.15 (d, J = 6.5 Hz, 1H). ¹³C-NMR (75MHz, DMSO-*d*6): δ 164.63, 162.35, 161.20, 159.87, 158.66, 158.61, 152.54, 138.15, 134.77, 131.34 (2C), 128.76 (2C), 122.67, 116.33, 107.82.

### 2-(2-Chloro-pyrimidin-4-yl)-3-(2-methoxy-phenyl)-thiazolo[3,2-a]pyrimidin-5-one (24e)

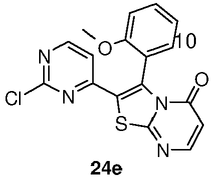

From 5-(2-Chloro-pyrimidin-4-yl)-4-(2-methoxy-phenyl)-thiazol-2-ylamine **(23e)** (1 eq, 1.715 g, 5.395 mmol). Purified by flash chromatography (DCM:MeOH 99:1 to 93:7). Product **24e** was a pale brown solid. (294 mg, Yield = 15%). LC-MS 2 method. Rt= 4.347 min, m/z = 371 [M+H]⁺. ¹H-NMR (300MHz, DMSO-*d*6): δ 8.58 (d, J = 5.4 Hz, 1H), 7.97 (d, J = 6.5 Hz, 1H), 7.59 (ddd, J = 8.4, 7.5, 1.7 Hz, 1H), 7.38 (dd, J = 7.5, 1.6 Hz, 1H), 7.21 (d, J = 8.1 Hz, 1H), 7.07 (td, J = 7.5, 0.8 Hz, 1H), 6.42 (d, J = 5.4 Hz, 1H), 6.16 (d, J = 6.5 Hz, 1H), 3.73 (s, 3H). ¹³C-NMR (75MHz, DMSO-*d*6): δ 162.29, 161.07, 159.87, 158.67, 158.29, 157.31, 152.45, 136.68, 132.15, 130.09, 122.21, 120.85, 119.48, 115.89, 111.60, 107.73, 55.86.

### 2-(2-Chloro-pyrimidin-4-yl)-3-(3-methoxy-phenyl)-thiazolo[3,2-alpyrimidin-5-one (24f)

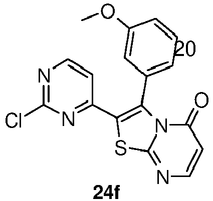

From 5-(2-Chloro-pyrimidin-4-yl)-4-(3-methoxy-phenyl)-thiazol-2-ylamine **(23f)** (1 eq, 1.995 g, 6.274 mmol). Purified by flash chromatography (Cyclohexane:EtOAc 50:50). Product **24f** was a pale brown solid. (595 mg, Yield = 26%). LC-MS 2 method. Rt= 4.423 min, m/z = 371 [M+H]⁺. ¹H-NMR (300MHz, DMSO-*d*6): δ 8.57 (d, J = 5.4 Hz, 1H), 7.97 (d, J = 6.5 Hz, 1H), 7.45-7.41 (m, 1H), 7.17 (m, 1H), 7.14-7.11 (m, 2H), 6.33 (d, J = 5.4 Hz, 1H), 6.15 (d, J = 6.5 Hz, 1H), 3.76 (s, 3H).

### 2-(2-Chloro-pyrimidin-4-yl)-3-(4-methoxy-phenyl)-thiazolo[3,2-a]pyrimidin-5-one (24g)

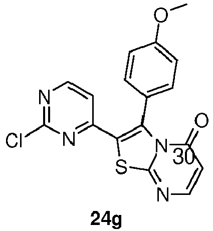

From 5-(2-Chloro-pyrimidin-4-yl)-4-(4-methoxy-phenyl)-thiazol-2-ylamine **(23g)** (1 eq, 1.082 g, 3.404 mmol). Purified by flash chromatography (Cyclohexane:EtOAc 100:0 to 0:100). Product **24g** was a pale brown solid. (205 mg, Yield = 16%). LC-MS 2 method. Rt= 4.356 min, m/z = 371 [M+H]⁺. ¹H-NMR (300MHz, DMSO-*d*6): δ 8.57 (d, J = 5.4 Hz, 1H), 7.95 (d, J = 6.5 Hz, 1H), 7.45 (d, J = 8.5 Hz, 2H), 7.07 (d, J = 8.5 Hz, 2H), 6.34 (d, J = 5.4 Hz, 1H), 6.13 (d, J = 6.5 Hz, 1H), 3.85 (s, 3H).

### 2-(2-Chloro-pyrimidin-4-yl)-3-(4-fluoro-phenyl)-thiazolo[3,2-a]pyrimidin-5-one (24h)

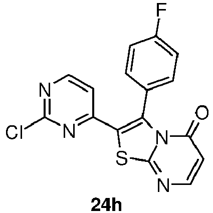

From 5-(2-Chloro-pyrimidin-4-yl)-4-(4-fluoro-phenyl)-thiazol-2-ylamine **(23h)** (1 eq, 1.396 g, 4.564 mmol). Purified by flash chromatography (Cyclohexane:EtOAc 100:0 to 0:100). Product **24h** was a pale brown solid. (895 mg, Yield = 55%). LC-MS 1 method. Rt= 4.988 min, m/z = 359 [M+H]⁺. ¹H-NMR (300MHz, DMSO-*d*6): δ 8.59 (d, J = 5.4 Hz, 1H), 7.97 (d, J = 6.5 Hz, 1H), 7.65-7.60 (m, 2H), 7.46-7.25 (m, 2H), 6.34 (d, J = 5.4 Hz, 1H), 6.15 (d, J = 6.5 Hz, 1H).

### 2-(2-Chloro-pyrimidin-4-yl)-3-(2,3-dichloro-phenyl)-thiazolo[3,2-a]pyrimidin-5-one (24i)

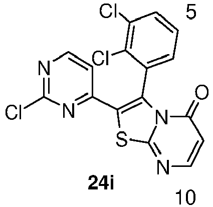

From 5-(2-Chloro-pyrimidin-4-yl)-4-(2,3-dichloro-phenyl)-thiazol-2-ylamine **(23i)** (1 eq, 1.194 g, 3.355 mmol). Purified by flash chromatography (DCM:MeOH 100:0 to 97:3). Product **24i** was a pale brown solid. (260 mg, Yield = 19%). LC-MS 2 method. Rt= 4.669 min, m/z = 409 [M+H]⁺. ¹H-NMR (300MHz, DMSO-*d*6): δ 8.67 (d, J = 5.4 Hz, 1H), 8.04 (d, J = 6.5 Hz, 1H), 7.90 (dd, J = 8.0, 1.6 Hz, 1H), 7.64 (dd, J = 7.7, 1.6 Hz, 1H), 7.52 (dd, J = 8.9, 6.9 Hz, 1H), 6.50 (d, J = 5.4 Hz, 1H), 6.22 (d, J = 6.5 Hz, 1H). ¹³C-NMR (75MHz, DMSO-*d*6) δ 161.93, 161.86, 160.01, 158.30, 157.69, 152.93, 134.64, 132.41, 132.23, 132.08, 131.39, 129.91, 128.89, 123.70, 116.09, 107.63.

### 2-(2-Chloro-pyrimidin-4-yl)-3-(3,4-dichloro-phenyl)-thiazolo[3,2-a]pyrimidin-5-one (24j)

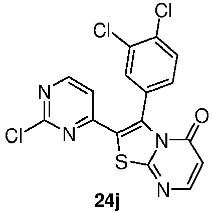

From 5-(2-Chloro-pyrimidin-4-yl)-4-(3,4-dichloro-phenyl)-thiazol-2-ylamine **(23j)** (1 eq, 1.393 g, 3.914 mmol). Purified by flash chromatography (DCM:MeOH 100:0 to 98.4:1.6). Product **24j** was a pale brown solid. (717 mg, Yield = 45%). LC-MS 2 method. Rt= 4.665 min, m/z = 409 [M+H]⁺. ¹H-NMR (300MHz, DMSO-*d*6): δ 8.64 (d, J = 5.4 Hz, 1H), 7.99 (d, J = 6.5 Hz, 1H), 7.94 (d, J = 1.9 Hz, 1H), 7.80 (d, J = 8.3 Hz, 1H), 7.61-7.56 (m, 1H), 6.56 (d, J = 5.4 Hz, 1H), 6.17 (d, J = 6.5 Hz, 1H). ¹³C-NMR (75MHz, DMSO-*d*6) δ 162.19, 161.49, 159.89, 158.71, 158.30, 152.63, 136.39, 132.87, 131.52, 131.36, 131.31, 130.82, 129.87, 123.19, 116.62, 107.82.

### 3-(3-Chloro-4-methoxy-phenyl)-2-(2-chloro-pyrimidin-4-yl)-thiazolo[3,2-idin-5-one (24k)

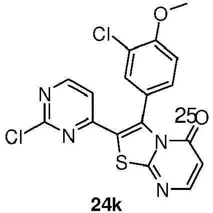

From 4-(3-Chloro-4-methoxy-phenyl)-5-(2-chloro-pyrimidin-4-yl)-thiazol-2-ylamine **(23k)** (1 eq, 1.196 g, 3.397 mmol). Purified by flash chromatography (DCM:MeOH 100:0 to 98:2). Product 24k was a pale brown solid. (841 mg, Yield = 61%). LC-MS 2 method. Rt= 4.563 min, m/z = 405 [M+H]⁺. ¹H-NMR (300MHz, DMSO-*d*6): δ 8.61 (d, J = 5.4 Hz, 1H), 7.96 (d, J = 6.5 Hz, 1H), 7.69 (d, J = 2.1 Hz, 1H), 7.49 (dd, J = 8.5, 2.1 Hz, 1H), 7.28 (d, J = 8.6 Hz, 1H), 6.47 (d, J = 5.4 Hz, 1H), 6.14 (d, J = 6.5 Hz, 1H), 3.96 (s, 3H). ¹³C-NMR (75MHz, DMSO-*d*6): δ 162.29, 161.14, 159.84, 158.79, 158.72, 155.62, 152.43, 138.06, 130.87, 129.74, 123.52, 122.81, 121.09, 116.33, 112.66, 107.91, 56.34.

### 2-(2-Chloro-pyrimidin-4-yl)-3-(4-fluoro-3-trifluoromethyl-phenyl)-thiazolo[3,2-a]pyrimidin-5-one (24l)

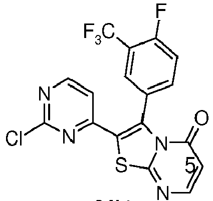

From 5-(2-Chloro-pyrimidin-4-yl)-4-(4-fluoro-3-trifluoromethyl-phenyl)-thiazol-2-ylamine (**23l**) (1 eq, 269 mg, 0.719 mmol). Residue was directly used in next steps without further purification. Product **24l** was a pale yellow solid. (219 mg, Yield =71%). LC-MS 2 method. Rt= 4.639 min, m/z = 427 [M+H]⁺.

### 2-(2-Chloro-pyrimidin-4-yl)-3-isopropyl-thiazolo[3,2-a]pyrimidin-5-one (24m)

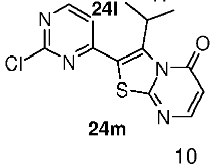

From 4-isopropyl-5-(2-methylsulfanyl-pyrimidin-4-yl)-thiazol-2-ylamine **(23m)** (1 eq, 419 mg, 1.651 mmol). Purified by flash chromatography (Cyclohexane:EtOAc 100:0 to 0:100). Product **24m** was a pale brown solid. (132 mg, Yield = 26%). LC-MS 2 method. Rt= 4.261 min, m/z = 307 [M+H]⁺. ¹H-NMR (300MHz, DMSO-*d*6): δ 8.93 (d, J = 5.2 Hz, 1H), 7.97 (d, J = 6.4 Hz, 1H), 7.80 (d, J = 5.2 Hz, 1H), 6.31 (d, J = 6.4 Hz, 1H), 4.05-3.89 (m, 1H), 1.41 (s, 3H), 1.39 (s, 3H).

### 2-(2-Chloro-pyrimidin-4-yl)-3-cyclopropyl-thiazolo[3,2-a]pyrimidin-5-one (24n)

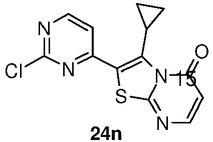

From 5-(2-chloro-pyrimidin-4-yl)-4-cyclopropyl-thiazol-2-ylamine **(23n)** (1 eq, 354 mg, 1.403 mmol). Purified by flash chromatography (Cyclohexane:EtOAc 100:0 to 0:100). Product **24n** was a pale yellow solid. (60 mg, Yield = 14%). LC-MS 2 method. Rt= 3.962 min, m/z = 305 [M+H]⁺. ¹H-NMR (300MHz, DMSO-*d*6): δ 8.93 (d, J = 5.3 Hz, 1H), 8.11 (d, J = 5.3 Hz, 1H), 7.93 (d, J = 6.5 Hz, 1H), 6.26 (d, J = 6.5 Hz, 1H), 2.60- 2.53 (m, 1H), 1.15-1.06 (m, 2H), 0.58 (q, J = 6.2 Hz, 2H).

### 2-(2-Chloro-pyrimidin-4-yl)-3-(tetrahydro-pyran-4-yl)-thiazolo[3,2-a]pyrimidin-5-one (24o)

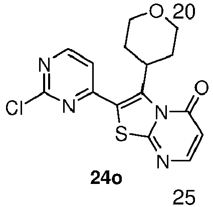

From 5-(2-chloro-pyrimidin-4-yl)-4-(tetrahydro-pyran-4-yl)-thiazol-2-ylamine (**23o**) (1 eq, 663 mg, 2.240 mmol). Purified by flash chromatography (Cyclohexane:EtOAc 100:0 to 0:100). Product **24o** was a pale yellow solid. (60 mg, Yield = 14%). LC-MS 2 method. Rt= 3.837 min, m/z = 349 [M+H]⁺. ¹H-NMR (700MHz, DMSO-*d*6): δ 8.95 (d, J = 5.1 Hz, 1H), 7.97 (d, J = 6.4 Hz, 1H), 7.84 (d, J = 5.1 Hz, 1H), 6.31 (d, J = 6.4 Hz, 1H), 3.88 (dd, J = 11.2, 4.0 Hz, 2H), 3.49 (m, 1H), 3.35-3.30 (m, 2H), 1.98-1.77 (m, 2H), 1.68 (m, 2H).

### 2-(2-Chloro-5-methoxy-pyrimidin-4-yl)-3-phenyl-thiazolo[3,2-idin-5-one (24r)

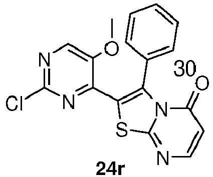

From 5-(2-chloro-5-methoxy-pyrimidin-4-yl)-4-phenyl-thiazol-2-ylamine **(23r)** (1 eq, 215 mg, 0.677 mmol). Purified by flash chromatography (Cyclohexane:EtOAc 80:20 to 20:80). Product **24r** was a pale brown solid. (189.1 mg, Yield = 75%). LC-MS 2 method. Rt= 4.223 min, m/z = 371 [M+H]⁺. ¹H-NMR (300MHz, DMSO-*d*6): δ 8.55 (s, 1H), 7.99 (d, J = 6.5 Hz, 1H), 7.40 - 7.22 (m, 5H), 6.18 (d, J = 6.4 Hz, 1H).

### General Procedure for the synthesis of intermediates 25

**23a** (1 eq.), corresponding β-keto ester (20 eq.) and BiCl₃ (0.2 eq.) were added to a pressure tube. Then, DPE (as less volume as possible, enough to cover the whole mixture) was added and mixture was heated at 140-160 ºC for 1 h. When the intermediate was completely formed, the temperature was increased to 200 ºC during 1-4 h. Crude was left to cool at room temperature. The crude was directly purified by flash chromatography to obtain the desired product.

### 2-(2-Chloro-pyrimidin-4-yl)-5-oxo-3-phenyl-5H-thiazolo[3,2-a]pyrimidine-6-carboxylic acid ethyl ester (25a)

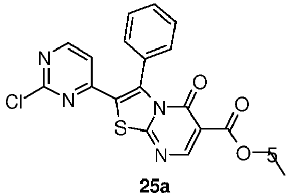

From 5-(2-chloro-pyrimidin-4-yl)-4-phenyl-thiazol-2-ylamine **(23a)** (1 eq, 500 mg, 1.732 mmol) and diethyl ethoxymethylenemalonate (1.5 eq, 0.52 mL, 2.597 mmol) as starting materials. Desired product was purified by flash chromatography (Cyclohexane:(EtOAc:MeOH 9:1) 90:10 to 0:100). Product **25a** was a pale brown solid. (213 mg, Yield = 30%). LC-MS 2 method. Rt= 4.616 min, m/z = 413 [M+H]⁺. ¹H-NMR (700MHz, DMSO-*d*6): δ 8.58 (s, 1H), 8.56 (d, J = 5.4 Hz, 1H), 7.58 (m, 5H), 6.20 (d, J = 5.4 Hz, 1H), 4.18 (q, J = 7.0 Hz, 2H), 1.21 (t, J = 7.0 Hz, 3H). ¹³C-NMR (75MHz, DMSO) δ 166.49, 163.63, 161.11, 159.88, 158.48, 157.08, 155.17, 140.50, 130.76, 130.03, 129.19 (2C), 128.78 (2C), 123.96, 116.12, 109.03, 60.41, 14.20.

### 2-(2-Chloro-pyrimidin-4-yl)-7-methyl-3-phenyl-thiazolo[3,2-a]pyrimidin-5-one (25b)

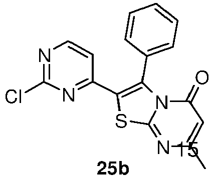

From 5-(2-chloro-pyrimidin-4-yl)-4-phenyl-thiazol-2-ylamine **(23a)** (1 eq, 200 mg, 0.693 mmol) and ethyl acetoacetate (2 eq, 0.175 mL, 1.385 mmol) as starting materials. Desired product was purified by flash chromatography (DCM:MeOH 100:0 to 95:5). Product **25b** was a pale brown solid. (37 mg, Yield = 15%). LC-MS 2 method. Rt= 4.494 min, m/z = 355 [M+H]⁺. ¹H-NMR (700MHz, DMSO-*d*6): δ 8.52 (d, J = 5.4 Hz, 1H), 7.64-7.47 (m, 5H), 6.20 (d, J = 5.4 Hz, 1H), 6.03 (s, 1H), 2.27 (s, 3H).

### 2-(2-Chloro-pyrimidin-4-yl)-7-isopropyl-3-phenyl-thiazolo[3,2-a]pyrimidin-5-one (25c)

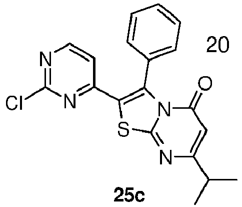

From 5-(2-chloro-pyrimidin-4-yl)-4-phenyl-thiazol-2-ylamine **(23a)** (1 eq, 200 mg, 0.693 mmol) and ethyl isobutyrylacetate (20 eq, 2 mL, 13.85 mmol) as starting materials. Desired product was purified by flash chromatography (Cyclohexane:EtOAc 100:0 to 0:100). Product **25c** was a pale brown solid. (103 mg, Yield = 39%). LC-MS 2 method. Rt= 4.915 min, m/z = 383 [M+H]⁺.

### 2-(2-Chloro-pyrimidin-4-yl)-7-ethyl-3-phenyl-thiazolo[3,2-a]pyrimidin-5-one (25d)

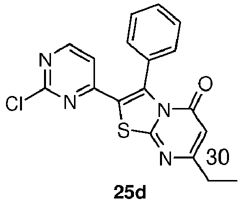

From 5-(2-chloro-pyrimidin-4-yl)-4-phenyl-thiazol-2-ylamine **(23a)** (1 eq, 200 mg, 0.693 mmol) and ethyl propionylacetate (20 eq, 2 mL, 13.85 mmol) as starting materials. Desired product was purified by flash chromatography (Cyclohexane:EtOAc 100:0 to 0:100). Product **25d** was a pale brown solid. (73 mg, Yield = 29%). LC-MS 2 method. Rt= 4.709 min, m/z = 369 [M+H]⁺.

### 2-(2-Chloro-pyrimidin-4-yl)-6,7-dimethyl-3-phenyl-thiazolo[3,2-a]pyrimidin-5-one (25e)

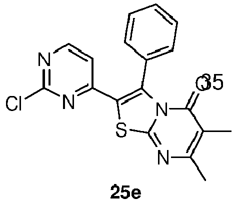

From 5-(2-chloro-pyrimidin-4-yl)-4-phenyl-thiazol-2-ylamine **(23a)** (1 eq, 200 mg, 0.693 mmol) and ethyl 2-methylacetoacetate (20 eq, 2 mL, 13.85 mmol) as starting materials. Desired product was purified by flash chromatography (Cyclohexane:EtOAc 100:0 to 50:50). Product **25e** was a pale brown solid. (68 mg, Yield = 27%). LC-MS 2 method. Rt= 4.647 min, m/z = 369 [M+H]⁺. ¹H-NMR (700MHz, DMSO-*d*6): δ 8.51 (d, J = 5.4 Hz, 1H), 7.62-7.40 (m, 5H), 6.20 (d, J = 5.4 Hz, 1H), 2.30 (s, 3H), 1.87 (s, 3H).

### 2-(2-Chloro-pyrimidin-4-yl)-6-isopropyl-7-methyl-3-phenyl-thiazolo[3,2-a]pyrimidin-5-one (25f)

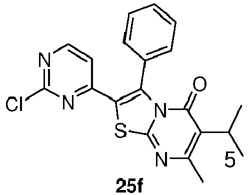

From 5-(2-chloro-pyrimidin-4-yl)-4-phenyl-thiazol-2-ylamine **(23a)** (1 eq, 200 mg, 0.693 mmol) and ethyl 2-acetyl-3-methylbutanoate (20 eq, 2.48 mL, 13.85 mmol) as starting materials. Desired product was purified by flash chromatography (Cyclohexane:EtOAc 100:0 to 0:100). Product **25f** was a pale brown solid. (82 mg, Yield = 30%). LC-MS 2 method. Rt= 4.989 min, m/z = 397 [M+H]⁺.

### 2-(2-Chloro-pyrimidin-4-yl)-6-ethyl-7-methyl-3-phenyl-thiazolo[3,2-a]pyrimidin-5-one (25g)

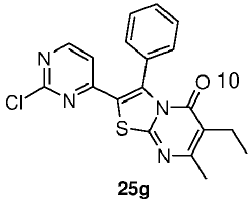

From 5-(2-chloro-pyrimidin-4-yl)-4-phenyl-thiazol-2-ylamine **(23a)** (1 eq, 257 mg, 0.890 mmol) and ethyl 2-ethylacetoacetate (20 eq, 2.87 mL, 17.8 mmol) as starting materials. Desired product was purified by flash chromatography (Cyclohexane:EtOAc 100:0 to 0:100). Product **25g** was a pale brown solid. (93 mg, Yield = 27%). LC-MS 2 method. Rt= 4.886 min, m/z = 383 [M+H]⁺.

### General Procedure for the synthesis of intermediates 28

**Step (1):** A mixture of 1-fluoro-3-nitrobenzene (1 eq), respective piperazine (2 eq) and K₂CO₃ (1.5 eq) in DMSO (1 mmol/mL) was heated in a sealed tube at 90 ºC for 16 h. On cooling, EtOAc was added and the mixture was washed with H₂O and brine. The organic layer was dried with Na₂SO₄, filtered and evaporated. Product **27a-b** was purified by flash chromatography.

**Step (2):** To a solution of **27a-b** (1 eq) in DCM/MeOH (1:1, 0.112 mmol/mL), ammonium chloride (10 eq) was added followed by zinc (8 eq). The mixture was stirred at RT for 16 h. The reaction was filtered through celite, washed with DCM/MeOH (1:1) and then evaporated. NaHCO₃ (sat) was added to the obtained residue. The aqueous phase was extracted with CHC₃:iPrOH (1:1). The organic layers were mixed and dried with Na₂SO₄. The solvent was evacuated under reduced pressure. The resulting residue was purified by flash chromatography to render **28a-b.**

### 3-(4-Methyl-piperazin-1-yl)-phenylamine (28a)

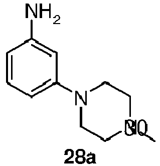

*1-Methyl-4-(3-nitro-phenyl)-piperazine (27a).* From 1-fluoro-3-nitrobenzene (1 eq, 1.509 mL, 14.174 mmol) and 1-methylpiperazine (2 eq, 3.141 mL, 28.349 mmol) as starting materials following Step (1). **27a** product was an orange oil and was used in next steps without further purification. (3.2 g, Yield = 100%). LC-MS 2 method. Rt= 0.661 min, m/z = 222 [M+H]⁺. *3-(4-Methyl-piperazin-1-yl)-phenylamine (28a).* From 1-methyl-4-(3-nitro-phenyl)-piperazine **(27a)** (1 eq, 3.2 g, 14.463 mmol) as starting materials following Step (2). Purified by flash chromatography (DCM:MeOH 100:0 to 85:15). **28a** was an orange solid (580 mg, Yield = 21%). LC-MS 2 method. Rt= 0.314 min, m/z = 192 [M+H]⁺. ¹H-NMR (300MHz, DMSO-*d*6): δ 6.83 (t, J = 7.9 Hz, 1H), 6.11 (m, 2H), 6.02 (d, J = 8.8 Hz, 1H), 4.84 (s, 2H), 3.01 (s, 4H), 2.41 (s, 4H), 2.20 (s, 3H).

### 2-[4-(3-Amino-phenyl)-piperazin-1-yl]-ethanol (28b)

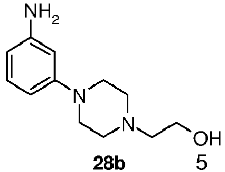

*2-[4-(3-Nitro-phenyl)-piperazin-1-yl]-ethanol (27b).* From 1-fluoro-3-nitrobenzene (1 eq, 2.264 mL, 21.261 mmol) and 1-piperazineethanol (2 eq, 5.218 mL, 42.523 mmol) as starting materials following Step (1). Purified by flash chromatography (DCM:MeOH 98:2 to 92:8). (3.85 g, Yield = 72%). LC-MS 2 method. Rt= 0.582 min, m/z = 252 [M+H]⁺. ¹H-NMR (300MHz, CDCl₃-*d*1): δ 7.70 (s, 1H), 7.64 (d, J = 8.0 Hz, 1H), 7.36 (s, 1H), 7.17 (d, J = 10.2 Hz, 1H), 3.74-3.61 (m, 2H), 3.35-3.23 (m, 4H), 2.74-2.66 (m, 4H), 2.60 (m, 2H).
*2-[4-(3-Amino-phenyl)-piperazin-1-yl]-ethanol (28b)*. From 2-[4-(3-nitro-phenyl)-piperazin-1-yl]-ethanol **(27b)** (1 eq, 3.85 g, 15.321 mmol) as starting materials following Step (2). Purified by flash chromatography (EtOAc:(EtOAc:MeOH/NH₃ 7N 9:1) 65:35 to 0:100). **28b** was a brown solid (1.12 mg, Yield = 33%). LC-MS 2 method. Rt= 0.325 min, m/z = 222 [M+H]⁺.
¹H-NMR (300MHz, DMSO-*d*6): δ 6.83 (t, J = 7.9 Hz, 1H), 6.11 (m, 2H), 6.05-5.99 (m, 1H), 4.85 (br, 2H), 4.45 (t, J = 5.3 Hz, 1H), 3.52 (q, J = 6.1 Hz, 2H), 3.07-2.95 (m, 4H), 2.53-2.48 (m, 4H), 2.41 (t, J = 6.3 Hz, 2H).

### B. Biological tests

**DDR1/2 binding assay:** The binding assay relies on the LanthaScreen ^{™} Eu-Kinase Binding Assay (ThermoFisher Scientfic). This is a kinase assay platform based on measuring the binding and displacement of an Alexa Fluor^{®} conjugate of an ATP-competitive kinase inhibitor or tracer at a kinase active site (Kinase Tracer 178. #PV5593). Binding of the tracer to the kinase is detected by addition of a europium (Eu)-labeled anti-GST antibody (#PV 5594) which specifically labels the kinase of interest. This binding results in a high degree of fluorescence resonance energy transfer (FRET), whereas displacement of the tracer with a kinase inhibitor results in a loss of FRET. The enzymes were purchased as a catalytic domain GST Tag DDR1(#PR6913A) and DDR2 (#PR7167A) from ThermoFisher. Assay conditions were as indicated by the kit manufacturers. Assays were performed in 384-well plates. The final read out was generated using an EnVision plate reader (Perkin-Elmer). The emission ratio was calculated by dividing the acceptor/tracer emission (665 nm) by the antibody/donor emission (615 nm). Values were plotted against the inhibitor concentration and fit to a sigmoid dose-response curve using Activity base by IDBS software.

**DDR1/2 cellular assay:** Transient transfection of HEK293T cells with DDR1 plasmid is used to study the inhibition of the autophosphorylation of DDR1 induced by Collagen I in cells. HEK293T are seeded, 6x10⁵ cells per well in 6-well plates and incubated for 6 h at 37 °C, 5% CO₂. Then, cells are transfected using Effectene reactive (Quiagen) with pcDNA3.1 DDR1 WT mouse plasmid. Cells are incubated with transfection complexes under normal growth conditions for 24 h. After that, medium is replaced with fresh medium and cells are incubated for an additional 18 h. Eight serial 1:3 compound dilutions are made in DMSO. The compounds are added to each well of 6 well cell plates and are incubated at 37 °C under CO₂ atmosphere 1 h. DDR1's autophosphorylation is stimulated by 1h treatment with 10 µg/mL Collagen I. Then, cells are washed in PBS, lyse adding 100 µl of complete RIPA protein lysis buffer (RIPA Buffer, 1 mM DTT, 5 mM Bezamidine, 10 µg/mL Aprotinin, 10 µg/mL Leupeptin, 710 ng/mL Pepstatin A, 100 µg/mL Trypsin Inhibitor, 40 µg/mL Bestatin, completed with PhosSTOP EASYpack and complete Mini, EDTA-free) and incubated 20 minutes at 4 ºC and then centrifugated. Lysates are kept at -80 ºC. The protein content of the lysates is determined by DC protein assay (Biorad, Ref. 5000116). The proteins are resolved by SDS-PAGE and transferred to nitrocellulose membrane (GE Healthcare #10600003). The membranes are incubated overnight at 4 ºC with antibodies specific for DDR1 (D1G6) (Cell signaling #5583), P-DDR1 (Y513) (E1N8F) (Cell Signaling # 14531) they are washed and then incubated with Alexa Fluor 680 goat anti-rabbit IgG secondary antibodies (Invitrogen, A21076). The bands are visualized and quantified using an Odyssey infrared imaging system (Li-Cor Biosciences). The percentage of DDR1 phosphorylation is finally plotted against concentration for each compound and EC₅₀s for intracellular DDR1 inhibition are calculated using ActivityBase from IDBS.

Compounds of the invention were found to inhibit DDR1 and DDR2, for example as tested in the binding and cellular assay described hereinbefore. Biological activity is represented in Table 1.

**Table 1: Inhibition of DDR1/2 biochemical and cellular activity expressed as IC50, EC50 values [M] for the compounds of the examples.**

| **Cpd number** | **DDR1 IC₅₀** | **DDR2 IC₅₀** | **pDDR1 EC₅₀** | **Cpd number** | **DDR1 IC₅₀** | **DDR2 IC₅₀** | **pDDR1 EC₅₀** |
|---|---|---|---|---|---|---|---|
| **29** | 6,99E-08 | 1,86E-07 | 3,39E-07 | **54** | 4,19E-09 | 2,88E-08 | 1,82E-08 |
| **30** | 1,39E-08 | 5,00E-08 | 8,74E-08 | **55** | 2,28E-09 | 6,37E-09 | 2,18E-08 |
| **31** | 1,55E-08 | 1,80E-08 | 1,92E-07 | **56** | 5,63E-09 | 1,75E-08 | 1,22E-08 |
| **34** | 1,34E-08 | 3,94E-08 | 1,57E-08 | **57** | 4,54E-09 | 1,50E-08 | 2,11E-08 |
| **37** | 5,19E-09 | 2,42E-08 | 3,60E-08 | **58** | 6,40E-09 | 3,05E-08 | 2,80E-08 |
| **38** | 4,22E-09 | 1,66E-08 | 2,81E-08 | **59** | 1,02E-08 | 4,73E-08 | 9,93E-08 |
| **39** | 7,03E-09 | 2,15E-08 | 3,53E-08 | **60** | 5,43E-09 | 1,83E-08 | 3,19E-08 |
| **40** | 1,64E-08 | 6,16E-08 | 1,13E-07 | **61** | 1,29E-08 | 4,08E-08 | 6,32E-08 |
| **41** | 6,24E-09 | 3,69E-08 | 4,04E-08 | **62** | 1,09E-08 | 5,40E-08 | 8,27E-08 |
| **42** | 5,80E-09 | 1,79E-08 | 3,07E-08 | **63** | 1,19E-07 | 3,25E-07 | 1,42E-07 |
| **43** | 1,16E-08 | 2,90E-08 | 3,14E-08 | **64** | 1,53E-08 | 9,06E-08 | 5,87E-08 |
| **44** | 2,73E-09 | 1,17E-08 | 2,49E-08 | **65** | 1,18E-07 | 4,66E-07 | 1,63E-07 |
| **45** | 4,23E-09 | 1,26E-08 | 7,63E-09 | **66** | 3,21E-07 | 1,48E-06 | 6,10E-07 |
| **46** | 5,30E-09 | 2,01E-08 | 3,06E-08 | **69** | 6,88E-06 | 8,62E-06 | N/A |
| **47** | 9,50E-09 | 2,44E-08 | 2,78E-08 | **71** | 1,47E-07 | 4,23E-07 | 3,46E-08 |
| **48** | 1,93E-08 | 7,12E-08 | 2,75E-08 | **72** | 9,06E-09 | 2,69E-08 | 5,90E-08 |
| **49** | 2,09E-08 | 1,03E-07 | 9,98E-08 | **73** | 1,25E-08 | 3,33E-08 | N/A |
| **50** | 2,11E-08 | 7,90E-08 | 3,34E-08 | **74** | 1,29E-08 | 1,16E-08 | 3,95E-08 |
| **51** | 8,34E-09 | 2,77E-08 | 1,92E-08 | **75** | 1,53E-08 | 5,58E-08 | 1,41E-07 |
| **52** | 1,09E-08 | 4,06E-08 | 1,12E-07 | **76** | 6,44E-08 | 2,92E-07 | N/A |
| **53** | 5,46E-09 | 1,68E-08 | 1,93E-08 | **77** | 2,55E-08 | 6,94E-08 | 4,68E-08 |

## Claims

1. A compound of formula (I): 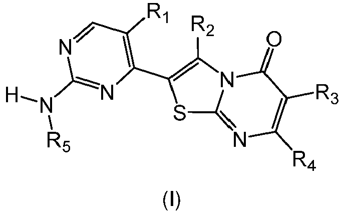 wherein:
R₁ is selected from H, halo, CN, O-alkyl C₁-C₆, alkyl C₁-C₆ optionally substituted by halo;
R₂ is selected from aryl optionally substituted by at least a Q₁ group, heteroaryl optionally substituted by at least a Q₂ group, alkyl C₁-C₆ optionally substituted by at least a Q₃ group, cycloalkyl C₃-C₆ optionally substituted by at least a Q₄ group, heterocycloalkyl C₃-C₆ optionally substituted by at least a Q₅ group, or CONRaRb being Ra and Rb independently selected from H or alkyl C₁-C₆;
R₃ and R₄ are independently selected from H, alkyl C₁-C₆ optionally substituted by at least a Q₆ group, cycloalkyl C₃-C₆ optionally substituted by at least a Q₇ group, heterocycloalkyl C₃-C₆ optionally substituted by at least a Q₈ group, COORc or CONRdRe, being Rc, Rd and Re independently selected from H or alkyl C₁-C₆;
R₅ is selected from aryl optionally substituted by at least a Q₉ group or heteroaryl optionally substituted by at least a Q₁₀ group;
groups Q₁ Q₂ Q₃ Q₄ Q₅ Q₆ Q₇ Q₈ Q₉ Q₁₀ are independently selected from halo, OH, CF₃, CN, SO₂NH₂, C(O)ORx, C(O)NRyRz, being Rx, Ry and Rz independently selected from H or alkyl C₁-C₄, O-alkyl C₁-C₆ optionally substituted by OH or alkyl C₁-C₄, alkyl C₁-C₆ optionally substituted by OH or alkyl C₁-C₄, cycloalkyl C₃-C₆ optionally substituted by OH or alkyl C₁-C₄, heterocycloalkyl C₃-C₆ optionally substituted by alkyl C₁-C₄ which may be optionally substituted by OH;
or a pharmaceutically acceptable ester, amide, solvate or salt thereof.

2. The compound according to claim 1, wherein R₁ is selected from H or O-alkyl C₁-C₄, preferably O-CH₃.

3. The compound according to any one of claims 1 or 2, wherein R₂ is selected from aryl optionally substituted by at least a Q₁ group, alkyl C₁-C₆ optionally substituted by at least a Q₃ group, cycloalkyl C₃-C₆ optionally substituted by at least a Q₄ group, heterocycloalkyl C₃-C₆ optionally substituted by at least a Q₅ group.

4. The compound according to claim 3, wherein R₂ is phenyl, optionally substituted by at least one group selected from OH, halo, O-alkyl C₁-C₄ or CF₃.

5. The compound according to any one of claims 1 to 4, wherein R₃ and R₄ are independently selected from H, alkyl C₁-C₄ or COORa, being Ra an alkyl C₁-C₄.

6. The compound according to any one of claims 1 to 5, wherein R₅ is selected from phenyl optionally substituted by at least a Q₉ group, wherein Q₉ is selected from SO₂NH₂ or the following group: 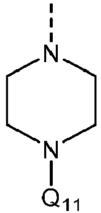 being Q₁₁ an alkyl C₁-C₄ optionally substituted by OH.

7. The compound according to claim 1, wherein said compound is selected from the following list:
- 3-Phenyl-2-(2-phenylamino-pyrimidin-4-yl)-thiazolo[3,2-a]pyrimidin-5-one **(29)**
- 3-[4-(5-Oxo-3-phenyl-5H-thiazolo[3,2-a]pyrimidin-2-yl)-pyrimidin-2-ylamino]-benzenesulfonamide **(30)**
- 2-(2-{3-[4-(2-Hydroxy-ethyl)-piperazin-1-yl]-phenylamino}-pyrimidin-4-yl)-3-phenyl-thiazolo[3,2-a]pyrimidin-5-one **(31)**
- 2-{2-[4-(4-Methyl-piperazin-1-yl)-phenylamino]-pyrimidin-4-yl}-3-phenyl-thiazolo[3,2-a]pyrimidin-5-one **(34)**
- 3-{4-[3-(2-Chloro-phenyl)-5-oxo-5H-thiazolo[3,2-a]pyrimidin-2-yl]-pyrimidin-2-ylamino}-benzenesulfonamide **(37)**
- 3-(2-Chloro-phenyl)-2-(2-{3-[4-(2-hydroxy-ethyl)-piperazin-1-yl]-phenylamino}-pyrimidin-4-yl)-thiazolo[3,2-a]pyrimidin-5-one **(38)**
- 3-(2-Chloro-phenyl)-2-{2-[4-(4-methyl-piperazin-1-yl)-phenylamino]-pyrimidin-4-yl}-thiazolo[3,2-a]pyrimidin-5-one **(39)**
- 3-{4-[3-(3-Chloro-phenyl)-5-oxo-5H-thiazolo[3,2-a]pyrimidin-2-yl]-pyrimidin-2-ylamino}-benzenesulfonamide **(40)**
- 3-(3-Chloro-phenyl)-2-(2-{3-[4-(2-hydroxy-ethyl)-piperazin-1-yl]-phenylamino}-pyrimidin-4-yl)-thiazolo[3,2-a]pyrimidin-5-one **(41)**
- 3-(3-Chloro-phenyl)-2-{2-[4-(4-methyl-piperazin-1-yl)-phenylamino]-pyrimidin-4-yl}-thiazolo[3,2-a]pyrimidin-5-one **(42)**
- 3-{4-[3-(4-Chloro-phenyl)-5-oxo-5H-thiazolo[3,2-a]pyrimidin-2-yl]-pyrimidin-2-ylamino}-benzenesulfonamide **(43)**
- 3-(4-Chloro-phenyl)-2-(2-{3-[4-(2-hydroxy-ethyl)-piperazin-1-yl]-phenylamino}-pyrimidin-4-yl)-thiazolo[3,2-a]pyrimidin-5-one **(44)**
- 3-(4-Chloro-phenyl)-2-{2-[4-(4-methyl-piperazin-1-yl)-phenylamino]-pyrimidin-4-yl}-thiazolo[3,2-a]pyrimidin-5-one **(45)**
- 3-{4-[3-(2-Methoxy-phenyl)-5-oxo-5H-thiazolo[3,2-a]pyrimidin-2-yl]-pyrimidin-2-ylamino}-benzenesulfonamide **(46)**
- 2-(2-{3-[4-(2-Hydroxy-ethyl)-piperazin-1-yl]-phenylamino}-pyrimidin-4-yl)-3-(2-methoxy-phenyl)-thiazolo[3,2-a]pyrimidin-5-one **(47)**
- 3-(2-Methoxy-phenyl)-2-{2-[4-(4-methyl-piperazin-1-yl)-phenylamino]-pyrimidin-4-yl}-thiazolo[3,2-a]pyrimidin-5-one **(48)**
- 3-{4-[3-(3-Methoxy-phenyl)-5-oxo-5H-thiazolo[3,2-a]pyrimidin-2-yl]-pyrimidin-2-ylamino}-benzenesulfonamide **(49)**
- 2-(2-{3-[4-(2-Hydroxy-ethyl)-piperazin-1-yl]-phenylamino}-pyrimidin-4-yl)-3-(3-methoxy-phenyl)-thiazolo[3,2-a]pyrimidin-5-one **(50)**
- 3-(3-Methoxy-phenyl)-2-{2-[4-(4-methyl-piperazin-1-yl)-phenylamino]-pyrimidin-4-yl}-thiazolo[3,2-a]pyrimidin-5-one **(51)**
- 3-{4-[3-(4-Methoxy-phenyl)-5-oxo-5H-thiazolo[3,2-a]pyrimidin-2-yl]-pyrimidin-2-ylamino}-benzenesulfonamide **(52)**
- 2-(2-{3-[4-(2-Hydroxy-ethyl)-piperazin-1-yl]-phenylamino}-pyrimidin-4-yl)-3-(4-methoxy-phenyl)-thiazolo[3,2-a]pyrimidin-5-one **(53)**
- 3-(4-Methoxy-phenyl)-2-{2-[4-(4-methyl-piperazin-1-yl)-phenylamino]-pyrimidin-4-yl}-thiazolo[3,2-a]pyrimidin-5-one **(54)**
- 3-(2-Hydroxy-phenyl)-2-{2-[4-(4-methyl-piperazin-1-yl)-phenylamino]-pyrimidin-4-yl}-thiazolo[3,2-a]pyrimidin-5-one **(55)**
- 3-(3-Hydroxy-phenyl)-2-{2-[4-(4-methyl-piperazin-1-yl)-phenylamino]-pyrimidin-4-yl}-thiazolo[3,2-a]pyrimidin-5-one **(56)**
- 3-(4-Hydroxy-phenyl)-2-{2-[4-(4-methyl-piperazin-1-yl)-phenylamino]-pyrimidin-4-yl}-thiazolo[3,2-a]pyrimidin-5-one **(57)**
- 3-(4-Fluoro-phenyl)-2-(2-{3-[4-(2-hydroxy-ethyl)-piperazin-1-yl]-phenylamino}-pyrimidin-4-yl)-thiazolo[3,2-a]pyrimidin-5-one **(58)**
- 3-{4-[3-(2,3-Dichloro-phenyl)-5-oxo-5H-thiazolo[3,2-a]pyrimidin-2-yl]-pyrimidin-2-ylamino}-benzenesulfonamide **(59)**
- 3-(2,3-Dichloro-phenyl)-2-(2-{3-[4-(2-hydroxy-ethyl)-piperazin-1-yl]-phenylamino}-pyrimidin-4-yl)-thiazolo[3,2-a]pyrimidin-5-one **(60)**
- 3-{4-[3-(3,4-Dichloro-phenyl)-5-oxo-5H-thiazolo[3,2-a]pyrimidin-2-yl]-pyrimidin-2-ylamino}-benzenesulfonamide **(61)**
- 3-(3-Chloro-4-methoxy-phenyl)-2-(2-{3-[4-(2-hydroxy-ethyl)-piperazin-1-yl]-phenylamino}-pyrimidin-4-yl)-thiazolo[3,2-a]pyrimidin-5-one **(62)**
- 3-(4-Fluoro-3-trifluoromethyl-phenyl)-2-(2-{3-[4-(2-hydroxy-ethyl)-piperazin-1-yl]-phenylamino}-pyrimidin-4-yl)-thiazolo[3,2-a]pyrimidin-5-one **(63)**
- 2-(2-{3-[4-(2-Hydroxy-ethyl)-piperazin-1-yl]-phenylamino}-pyrimidin-4-yl)-3-isopropyl-thiazolo[3,2-a]pyrimidin-5-one **(64)**
- 3-Cyclopropyl-2-(2-{3-[4-(2-hydroxy-ethyl)-piperazin-1-yl]-phenylamino}-pyrimidin-4-yl)-thiazolo[3,2-a]pyrimidin-5-one **(65)**
- 2-(2-{3-[4-(2-Hydroxy-ethyl)-piperazin-1-yl]-phenylamino}-pyrimidin-4-yl)-5-oxo-3-phenyl-5H-thiazolo[3,2-a]pyrimidine-6-carboxylic acid ethyl ester **(71)**
- 2-(2-{3-[4-(2-Hydroxy-ethyl)-piperazin-1-yl]-phenylamino}-pyrimidin-4-yl)-7-methyl-3-phenyl-thiazolo[3,2-a]pyrimidin-5-one **(72)**
- 2-(2-{3-[4-(2-Hydroxy-ethyl)-piperazin-1-yl]-phenylamino}-pyrimidin-4-yl)-7-isopropyl-3-phenyl-thiazolo[3,2-a]pyrimidin-5-one **(73)**
- 7-Ethyl-2-(2-{3-[4-(2-hydroxy-ethyl)-piperazin-1-yl]-phenylamino}-pyrimidin-4-yl)-3-phenyl-thiazolo[3,2-a]pyrimidin-5-one **(74)**
- 2-(2-{3-[4-(2-Hydroxy-ethyl)-piperazin-1-yl]-phenylamino}-pyrimidin-4-yl)-6,7-dimethyl-3-phenyl-thiazolo[3,2-a]pyrimidin-5-one **(75)**
- 2-(2-{3-[4-(2-Hydroxy-ethyl)-piperazin-1-yl]-phenylamino}-pyrimidin-4-yl)-6-isopropyl-7-methyl-3-phenyl-thiazolo[3,2-a]pyrimidin-5-one **(76)**
- 6-Ethyl-2-(2-{3-[4-(2-hydroxy-ethyl)-piperazin-1-yl]-phenylamino}-pyrimidin-4-yl)-7-methyl-3-phenyl-thiazolo[3,2-a]pyrimidin-5-one **(77)**

8. The compound according to claim 7, wherein said compound is selected from the following list:
- 3-Phenyl-2-(2-phenylamino-pyrimidin-4-yl)-thiazolo[3,2-a]pyrimidin-5-one **(29)**
- 3-[4-(5-Oxo-3-phenyl-5H-thiazolo[3,2-a]pyrimidin-2-yl)-pyrimidin-2-ylamino]-benzenesulfonamide (30)
- 2-(2-{3-[4-(2-Hydroxy-ethyl)-piperazin-1-yl]-phenylamino}-pyrimidin-4-yl)-3-phenyl-thiazolo[3,2-a]pyrimidin-5-one **(31)**
- 2-{2-[4-(4-Methyl-piperazin-1-yl)-phenylamino]-pyrimidin-4-yl}-3-phenyl-thiazolo[3,2-a]pyrimidin-5-one **(34)**
- 3-{4-[3-(2-Chloro-phenyl)-5-oxo-5H-thiazolo[3,2-a]pyrimidin-2-yl]-pyrimidin-2-ylamino}-benzenesulfonamide **(37)**
- 3-(2-Chloro-phenyl)-2-(2-{3-[4-(2-hydroxy-ethyl)-piperazin-1-yl]-phenylamino}-pyrimidin-4-yl)-thiazolo[3,2-a]pyrimidin-5-one **(38)**
- 3-(2-Chloro-phenyl)-2-{2-[4-(4-methyl-piperazin-1-yl)-phenylamino]-pyrimidin-4-yl}-thiazolo[3,2-a]pyrimidin-5-one **(39)**
- 3-{4-[3-(3-Chloro-phenyl)-5-oxo-5H-thiazolo[3,2-a]pyrimidin-2-yl]-pyrimidin-2-ylamino}-benzenesulfonamide **(40)**
- 3-(3-Chloro-phenyl)-2-(2-{3-[4-(2-hydroxy-ethyl)-piperazin-1-yl]-phenylamino}-pyrimidin-4-yl)-thiazolo[3,2-a]pyrimidin-5-one **(41)**
- 3-(3-Chloro-phenyl)-2-{2-[4-(4-methyl-piperazin-1-yl)-phenylamino]-pyrimidin-4-yl}-thiazolo[3,2-a]pyrimidin-5-one **(42)**
- 3-{4-[3-(4-Chloro-phenyl)-5-oxo-5H-thiazolo[3,2-a]pyrimidin-2-yl]-pyrimidin-2-ylamino}-benzenesulfonamide **(43)**
- 3-(4-Chloro-phenyl)-2-(2-{3-[4-(2-hydroxy-ethyl)-piperazin-1-yl]-phenylamino}-pyrimidin-4-yl)-thiazolo[3,2-a]pyrimidin-5-one **(44)**
- 3-(4-Chloro-phenyl)-2-{2-[4-(4-methyl-piperazin-1-yl)-phenylamino]-pyrimidin-4-yl}-thiazolo[3,2-a]pyrimidin-5-one **(45)**
- 3-{4-[3-(2-Methoxy-phenyl)-5-oxo-5H-thiazolo[3,2-a]pyrimidin-2-yl]-pyrimidin-2-ylamino}-benzenesulfonamide **(46)**
- 2-(2-{3-[4-(2-Hydroxy-ethyl)-piperazin-1-yl]-phenylamino}-pyrimidin-4-yl)-3-(2-methoxy-phenyl)-thiazolo[3,2-a]pyrimidin-5-one **(47)**
- 3-(2-Methoxy-phenyl)-2-{2-[4-(4-methyl-piperazin-1-yl)-phenylamino]-pyrimidin-4-yl}-thiazolo[3,2-a]pyrimidin-5-one **(48)**
- 3-{4-[3-(3-Methoxy-phenyl)-5-oxo-5H-thiazolo[3,2-a]pyrimidin-2-yl]-pyrimidin-2-ylamino}-benzenesulfonamide **(49)**
- 2-(2-{3-[4-(2-Hydroxy-ethyl)-piperazin-1-yl]-phenylamino}-pyrimidin-4-yl)-3-(3-methoxy-phenyl)-thiazolo[3,2-a]pyrimidin-5-one **(50)**
- 3-(3-Methoxy-phenyl)-2-{2-[4-(4-methyl-piperazin-1-yl)-phenylamino]-pyrimidin-4-yl}-thiazolo[3,2-a]pyrimidin-5-one **(51)**
- 3-{4-[3-(4-Methoxy-phenyl)-5-oxo-5H-thiazolo[3,2-a]pyrimidin-2-yl]-pyrimidin-2-ylamino}-benzenesulfonamide **(52)**
- 2-(2-{3-[4-(2-Hydroxy-ethyl)-piperazin-1-yl]-phenylamino}-pyrimidin-4-yl)-3-(4-methoxy-phenyl)-thiazolo[3,2-a]pyrimidin-5-one **(53)**
- 3-(4-Methoxy-phenyl)-2-{2-[4-(4-methyl-piperazin-1-yl)-phenylamino]-pyrimidin-4-yl}-thiazolo[3,2-a]pyrimidin-5-one **(54)**
- 3-(2-Hydroxy-phenyl)-2-{2-[4-(4-methyl-piperazin-1-yl)-phenylamino]-pyrimidin-4-yl}-thiazolo[3,2-a]pyrimidin-5-one **(55)**
- 3-(3-Hydroxy-phenyl)-2-{2-[4-(4-methyl-piperazin-1-yl)-phenylamino]-pyrimidin-4-yl}-thiazolo[3,2-a]pyrimidin-5-one **(56)**
- 3-(4-Hydroxy-phenyl)-2-{2-[4-(4-methyl-piperazin-1-yl)-phenylamino]-pyrimidin-4-yl}-thiazolo[3,2-a]pyrimidin-5-one **(57)**
- 3-(4-Fluoro-phenyl)-2-(2-{3-[4-(2-hydroxy-ethyl)-piperazin-1-yl]-phenylamino}-pyrimidin-4-yl)-thiazolo[3,2-a]pyrimidin-5-one **(58)**
- 3-{4-[3-(2,3-Dichloro-phenyl)-5-oxo-5H-thiazolo[3,2-a]pyrimidin-2-yl]-pyrimidin-2-ylamino}-benzenesulfonamide **(59)**
- 3-(2,3-Dichloro-phenyl)-2-(2-{3-[4-(2-hydroxy-ethyl)-piperazin-1-yl]-phenylamino}-pyrimidin-4-yl)-thiazolo[3,2-a]pyrimidin-5-one **(60)**
- 3-{4-[3-(3,4-Dichloro-phenyl)-5-oxo-5H-thiazolo[3,2-a]pyrimidin-2-yl]-pyrimidin-2-ylamino}-benzenesulfonamide **(61)**
- 3-(3-Chloro-4-methoxy-phenyl)-2-(2-{3-[4-(2-hydroxy-ethyl)-piperazin-1-yl]-phenylamino}-pyrimidin-4-yl)-thiazolo[3,2-a]pyrimidin-5-one **(62)**
- 2-(2-{3-[4-(2-Hydroxy-ethyl)-piperazin-1-yl]-phenylamino}-pyrimidin-4-yl)-3-isopropyl-thiazolo[3,2-a]pyrimidin-5-one **(64)**
- 2-(2-{3-[4-(2-Hydroxy-ethyl)-piperazin-1-yl]-phenylamino}-pyrimidin-4-yl)-7-methyl-3-phenyl-thiazolo[3,2-a]pyrimidin-5-one **(72)**
- 2-(2-{3-[4-(2-Hydroxy-ethyl)-piperazin-1-yl]-phenylamino}-pyrimidin-4-yl)-7-isopropyl-3-phenyl-thiazolo[3,2-a]pyrimidin-5-one **(73)**
- 7-Ethyl-2-(2-{3-[4-(2-hydroxy-ethyl)-piperazin-1-yl]-phenylamino}-pyrimidin-4-yl)-3-phenyl-thiazolo[3,2-a]pyrimidin-5-one **(74)**
- 2-(2-{3-[4-(2-Hydroxy-ethyl)-piperazin-1-yl]-phenylamino}-pyrimidin-4-yl)-6,7-dimethyl-3-phenyl-thiazolo[3,2-a]pyrimidin-5-one **(75)**
- 2-(2-{3-[4-(2-Hydroxy-ethyl)-piperazin-1-yl]-phenylamino}-pyrimidin-4-yl)-6-isopropyl-7-methyl-3-phenyl-thiazolo[3,2-a]pyrimidin-5-one **(76)**
- 6-Ethyl-2-(2-{3-[4-(2-hydroxy-ethyl)-piperazin-1-yl]-phenylamino}-pyrimidin-4-yl)-7-methyl-3-phenyl-thiazolo[3,2-a]pyrimidin-5-one **(77)**

9. A compound of formula (I) according to any one of claims 1 to 8 for use as a medicament.

10. A compound of formula (I) according to any one of claims 1 to 8 for use in the prevention or treatment of a disease in which DDR1/2 is overexpressed, wherein said disease is selected from cancer, pulmonary fibrosis, cirrhotic liver, liver fibrosis, lupus nephritis, Goodpasture's syndrome, glomerulonephritis, osteoarthritis, rheumatoid arthritis and atherosclerosis.

11. The compound for use according to claim 10, wherein the cancer is selected from hepatocarcinoma, glioblastoma, gastric cancer, prostate cancer, Hodgkin lymphoma, lung cancer, breast cancer, colorectal cancer, pancreatic cancer, melanoma, ovarian cancer, papillary thyroid carcinoma, bladder cancer or sarcoma.

12. A composition comprising a compound of formula (I) according to any one of claims 1 to 8 and a pharmaceutically acceptable excipient, diluent or carrier.

13. The composition according to claim 12, which further comprises an antitumoral compound.

14. A method to obtain a compound of formula (I) according to any one of claims 1 to 8 which comprises reacting a compound of formula (II): 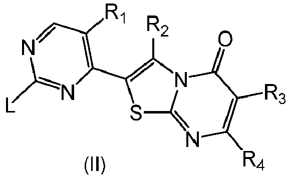 being R₁ to R₄ as defined in claim 1 and L a leaving group, preferably Cl, with a compound of formula (III):
NH₂-R₅ (III)
being R₅ as defined in claim 1.
